Europäisches Patentamt

European Patent Office

Office européen des brevets

(19)

(11)  **EP 1 723 913 A1**

(12)  **EUROPEAN PATENT APPLICATION**
published in accordance with Art. 158(3) EPC

(43) Date of publication:
   **22.11.2006 Bulletin 2006/47**

(51) Int Cl.:
   *A61B 17/06* (2006.01)   *A61B 17/28* (2006.01)

(21) Application number: **05720507.2**

(86) International application number:
   **PCT/JP2005/004236**

(22) Date of filing: **10.03.2005**

(87) International publication number:
   **WO 2005/084556 (15.09.2005 Gazette 2005/37)**

(84) Designated Contracting States:
   **DE FR GB**

(30) Priority: **10.03.2004   JP 2004068213
   10.03.2004   JP 2004068214
   03.06.2004   JP 2004166362
   16.09.2004   JP 2004270294**

(71) Applicant: **Olympus Corporation
   Tokyo 151-0072 (JP)**

(72) Inventors:
   • **MIYAMOTO, Manabu
     c/o OLYMPUS INTELLECTUAL PROPERTY
     Tokyo 192-8512 (JP)**

   • **BANJU, Kazuo
     c/o OLYMPUS INTELLECTUAL PROPERTY
     Tokyo 192-8512 (JP)**
   • **AKUI, Nobuaki
     c/o OLYMPUS INTELLECTUAL PROPERTY
     Tokyo 192-8512 (JP)**
   • **IIZUKA, Shuhei
     c/o OLYMPUS INTELLECTUAL PROPERTY
     Tokyo 192-8512 (JP)**

(74) Representative: **von Hellfeld, Axel
   Wuesthoff & Wuesthoff
   Schweigerstrasse 2
   81541 München (DE)**

(54)   **TREATMENT TOOL FOR SURGERY**

(57)   A surgical instrument of the present invention comprises: an insertion unit, an operation unit provided at the proximal end side of the insertion unit, and a treatment unit provided at the other end of the insertion unit and having a clamping unit that can rotate about one axis and can perform an opening and closing action and a bending action. The operation unit comprises: an opening and closing lever for performing the operation of opening and closing the treatment unit, an angle variation dial for performing the operation of bending the treatment unit, and a rotation dial for performing the operation of rotating the treatment unit. The angle variation dial and rotation dial are provided within the operation range of the same operation finger.

**FIG.1**

EP 1 723 913 A1

**Description**

Technical Field

The present invention relates to a surgical instrument for grasping a needle and suturing the tissues, for example, during coronary artery bypass graft (CABG) of the heart, under observation by an endoscope.

Background Art

The following procedure is known for conducting, for example, coronary artery bypass graft (CABG) of the heart under observation by an endoscope. That is, a bypass procedure is known in which an endoscope, a surgical instrument as a needle-holding device, a forceps and the like, are inserted into a chest cavity via a trocar piercing a thoracic wall, an anastomosis opening is provided by cutting open a part of the coronary artery with a scissor-like forceps, an internal thoracic artery is introduced with a grasping forceps into the anastomosis opening, and the internal thoracic artery is anastomosed and connected to the anastomosis opening. This procedure is described in greater detail, for example, in Min Invas Ther & Technol, 10:227-230,2001.

Furthermore, a surgical instrument having a structure in which an insertion unit having a curving section in the distal end section thereof is provided and jaws as a pair of treatment sections that can be opened and closed and can rotate about the axis of the insertion unit are provided in the distal end section of the insertion unit is described in US Patent No. 5,951,575 as a surgical instrument suitable for such procedures, that is, as a needle handling device for grasping a needle and anastomosing the tissues.

In such surgical instrument, as described above, jaws performing the function of a treatment unit that can be opened, closed, and rotated are provided in the distal end section of the insertion unit. The operation of rotating the jaws is performed by rotating the rotary operation dial provided in an operation unit, but the operation of opening and closing the jaws is performed by operating a lever for opening and closing operation that is provided in the operation unit.

The surgical instrument (as a needle handling device) disclosed by US Patent No. 5,951,575 as mentioned above has a pair of jaws that can be opened and closed in the distal end section of the insertion unit. When the pair of jaws are to be closed, they are drawn into a cylindrical member via a cable by the operation of the operation unit, so as to be closed for grasping the needle. Thus, the jaws are at a forward position when they are opened and at a rearward position when they are closed. Hence, if an operator has the jaws opened and approach the needle, it may come about that if he then tries to close the jaws so as to grasp the needle, the position of the jaws changes thereby causing a failure to grasp the needle. So, it has been said that the operability thereof is not good.

Also, since the surgical instrument disclosed by US Patent No. 5,951,575 as mentioned above is of the constitution in which when anastomosing the tissues under the endoscope, the friction of a bearing portion that is subject to the rotation of the jaws increases in a state wherein the jaws are closed. As a consequence, it is noted that (1) the load of the rotating force is increased by keeping the holding force, and (2) the operator has to perform the rotating operation as he applies the holding force. That is to say, it has been said to be difficult to stably hold the needle for suturing while performing the rotating operation.

Further, as for the pair of treatment sections having the jaws provided in the distal end section of the insertion unit in the surgical instrument disclosed by US Patent No. 5,951,575 as mentioned above, it is noted that although the treatment sections make it possible to change the angle of the extending direction with respect the insertion axis, it is required to slide an outer sheath in the insertion unit of the surgical instrument along the insertion axis when changing the angle of the extending direction. Then, when performing the operation using the surgical instrument, the operator has to move from the state wherein he grasps the operation unit to the state wherein he slides the outer sheath along the insertion axis in order to change the angle of the extending direction of the treatment sections. That is to say, the operator has to change his holding of the operation unit during the operation, and as a matter of fact, it is considered to be difficult to change the angle of the extending direction of the treatment sections without affecting his operation techniques.

The present invention is to provide a surgical instrument by which the tissues can be sutured easily and reliably under observation by an endoscope.

Furthermore, a forceps (for example, a surgical instrument used as a needle-holding device) is disclosed, for example in Japanese Patent Application Laid-open No. 2002-306495. A treatment unit (grasping unit) having first and second treatment pieces that can be opened and closed with respect to each other is provided in the distal end section of the insertion unit of the forceps. The treatment unit can rotate in one plane in the opening and closing direction of the first and second treatment pieces with respect to the axial direction of the insertion unit in the distal end section of the insertion unit and can also rotate about the lengthwise axis of the insertion unit. The forceps is thus a forceps with multiple degrees of freedom and, for example, after an anastomosis needle has been grasped by the first and second treatment pieces, the anastomosis needle can be operated in various directions and a complex anastomosis operation can be performed.

Although the forceps disclosed in the Japanese Patent Application Laid-open No. 2002-306495 is the one with multiple degrees of freedom and thus makes it possible to easily rotate the anastomosis needle or the like which is grasped to anastomose on complex locations, it may come to pass that the operation by the operator becomes rather complex when he performs anastomosis operation or the like because the degrees of freedom are too multiple. Further, since the forceps is of the complex constitution to realize the multiple degrees of freedom, there is such problem that the weight of it becomes larger than that of the conventional ones.

The present invention is to provide a surgical instrument which can change the angle of the anastomosis needle or the like which is grasped with respect to the insertion unit and easily perform the treatment such as anastomosis in a state wherein the anastomosis needle or the like has been grasped and which is of light weight and easy to operate.

Disclosure of Invention

Means for Solving the Problem

The present invention provides a surgical instrument comprising an insertion unit, an operation unit provided at the proximal end side of the insertion unit, and a treatment unit provided at the other end of the insertion unit and having a clamping unit that can freely rotate about one axis and can perform an opening and closing action and a bending action, wherein the operation unit comprises an opening and closing lever for performing the operation of opening and closing the treatment unit, an angle variation dial for performing the operation of bending the treatment unit, and a rotation dial for performing the operation of rotating the treatment unit. The angle variation dial and rotation dial are provided within the operation range of the operator's same finger.

Brief Description of the Drawings

Fig. 1 is a perspective view illustrating the external appearance of a needle driver of a first embodiment of the present invention as viewed from one side at an angle in a front view;

Fig. 2 is a first drawing explaining the operation of the treatment unit in the needle driver of the first embodiment;

Fig. 3 is a second drawing explaining the operation of the treatment unit in the needle driver of the first embodiment;

Fig. 4 is a third drawing explaining the operation of the treatment unit in the needle driver of the first embodiment;

Fig. 5 is a flow chart illustrating the flow of a procedure of anastomosing an internal thoracic artery and a coronary artery by using the needle driver of the first embodiment;

Fig. 6 is a main-part enlarged front view illustrating a state in the procedure of anastomosing an internal thoracic artery and a coronary artery by using the needle driver of the first embodiment;

Fig. 7 is a main-part enlarged front view illustrating another state in the procedure of anastomosing an internal thoracic artery and a coronary artery by using the needle driver of the first embodiment;

Fig. 8 is a perspective view illustrating external appearance of the needle driver of the first embodiment of the present invention as viewed from another side at an angle in a rear view;

Fig. 9 is a front view of the needle driver of the first embodiment;

Fig. 10 is a left side view of the needle driver of the first embodiment as viewed from one side;

Fig. 11 is a right side view of the needle driver of the first embodiment as viewed from another side (right side);

Fig. 12 is a rear view of the needle driver of the first embodiment;

Fig. 13 is a side view of a distal end portion comprising the treatment unit in the needle driver of the first embodiment;

Fig. 14 is a rear view of a distal end portion comprising the treatment unit in the needle driver of the first embodiment;

Fig. 15 is a sectional view along the IX-IX line in Fig. 13;

Fig. 16 is a sectional view along the XVI-XVI line in Fig. 14;

Fig. 17 is a sectional view along the XVII-XVII line in Fig. 14;

Fig. 18 is a main-part enlarged perspective view illustrating the internal structure of the distal end section in the needle driver of the first embodiment, the internal structure being seen through the distal end fixing member shown by a broken line indicating the omission of it;

Fig. 19 is a main-part enlarged perspective view illustrating the internal structure of the distal end section in the needle driver of the first embodiment, the internal structure being seen through the distal end fixing member shown by a broken line indicating the omission of it;

Fig. 20 is a rear view of the distal end section in the needle driver of the first embodiment;

Fig. 21 is a sectional view along the XXI-XXI line in Fig. 20;

Fig. 22 is a sectional view along the XXII-XXII line in Fig. 20;

Fig. 23 is a main-part enlarged perspective view illustrating the external appearance of the treatment unit in the needle driver of the first embodiment;

Fig. 24 is a main-part enlarged perspective view illustrating the internal structure in which some members in the needle driver of the first embodiment are partially omitted;

Fig. 25 is a main-part enlarged perspective view illustrating the internal structure in which some members in the needle driver of the first embodiment are partially omitted;

Fig. 26 is a main-part enlarged perspective view illustrating the internal structure in which some members in the needle driver of the first embodiment are partially omitted;

Fig. 27 is a main-part enlarged perspective view illustrating the state where the clamping unit of the treatment unit in the needle driver of the first embodiment is open;

Fig. 28 is a rear view of the treatment unit and peripheral unit in a state where the clamping unit of the treatment unit in the needle driver of the first embodiment is open;

Fig. 29 is a sectional view of the treatment unit and peripheral unit in a state where the clamping unit of the treatment unit in the needle driver of the first embodiment is open;

Fig. 30 is a sectional view of the treatment unit and peripheral unit in a state where the treatment unit in the needle driver of the first embodiment is curved;

Fig. 31 is a first drawing explaining the curving center of the treatment unit in the needle driver of the first embodiment;

Fig. 32 is a second drawing explaining the curving center of the treatment unit in the needle driver of the first embodiment;

Fig. 33 is a third drawing explaining the curving center of the treatment unit in the needle driver of the first embodiment;

Fig. 34 is a fourth drawing explaining the curving center of the treatment unit in the needle driver of the first embodiment;

Fig. 35 is a fifth drawing explaining the curving center of the treatment unit in the needle driver of the first embodiment;

Fig. 36 is a front view of the needle driver of the first embodiment;

Fig. 37 is a rear view of the needle driver of the first embodiment;

Fig. 38 is a main-part side view illustrating the operation unit in the needle driver of the first embodiment from one side thereof;

Fig. 39 is a main-part sectional view in which the operation unit in the needle driver of the first embodiment is cut along the XXXVII-XXXVII line in Fig. 38;

Fig. 40 is a main-part perspective view illustrating the rotation dial, angle variation dial, and opening and closing lever in the needle driver of the first embodiment, wherein the external section of the operation unit is omitted;

Fig. 41 is a main-part enlarged perspective view illustrating the rotation dial and angle variation dial in the needle driver of the first embodiment, wherein the external section of the operation unit is omitted; this figure shows a state where the treatment unit is curved;

Fig. 42 is a main-part enlarged perspective view illustrating the rotation dial and angle variation dial in the needle driver of the first embodiment, wherein the external section of the operation unit is omitted; this figure shows the initial state of the treatment unit (no curving);

Fig. 43 is a main-part enlarged perspective view illustrating the opening and closing lever in the needle driver of the first embodiment, wherein the external section of the operation unit is omitted; this figure shows a state where the treatment unit is closed;

Fig. 44 is a main-part enlarged perspective view illustrating the opening and closing lever in the needle driver of the first embodiment, wherein the external section of the operation unit is omitted; this figure shows a state where the treatment unit is open;

Fig. 45 is a perspective view illustrating the external appearance of the needle driver of a variation example of the first embodiment, as viewed from one side at an angle in a front view;

Fig. 46 is an explanatory drawing illustrating the state where the needle driver of the variation example of the first embodiment is gripped;

Fig. 47 is a perspective view illustrating the external appearance of the needle driver of another variation example of the first embodiment, as viewed from one side at an angle in a front view;

Fig. 48 is a partial sectional view of the operation unit of the variation example of the first embodiment;

Fig. 49 is an external appearance drawing illustrating the state where an opening and closing press-button of the operation unit of the variation example of the first embodiment is pressed down;

Fig. 50 is an explanatory drawing illustrating the opening and closing action mechanism of the treatment unit actuated by the opening and closing press-button of the operation unit of the variation example of the first embodiment;

Fig. 51 is a side surface view of the treatment unit of the variation example of the first embodiment;

Fig. 52 is an explanatory drawing illustrating the action of clamping a needle by using the needle driver of the first embodiment;

Fig. 53 is an explanatory drawing illustrating the action of clamping a needle by using the needle driver of the variation example of the first embodiment;

Fig. 54 is another explanatory drawing illustrating the action of clamping a needle by using the needle driver of the first embodiment;

Fig. 55 is another explanatory drawing illustrating the action of clamping a needle by using the needle driver of the variation example of the first embodiment;

Fig. 56(A) is a schematic perspective view illustrating the needle-holding device of the second embodiment. Fig. 56 (B) is a schematic perspective view illustrating the anastomosis of blood vessels in a state where the anastomosis needle is clamped with the needle-holding unit of the needle-holding device;

Fig. 57 is a schematic longitudinal sectional view illustrating the operation unit and the proximal end section of the insertion unit of the needle-holding device of a second embodiment;

Fig. 58(A) is a schematic longitudinal sectional view illustrating the needle-holding unit and the distal end section of the insertion unit of the needle-holding device of the second embodiment; Fig. 58(B) is a schematic view from the direction of arrow 3B shown in Fig. 58(A);

Fig. 59 is a schematic longitudinal sectional view illustrating the needle-holding unit and the distal end section of the insertion unit of the needle-holding device of a third embodiment;

Fig. 60 is a schematic view of the needle-holding device of the third embodiment;

Fig. 61 is a schematic longitudinal sectional view illustrating the needle-holding unit and the distal end section of the insertion unit of the needle-holding device of a fourth embodiment;

Fig. 62 is a schematic view of the needle-holding device of a fifth embodiment;

Fig. 63 is a schematic longitudinal sectional view illustrating the operation unit and the proximal end section of the insertion unit of the needle-holding device of the fifth embodiment;

Fig. 64 is a schematic longitudinal sectional view illustrating the needle-holding unit and the distal end section of the insertion unit of the needle-holding device of the fifth embodiment;

Fig. 65 is a schematic perspective view illustrating the linking structure between the needle-holding unit and operation unit of the needle-holding device of the fifth embodiment;

Fig. 66 is a schematic longitudinal sectional view of the needle-holding unit and the distal end section of the insertion unit of the needle-holding device of the fifth embodiment;

Fig. 67 is a schematic longitudinal sectional view illustrating the needle-holding unit and the distal end section of the insertion unit of the needle-holding device of the fifth embodiment;

Fig. 68(A) is a schematic longitudinal sectional view illustrating the needle-holding unit and the distal end section of the insertion unit of the needle-holding device of a sixth embodiment; Fig. 68(B) is a schematic perspective view illustrating the electromagnet shown in Fig. 68(A);

Fig. 69(A) is a schematic longitudinal sectional view illustrating the needle-holding unit and the distal end section of the insertion unit of the needle-holding device of a seventh embodiment; Fig. 69(B) is a schematic view illustrating the structure on the side of the operation unit that links the needle-holding unit and the distal end section of the insertion unit;

Fig. 70(A) is a schematic longitudinal sectional view illustrating the needle-holding unit and the distal end section of the insertion unit of the needle-holding device of the seventh embodiment; Fig. 70(B) is a schematic view illustrating the structure on the side of the operation unit that links the needle-holding unit and the distal end section of the insertion unit;

Fig. 71(A) is a schematic longitudinal sectional view illustrating the needle-holding unit and the distal end section of the insertion unit of the needle-holding device of an eighth embodiment; Fig. 71(B) is a schematic view from the direction of arrow 16B shown in Fig. 71(A); and

Fig. 72 is a schematic longitudinal sectional view illustrating the needle-holding unit and the distal end section of the insertion unit of the needle-holding device of a ninth embodiment.

Best Mode for Carrying Out the Invention

The embodiments of the present invention will be described below with reference to the appended drawings.

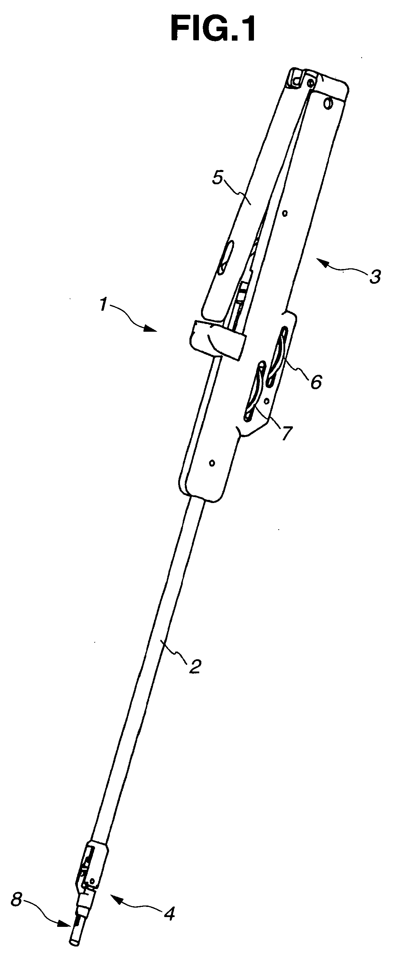

Fig. 1 is a perspective view illustrating the external appearance of the needle driver of a first embodiment of the present invention as viewed from one side at an angle in a front view.

As shown in Fig. 1, a needle driver 1 serving as a needle-holding device, which is a surgical instrument, of the first embodiment comprises an insertion unit 2, an operation unit 3, and a treatment unit 4 provided at a distal end of the insertion unit 2 as the main components. The needle driver will be described in greater detail hereinbelow. The structure of the insertion unit 2, operation unit 3, and treatment unit 4 constituting the needle driver 1 will be schematically described below, followed by detailed explanation thereafter.

The operation unit 3 comprises an opening and closing lever 5, an angle variation dial 6, and a rotation dial 7. The opening and closing lever 5 serves for performing the operation of opening and closing the treatment unit 4. The angle variation dial 6 serves for performing the operation of changing the angle of the extension direction of the treatment unit 4. The rotation dial 7 serves for performing the operation of rotating the treatment unit 4.

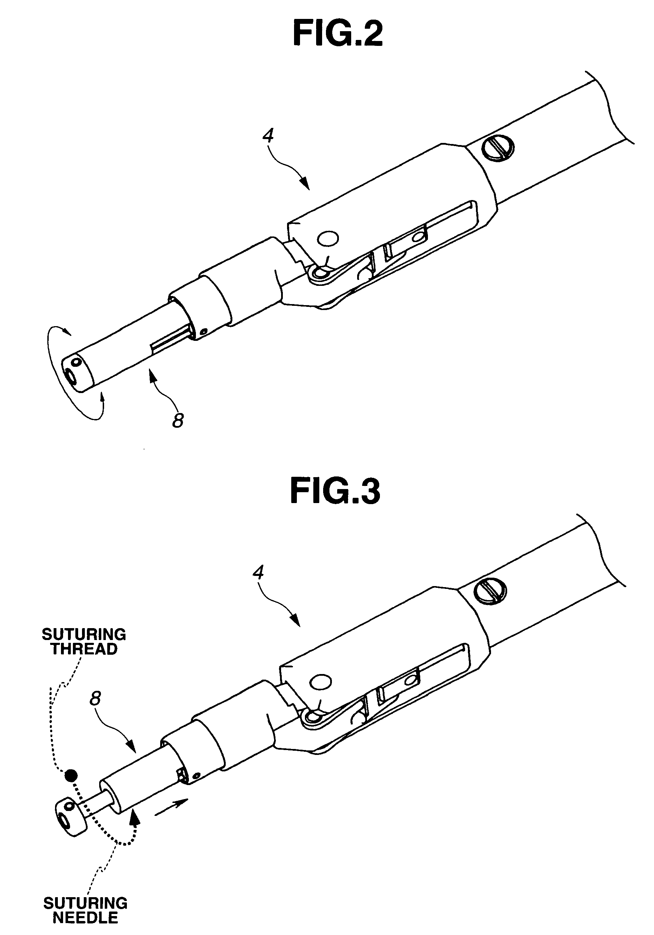

As shown in Fig. 2, the treatment unit 4 provided so as to extend from one end of the insertion unit 2 comprises a clamping unit 8 at the distal end side thereof. When the opening and closing lever 5 is pressed down, the clamping unit 8 is opened, as shown in Fig. 3, and when the operation of pressing the opening and closing lever 5 down is stopped, the clamping unit 8 is closed, as shown in Fig. 2.

Performing the operation of opening and closing the clamping unit 8 with the opening and closing lever 5 makes it possible to clamp/release a suturing needle (not shown in the figure) having a suturing thread for suturing a wound with the clamping unit 8.

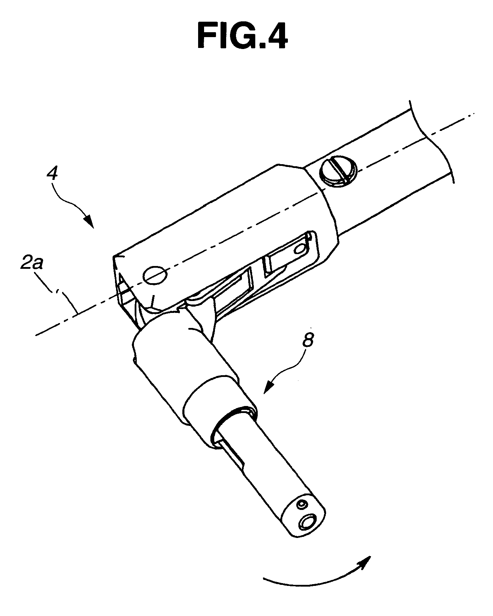

Furthermore, by rotating the angle variation dial 6, as shown in Fig. 4, the angle of the extension direction of the clamping unit 8 of the treatment unit 4 can be rotated in the prescribed plane comprising an insertion axis 2a of the insertion unit 2, and the extension direction of the treatment unit 4 can be changed through any angle with respect to the insertion axis 2a.

Moreover, by rotating the rotation dial 7, as shown in Fig. 2, the clamping unit 8 of the treatment unit 4 can be rotated about a long axis of the treatment unit 4, and by operating the rotation dial 7 in a state where the suturing needle is clamped by the clamping unit 8, the tissue of a living body is sutured with the suturing needle.

A surgical procedure of a coronary artery bypass surgery will be explained hereinbelow as a method for endoscopic anastomosis using the needle driver that is a surgical instrument of the present embodiment.

In the coronary artery bypass surgery, the skin in the prescribed position of the chest (for example, in a position between the third, fourth, and sixth ribs on the left side) is cut with a scalpel.

Then, a finger or an inner sheath with a conical distal end is inserted through an outer sheath tube of a trocar and thrust through the distal end thereof to press and expand the cut section of the skin and form a hole inside the body. Pulling the inner sheath from the outer sheath tube of the trocar at the stage of forming the holes in the prescribed positions produces port holes leading to the inside of the body with a plurality of, for example, three trocars. As a result, various instruments can be introduced into the left chest cavity via a plurality of trocars.

Then, the ventilation of one lung is executed to ensure a view field, as employed in the usual (publicly known) thoracoscopy. Thus, an air tube for lung ventilation is inserted through a pipe, the ventilation of only one (right) lung is executed, and the other (left) lung is caused to collapse.

Then, an internal thoracic artery ablation procedure is performed. In the internal thoracic artery ablation procedure, an ultrasonic instrument, grasping forceps, and an endoscope (not shown in the figure) are inserted into a plurality of trocars provided in the port holes. The ultrasonic instrument is connected to an ultrasonic control device for supplying and controlling the ultrasonic drive energy to the ultrasonic instrument. Furthermore, the endoscope is connected to a light source for supplying illumination light and a CCU (camera control unit) for signal processing and displaying endoscopic images.

Then, the ultrasonic instrument is brought close to the internal thoracic artery under endoscopic observations and the pleura covering the internal thoracic artery is cut. The cut open portion of the pleura is pulled and the internal thoracic artery and collaterals thereof are exposed from the surrounding tissue with the grasping forceps and ultrasonic instrument, the collateral (blood vessel) extending from the side wall of the internal thoracic artery is cut with the ultrasonic instrument, hemostasis of the cut collateral (blood vessel) is performed with the ultrasonic instrument, and partial ablation of the internal thoracic artery is performed.

The region is expanded, the operation of cutting the pleura is continued and the above-described partial ablation is repeated till the prescribed amount (for example, about 15 cm to 20 cm) of internal thoracic artery ablation is achieved.

When the prescribed amount of internal thoracic artery ablation is achieved, the blood is stopped with hemostasis clips in two places in the cut positions of the internal thoracic artery at the periphery thereof. The internal thoracic artery is then cut in the cutting positions of the internal thoracic artery between the hemostasis clips by using scissor-type forceps instead of the ultrasonic instrument, thereby completing the internal thoracic artery ablation procedure.

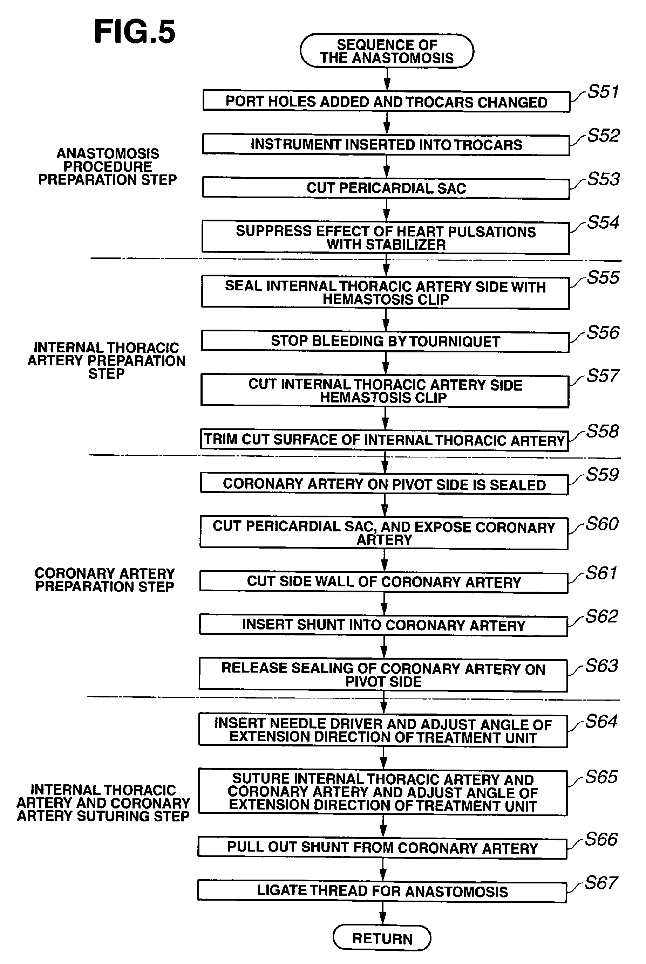

Once the internal thoracic artery ablation procedure has thus been completed, the anastomosis procedure of the internal thoracic artery and coronary artery is performed. The sequence of the anastomosis procedure will be explained below by using the flowchart shown in Fig. 5.

In the anastomosis procedure of the internal thoracic artery and coronary artery, as shown in Fig. 5, in step S51, for example, three port holes are added in the positions making it possible to bring various instruments close to the heart from above, and the insertion positions of a plurality of trocars are changed. Then, an endoscope is inserted via a trocar immediately above the coronary artery. That is, the surgery is performed, while conducting observations from substantially directly above the surgery site.

Then, in step S52, a stabilizer, the needle driver 1, which is the surgical instrument of the present embodiment, or other forceps, endoscopes, and grasping forceps are inserted through a plurality of trocars. For example, the insertion unit 2 of the needle driver 1 is inserted into the chest cavity and the operation unit 3 of the needle driver 1 is positioned outside the body cavity.

The stabilizer is the instrument to suppress the effect of heart pulsations. Such an instrument is disclosed, for example, in US Patent No. 5,807,243 and explanation thereof is herein omitted.

Then, in step S53, a pericardial sac is cut, the epicardium surface is exposed, and in step S54, the effect of heart pulsations in the vicinity of the target coronary artery is suppressed with the stabilizer.

The above-described step S51 through step S54 are preparatory steps for anastomosis procedure in the sequence of anastomosis operations.

Then, in step S56, a tourniquet for binding tight the internal thoracic artery is inserted, while suppressing the effect of heart pulsations with the stabilizer, the internal thoracic artery is sealed with the tourniquet, then in step S57, the portions where the hemastosis clips are attached are cut, and, in step S58, the cut surface of the internal thoracic artery is trimmed to the prescribed shape by using the scissor-like forceps.

The above-described step S55 to step S58 are the internal thoracic artery preparation steps in the anastomosis procedure sequence.

Then, in step S59, the coronary artery on the pivot side is sealed with the tourniquet. Then, in step S60, the pericardial sac covering the coronary artery is cut with a Beaver scalpel (or micro scalpel) with a round distal end, the coronary artery is exposed, and side wall of the coronary artery is cut with a micro scalpel having a sharp tip in step S61, the opening of prescribed size is provided with the scissors-like forceps, and an anastomosis orifice is formed.

Then, in step S62, a shunt is inserted into the coronary artery from the anastomosis orifice of the coronary artery. In step S63, the tourniquet is loosened, and tight binding of the coronary artery is released. The blood flow in the coronary artery is thereby ensured.

The above-described step S59 to step S63 are the coronary artery preparation steps of the anastomosis procedure sequence.

Then, in step S64, the needle driver 1, which is a surgical instrument of the present embodiment, is inserted, and the angle of the extension direction of the clamping unit 8 of the treatment unit 4 of the needle driver 1 is adjusted.

Then, a procedure of blood vessel anastomosis (suturing) of the internal thoracic artery and coronary artery with the needle driver 1, which is the surgical instrument of the present embodiment, is conducted in step S65. In the procedure of blood vessel anastomosis (suturing) of the internal thoracic artery and coronary artery, the angle of the extension direction of the clamping unit 8 of the treatment unit 4 of the needle driver 1 is readjusted according to the procedure state, while advancing the continuous suturing of the internal thoracic artery and coronary artery.

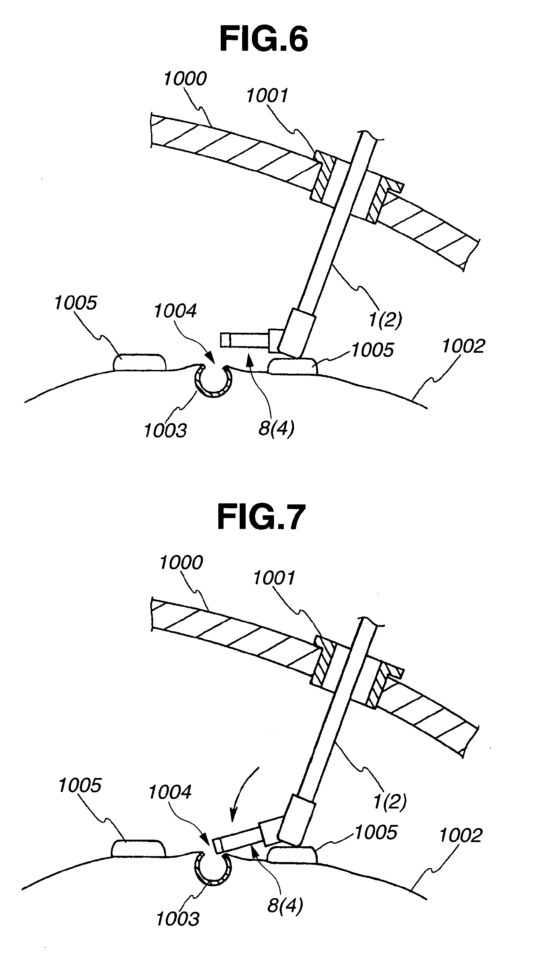

More specifically, as shown in Fig. 6, when the treatment unit 4 located at the distal end of the needle driver 1 is caused to access the vicinity of a heart 1002 via a trocar 1001 inserted through a chest wall 1000 and a continuous suturing is performed to join the cut surface of the internal thoracic artery (not shown in the figure) and an anastomosis orifice 1004 of a coronary artery 1003 by using the needle driver 1, the effect of pulsations of the heart 1002 is suppressed, as described above, with a stabilizer 1005. For this reason, the stabilizer 1005 sometimes interferes with the approach of the clamping unit 8 of the treatment unit 4 to the suturing portion of the cut surface of the internal thoracic artery and the anastomosis orifice 1004 of the coronary artery 1003.

Accordingly, as shown in Fig. 7, the angle of the extension direction of the clamping unit 8 of the treatment unit 4 is adjusted to the desired angle during the insertion of the needle driver 1 or during the continuous suturing by rotating the angle variation dial 6 provided in the operation unit 3.

In the needle driver 1 of the present embodiment, because the angle variation dial 6 is provided in the operation unit 3, the surgeon can rotate the angle variation dial 6 in a state where the gripping mode is maintained, without causing any changes in the gripping mode of the operation unit 3, can readjust the angle of the extension direction of the clamping unit 8 of the treatment unit 4 of the needle driver 1, without impeding the procedure, and can avoid the interference with the stabilizer 1005.

One or two stitches before the procedure of blood vessel anastomosis (suturing) of the internal thoracic artery and coronary artery is completed, in step S66, the shunt retained in the coronary artery is pulled out, one or two stitches are made, and a thread ligating procedure is implemented in step S67.

The above-described step S64 to step S67 are the steps of anastomosing the internal thoracic artery and coronary artery in the anastomosis procedure sequence.

The structure of the needle driver 1, which is the above-described surgical instrument serving as a needle-holding device, will be described below with reference to the appended drawings.

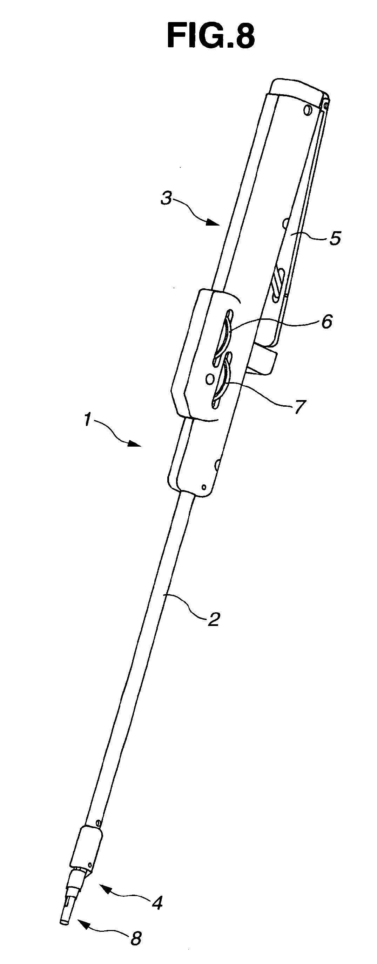

As described above, Fig. 1 is a perspective view illustrating the external appearance of the needle driver of the first embodiment of the present invention as viewed from one side at an angle in a front view. Fig. 8 is a perspective view illustrating external appearance of the needle driver of the first embodiment of the present invention as viewed from another side at an angle in a rear view.

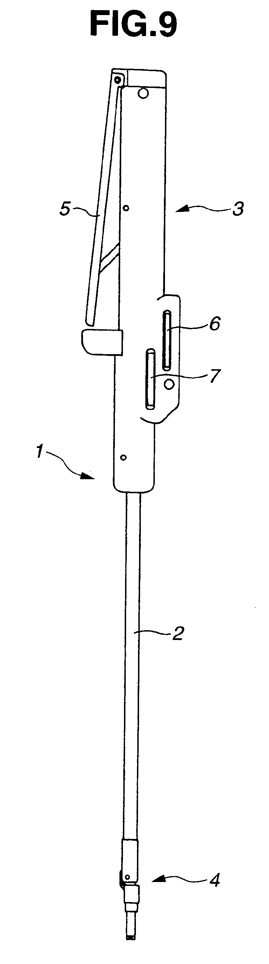

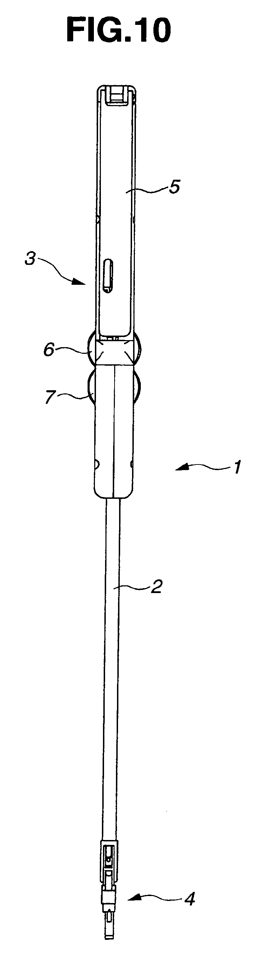

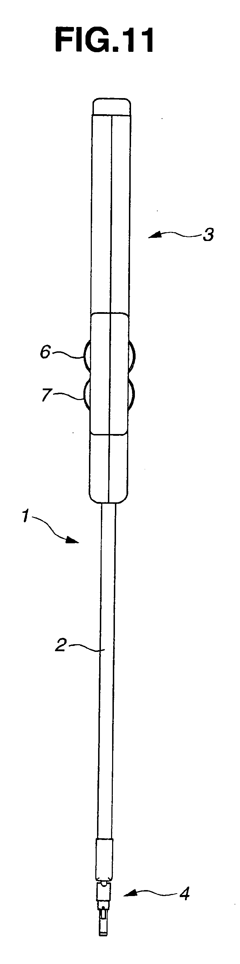

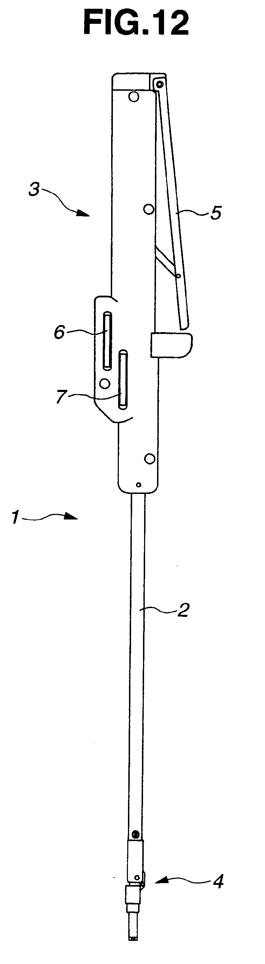

Furthermore, of Fig. 9 to Fig. 12, Fig. 9 is a front view of the needle driver of the first embodiment, Fig. 10 is a left side view of the needle driver of the first embodiment as viewed from one side, Fig. 11 is a right side view of the needle driver of the first embodiment as viewed from another side (right side), and Fig. 12 is a rear view of the needle driver of the first embodiment.

The needle driver 1 comprises as the main components the insertion unit 2, the operation unit 3 provided at one end (proximal end side) of the insertion unit 2, and the treatment unit 4 provided so as to extend form the other end of the insertion unit 2.

The insertion unit 2 has a substantially cylindrical shape having the prescribed length. The operation unit 3 is a member having a shape close to that of a rectangular parallelepiped and is disposed integrally on the same axis as the long axis of the insertion unit 2 at the proximal end side of the insertion unit 2. The shape of the operation unit is such that the surgeon can grip it with one hand and perform the below-described operations.

Furthermore, the opening and closing lever 5 serving as an opening and closing operation unit for performing the operations of opening and closing the treatment unit 4, the angle variation dial 6 serving as an angle variation operation unit for performing the operation of changing the angle of the extension direction of the treatment unit 4, and the rotation dial 7 serving as a rotational operation unit for performing the operation of rotating the treatment unit 4 are provided in the operation unit 3.

The proximal end section of the opening and closing lever 5, which is described hereinbelow in greater detail, is pivotally supported on one side of the proximal end section of the operation unit 3, whereas the free end section of the opening and closing lever 5 extends toward the distal end side of the operation unit 3 and is impelled in the direction of going away from the external cover section of the operation unit 3 by an impelling force of the below-described spring 33.

One end of the proximal end side of a pulling wire (not shown in Fig. 8 to Fig. 12) is fixed to an opening and closing base member (not shown in Fig. 8 to Fig. 12) engaged with the opening and closing lever 5, and the impelling force of the spring 33 is applied to the opening and closing lever 5 via this pulling wire, this configuration being described hereinbelow in greater detail.

The treatment unit 4 provided so as to extend from one end of the insertion unit 2 has a clamping unit 8 at the distal end side thereof, and the axial direction of the clamping unit 8, that is, the extension direction of the treatment unit 4, can vary within the prescribed angle range with respect to the axial direction of the insertion unit 2. In other words, angle variation means for changing the angle of the extension direction of the treatment unit 4 with respect to the axis of the insertion unit 2 is provided in the needle driver 1.

The internal structure of the needle driver 1 will be described hereinbelow by using the appended drawings.

First, the structure of the distal end section of the needle driver 1 will be explained.

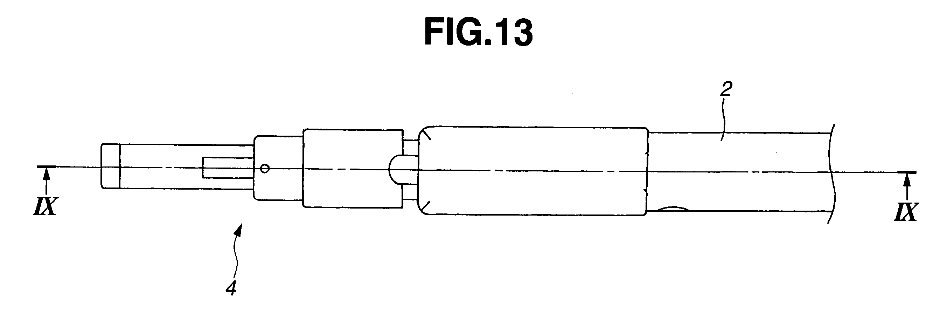

Fig. 13 to Fig. 17 are explanatory drawings illustrating the structure of the distal end portion comprising the treatment unit 4 of the needle driver 1.

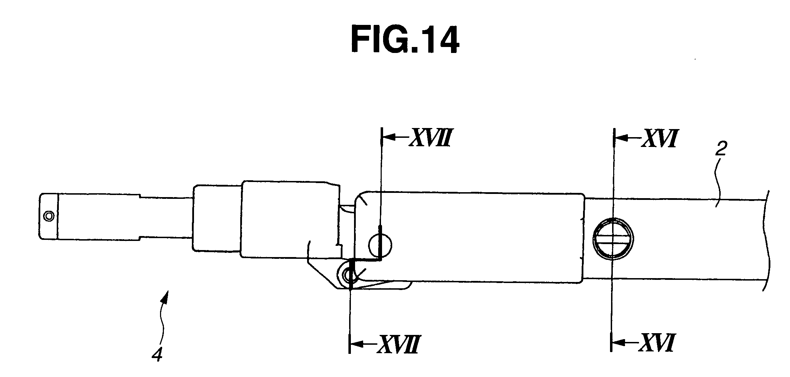

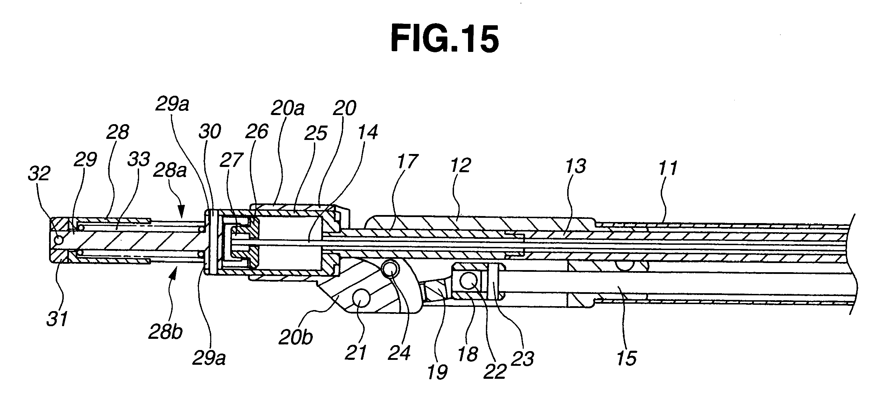

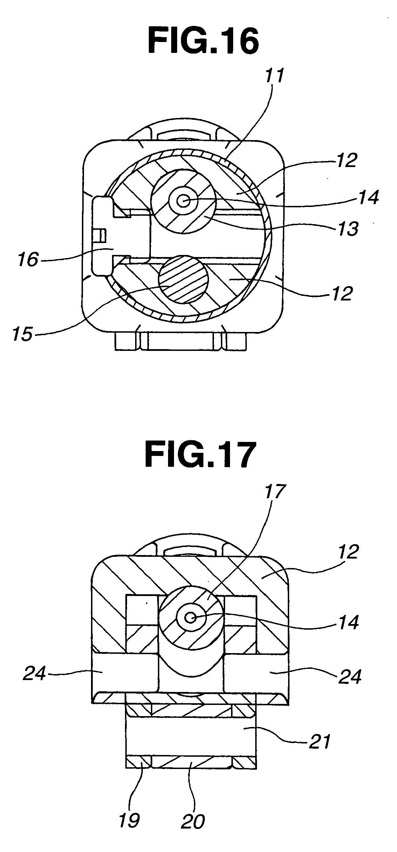

Fig. 13 is a side view of the distal end portion comprising the treatment unit 4 in the needle driver 1 of the first embodiment. Fig. 14 is a rear view of a distal end portion comprising the treatment unit 4 in the needle driver 1 of the first embodiment. Fig. 15 is a sectional view of the distal end portion comprising the treatment unit 4 of the needle driver 1. More specifically, Fig. 15 is a sectional view along the IX-IX line in Fig. 13. Fig. 16 and Fig. 17 are sectional views of the distal end portion of the needle driver 1. More specifically, Fig. 16 is a sectional view along the XVI-XVI line in Fig. 14, and Fig. 17 is a sectional view along the XVII-XVII line in Fig. 14.

The insertion unit 2 has a sheath 11 in the form of a stainless steel pipe, that is, a cylindrical member. A distal end fixing member 12 made of stainless steel is fixed to the distal end side, that is, on the side of the treatment unit 4, of the sheath 11. The distal end fixing member 12 has a cylindrical joining section for joining to the inner peripheral surface of the sheath 11 on the proximal end side of the distal end fixing member 12, that is, on the side of the sheath 11, and a channel-shaped section that has an internal cavity and a channel-like cross section perpendicular to the axis of the insertion unit 2 on the distal end side of the distal end fixing member 12, that is, on the side of the clamping unit 8.

A rotation power transmission pipe 13 made of stainless steel is passed as a shaft member through the sheath 11. The rotation power transmission pipe 13 is a pipe for transmitting a rotation power to the distal end section. A pulling wire 14 made of stainless steel and serving to open and close the below-described clamping unit 8 is passed through inside the rotation power transmission pipe 13.

The pulling wire 14 is a wire member that is pulled toward the side of the operation unit 3 to open the clamping unit 8 and has a flexible structure obtained by twisting thin stainless steel wires. Furthermore, the wire surface is coated with a fluorine resin to reduce the sliding resistance and facilitate back and forth movement of the wire inside the pipe.

The joining section of the distal end fixing member 12 is a rod-like member in which the cross section perpendicular to the axis of the insertion unit 2 has a substantially circular shape, but the portion of the joining section which is fixed with a locking screw 16 (described hereinbelow) made of a stainless steel, as shown in Fig. 16, has a semi-cylindrical shape such that the cross section in this portion has a substantially semi-cylindrical shape. The rotation power transmission pipe 13 and a curving power transmission rod 15 made of stainless steel are inserted in positions substantially linearly symmetrical with respect to the axis of the joining section of the distal end fixing member 12. The rotation power transmission pipe 13 is so inserted that it can slide and rotate about the axis of the rotation power transmission pipe 13 as a rotary center, and the curving power transmission rod 15, which is a rod-like member and made of stainless steel, is inserted so that it can move back and forth in the axial direction of the curving power transmission rod 15.

The sheath 11 and distal end fixing member 12 are fixed with a locking screw 16 made of stainless steel, and the distal end section of the sheath 11 and the distal end fixing member 12 are further fixed by applying an adhesive, for example, an epoxy resin adhesive.

A rotation power transmission coil 17 made from stainless steel is fixed to the distal end of the rotation power transmission pipe 13. The rotation power transmission coil 17 is a flexible coil for transmitting a rotation power to the distal end portion of the insertion unit 2. The pulling wire 14 is passed through inside the rotation power transmission coil 17. Because the rotation power transmission pipe 13 is made from a metal, the rotation power created by the operation of rotating the rotation dial 7 in the operation unit 3 can be reliably transmitted to the rotation power transmission coil 17.

The rotation power transmission coil 17 connected to the rotation power transmission pipe 13 has a triple-winding intimate contact structure composed of three stacked coils. The second coil wound in the direction opposite that of the first coil is provided so as to be on the first coil, and the third coil wound in the direction (winding direction identical to that of the first coil) opposite that of the second coil is provided so as to be on the second coil.

Both end sections of the rotation power transmission coil 17 are soldered and cut upon soldering. As a result, the thickness of both end sections is less than that of the central section. Furthermore, the two end sections are fixed by soldering to the rotation power transmission pipe 13 and a rotation section base member 25.

The curving power transmission rod 15 is linked to a curving section base member 20 via a link joint 18 made of a stainless steel and a H-shaped link member 19.

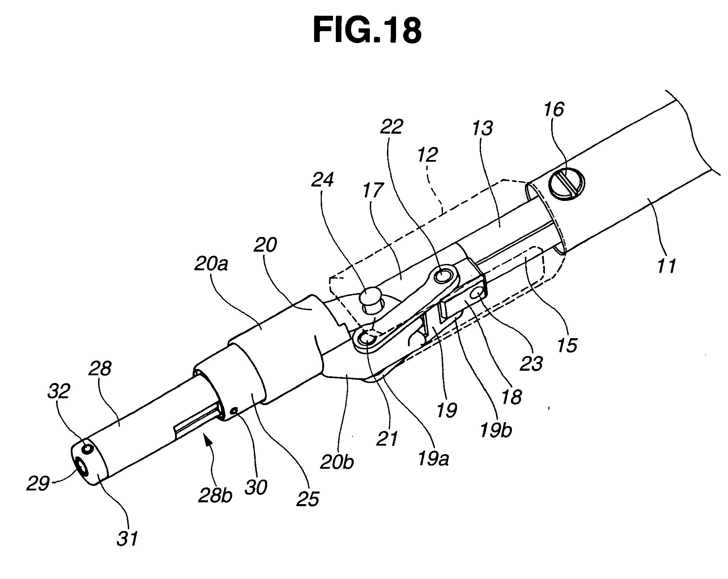

Fig. 18 is a main-part enlarged perspective view illustrating the internal structure of the distal end section, the internal structure being seen through the distal end fixing member 12 shown by a broken line indicating the omission of it. As shown in Fig. 18, the H-shaped link member 19 is of a H-like shape having two arm sections 19a, 19b at each side of a base section. The length of two arm sections 19a is larger than the length of other two arm sections 19b.

The curving section base member 20 has a cylindrical section 20a at the distal end side and has a protruding section 20b at the proximal end side, this protruding section extending toward the curving power transmission rod 15 so as to protrude from the side surface and bottom of the proximal section of the cylindrical section 20a. An opening is provided in the distal end side of the cylindrical section 20a. As shown in Fig. 18, the protruding section 20b is linked by a pin 21 passing therethrough so that part of the protruding section 20b is sandwiched between the two arm sections 19a of the H-shaped link member 19 that have a larger length. The pin 21 is fixed by laser welding in the end section of the H-shaped link member 19, and the protruding section 20b can rotate about the axis of the pin 21 as a rotation center.

Furthermore, as shown in Fig. 18, one hole of the link joint 18 is linked by a pin 22 passing therethrough so that part of the link joint 18 is sandwiched between other two arm sections 19b of the H-shaped link member 19 that have a smaller length. The link joint 18 has a shape of a substantially rectangular parallelepiped and has three holes formed therein. The pin 22 is fixed by laser welding to the distal section of the H-shaped link member 19, and the link section 18 can rotate about the axis of the pin 22 as a rotation center.

The link joint 18 further has a hole section into which the curving power transmission rod 15 can be inserted. The curving power transmission rod 15 inserted into the hole section is linked by the pin 23 passing through the curving power transmission rod 15. The pin 23 has an axis in the direction perpendicular to the axis of the pin 22. The pin 23 is fixed by laser welding in the end section of the link joint 18.

The protruding section 20b of the curving section base member 20 is disposed in the space inside the channel-shaped section of the distal end fixing member 12, the protruding section 20b and the distal end fixing member 12 are linked by fitting pins 24 inserted from the positions facing both side surfaces of the distal end fixing member 12, and the curving section base member 20 can rotate about the common axis of those two pins 24 as a rotation center.

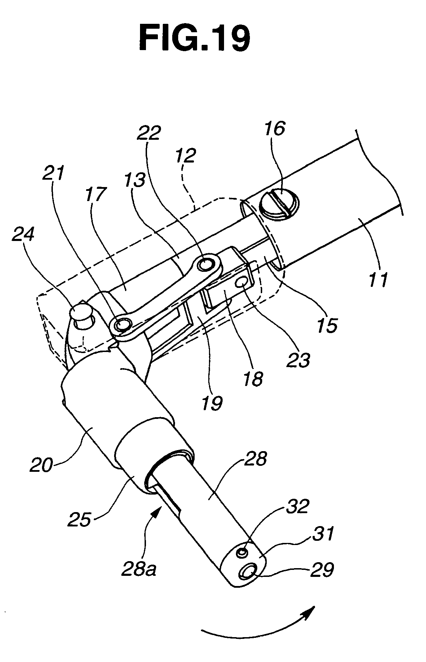

Therefore, as shown in Fig. 19, when the curving power transmission rod 15 is moved back and forth in the axial direction of the operation unit 3 by rotating the angle variation dial 6 of the operation unit 3, the curving section base member 20 will rotate about the pins 24 as a rotation center. Fig. 19 is a perspective view for explaining the internal structure of the distal end section, the internal structure being seen through the distal end fixing member 12 shown by a broken line indicating the omission of it. Pins 21, 22, 23, and 24 are made of stainless steel. The mechanism of moving the curving power transmission rod 15 back and forth in the axial direction of the operation unit 3 by rotating the angle variation dial 6 of the operation unit 3 will be described below.

Returning to Fig. 15, the cylindrical rotation section base member 25 is inserted into the cylindrical section 20a of the curving section base member 20 so that the cylindrical rotation section base member 25 can rotate about the axis of itself as a rotation center. The rotation section base member 25 has an opening at the distal end side and a bottom section at the proximal end side. A hole is formed in the bottom section at the proximal end side of the rotation section base member 25, and the distal end section of the rotation power transmission coil 17 is inserted into this hole and fixed therein by soldering, as described hereinabove.

The rotation power transmission coil 17 is fixed by soldering, as described hereinabove, to the rotation power transmission pipe 13 at the proximal end side and fixed to the rotation section base member 25 at the distal end side. The distal end section of the rotation power transmission coil 17 is inserted into the undersection on the proximal end side of the rotation section base member 25 and soldered thereto. The proximal end section of the rotation power transmission coil 17 is inserted into a step formed inside the distal end section of the rotation power transmission pipe 13 and soldered thereto. As a result, when the rotation power transmission pipe 13 is rotated about the axis of the rotation power transmission pipe 13 as a rotational center, the rotation power transmission coil 17 and rotation section base member 25 rotate similarly so that the rotation quantity of the rotation power transmission pipe 13 is transmitted to the treatment unit 4.

The rotation section base member 25 is made of stainless steel, and a wire retaining and receiving member 26 is inserted into the rotation section base member 25. The wire retaining and receiving member 26 is a cylindrical member made of stainless steel and having a flange section at the proximal end side thereof.

A hole through which the pulling wire 14 can be passed is provided in the bottom section on the proximal end side of the wire retaining and receiving member 26. A cylindrical wire retaining member 27 made of stainless steel is inserted into the cylindrical section on the distal end side of the wire retaining and receiving member 26. The wire retaining member 27 is a cylindrical member provided at the distal end section of the pulling wire 14 for preventing the pulling wire 14 from slipping.

The pulling wire 14 is passed through inside the cylinder of the wire retaining member 27, and the pulling wire 14 and wire retaining member 27 are fixed by soldering. Enabling the wire retaining member 27 to come into contact and be caught on the bottom section on the distal end side of the wire retaining and receiving member 26 allows the wire retaining and receiving member 26 to move toward the operation unit 3 when the pulling wire 14 is pulled toward the operation unit 3.

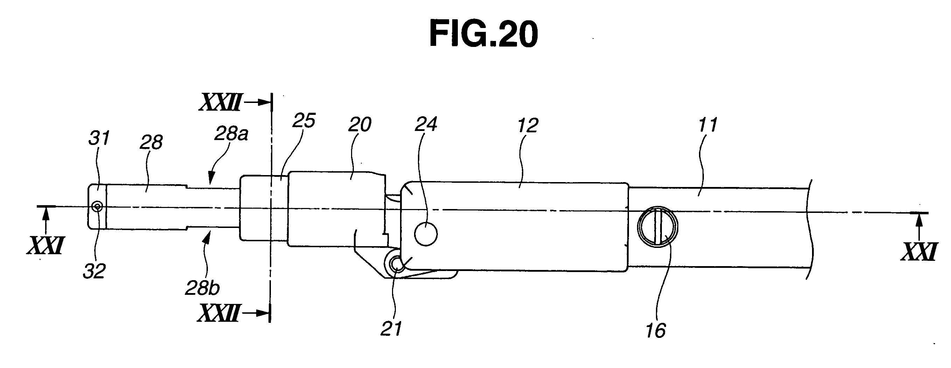

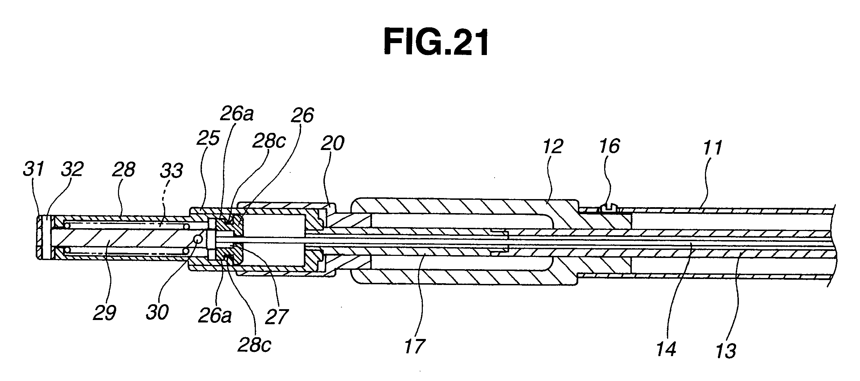

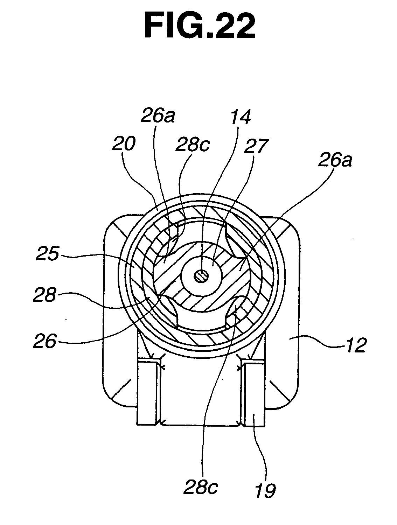

The clamping unit 8 comprising two clamping members for clamping a needle is provided in the distal end section of the treatment unit 4. The construction of the clamping unit 8 will be described below. Fig. 20 is a rear view of the distal end section in the needle driver 1 of the first embodiment. Fig. 21 is a sectional view along the XXI-XXI line in Fig. 20. Fig. 22 is a sectional view along the XXII-XXII line in Fig. 20.

Part of a movable clamping piece 28 made of stainless steel, which is one clamping member, is inserted into the rotation section base member 25. The movable clamping piece 28 has a substantially cylindrical shape having two notched sections 28a, 28b and is fixed to the wire retaining and receiving member 26 located inside the rotation section base member 25 with an adhesive, for example, an epoxy resin adhesive, in the end section on the proximal end side. The distal end section of the movable clamping piece 28 has a flat section for clamping the needle, and the plane of the flat section is perpendicular to the axis of the movable clamping piece 28 that has an almost cylindrical shape.

Furthermore, as shown in Fig. 21 and Fig. 22, a convex section 26a is formed in part of the cylindrical section of the wire retaining and receiving member 26. On the other hand, a convex section 28c also is formed in part of the inner peripheral surface on the distal end side of the movable clamping piece 28. After the convex sections respectively fit in the zones where the other convex section is not provided each other so as to prevent them from hitting, the convex sections are rotated about their axes so as to be superimposed in the axial direction of the distal end section and the wire retaining and receiving member 26 and movable clamping piece 28 are fixed with an adhesive as described hereinabove. With such a configuration, when the pulling wire 14 is pulled and moves toward the operation unit 3, the movable clamping piece 28 moves together with the wire retaining and receiving member 26 toward the operation unit 3.

A distal-end clamping piece attachment member 29 made of stainless steel is fixed with a pin 30 to the distal end section of the rotation section base member 25.

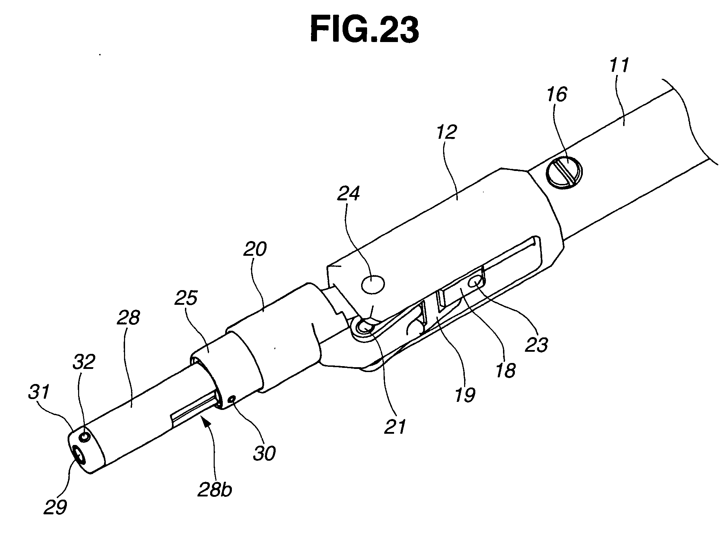

Fig. 23 to Fig. 26 illustrate the configuration of the clamping unit 8.

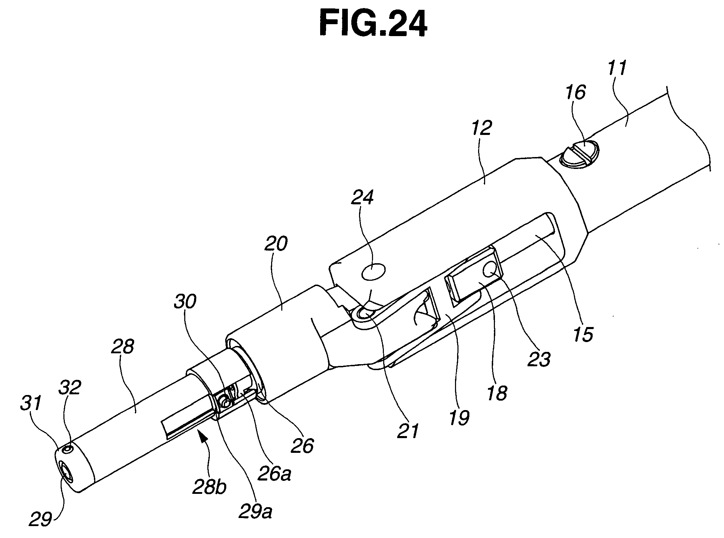

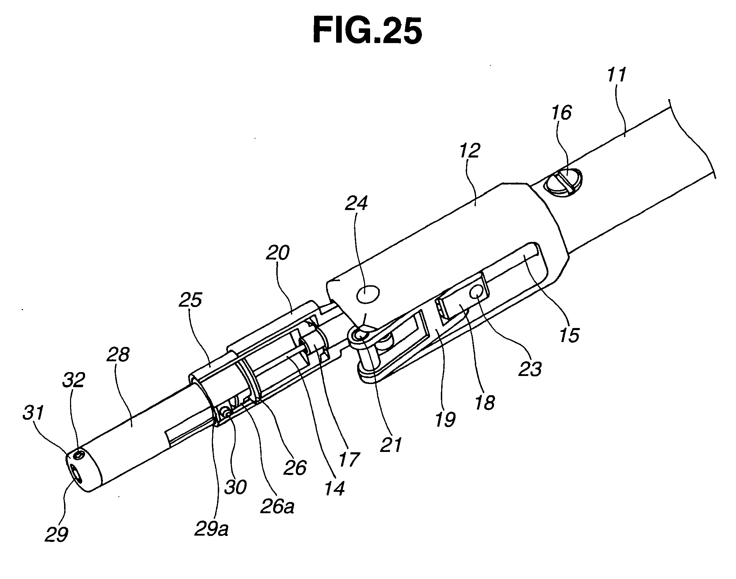

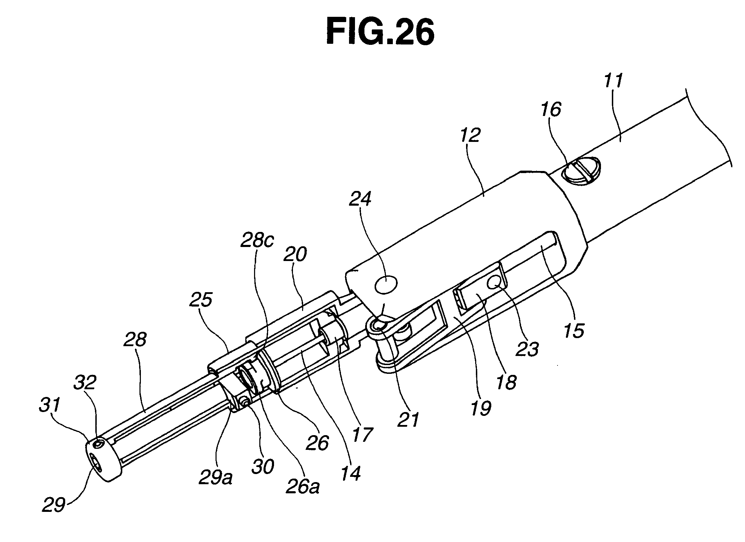

Fig. 23 is a perspective view illustrating the external appearance of the treatment unit in the needle driver. Fig. 24 is a perspective view illustrating the internal structure of the clamping unit in which the rotation section base member is omitted. Fig. 25 is a perspective view illustrating the internal structure of the clamping unit in which the rotation section base member and the curving section base member are partially omitted. Fig. 26 is a perspective view illustrating the internal structure of the clamping unit in which the rotation section base member, curving section base member, and movable clamping piece are partially omitted.

As shown in Fig. 15, Fig. 24, and Fig. 26, the distal-end clamping piece attachment member 29 has a T-shaped section at the proximal end side and a rod-shaped section at the distal end side. The T-shaped section located at the proximal end side has protruding sections 29a protruding in the direction perpendicular to the axis of the rotation section base member 25. The protruding sections 29a are slidably inserted into the notched sections 28a, 28b of the movable clamping piece 28, and the protruding sections 29a and the rotation section base member 25 are fixed with a pin 30 passing through the protruding sections 29a. The pin 30 is fixed to the rotation section base member 25 by laser welding at the end thereof.

A distal-end clamping piece 31 made of stainless steel and serving as one clamping member is fixed with a stainless steel pin 32 to the distal end section of the distal-end clamping piece attachment member 29. The pin 32 is fixed to the distal-end clamping piece 31 by laser welding in the end section thereof. The distal-end clamping piece 31 is of an annular shape and has a flat section parallel to the flat section of the distal end section of the movable clamping piece 28. Therefore, as described hereinabove, the needle is clamped so as to be sandwiched between the flat section of the distal-end clamping piece 31 and the flat section of the movable clamping piece 28 in response to the opening and closing action of the opening and closing lever 5.

The surfaces of the flat section of the distal-end clamping piece 31 and the flat section of the movable clamping piece 28, which are the clamping surfaces for clamping the needle are subjected to processing that prevents slipping. Examples of processing that prevents slipping include electric discharge processing, roulette processing, and blowing fine diamond powder onto a plated metal layer.

An inward flange is formed in the distal end section of the cylindrical movable clamping piece 28, and a stainless steel spring 33 is provided in a compressed state thereof, so as to be fit on the rod-like section of the distal-end clamping piece attachment member 29, between the inner surface of this inward flange and the side surface of the distal end of the protruding section 29a of the distal-end clamping piece attachment member 29 that faces this inner surface. Therefore, the spring 33 constitutes part of impelling means that constantly impels at least one of the two clamping members in the direction of bringing them into intimate contact with each other.

The action of the treatment unit 4 of the needle driver 1 having the above-described configuration will be described below.

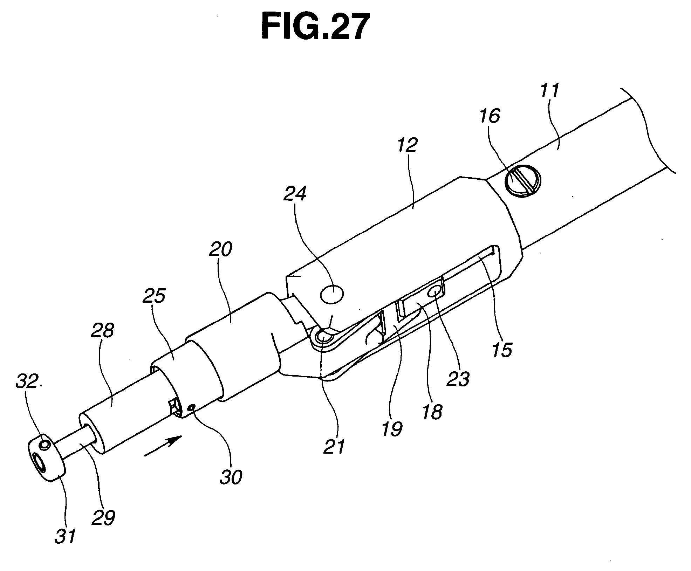

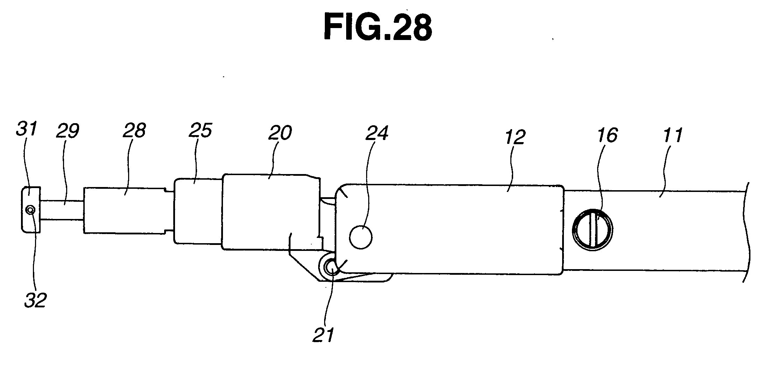

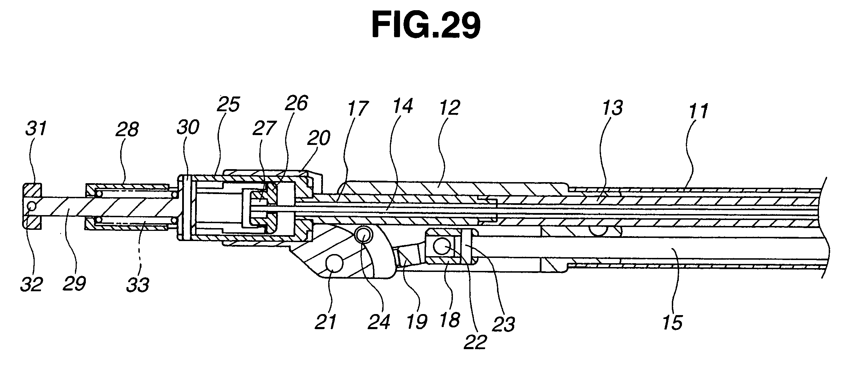

First, the opening and closing action of the clamping unit 8 will be explained. Fig. 27 to Fig. 29 illustrate the states where the clamping unit 8 of the treatment unit 4 of the needle driver 1 is open. Fig. 27 is a perspective view illustrating the state where the clamping unit 8 of the treatment unit 4 in the needle driver 1 is open. Fig. 28 is a rear view illustrating a state where the clamping unit 8 of the treatment unit 4 in the needle driver 1 is open. Fig. 29 is a sectional view illustrating a state where the clamping unit 8 of the treatment unit 4 in the needle driver 1 of the first embodiment is open.

Because the distal-end clamping piece attachment member 29 is fixed to the rotation section base member 25, the distal-end clamping piece 31 that is fixed to the distal-end clamping piece attachment member 29 has a fixed position with the rotation section base member 25. In other words, the distal-end clamping piece 31 has a fixed position with the curving section base member 20 in the long axis direction.

On the other hand, performing the operation of opening the opening and closing lever 5, that is, pressing the opening and closing lever 5 and pulling the pulling wire 14 makes it possible to move the movable clamping piece 28 that is fixed to and caught on the wire retaining and receiving member 26 on the side of the operation unit 3 toward the operation unit 3, that is, in the direction of going away from the distal-end clamping piece 31, and in this process the movable clamping piece 28 moves against the force applied in the direction of extending by the spring 33. Therefore, when the pulling wire 14 is pulled, the movable clamping piece 28 moves in the direction shown by an arrow in Fig. 27 by the distance corresponding to the reciprocating action of the pulling wire 14. Thus, the movable clamping piece 28, which is one clamping member, is moved in the direction of going away from the distal-end clamping piece 31 positioned in the distal end section of the treatment unit 4 by an opening operation of the opening and closing cycle against the impelling force created by the spring 33 and acting in the direction of bringing this clamping piece into intimate contact with the distal-end clamping piece 31, which is the other clamping member. In this process, as shown in Fig. 29, the spring 33 is in a state of greater compression than in a state in which the operation of opening the opening and closing lever 5 shown in Fig. 15 has not been performed, and when the opening and closing lever 5 is not pressed and the opening operation is not performed, the extension force of the spring 33 drags the pulling wire 14 toward the treatment unit 4 by the impelling force created by the spring 33 and acting in the direction of bringing the movable clamping piece 28 into intimate contact with the distal-end clamping piece 31. As a result, the needle positioned between the flat section of the distal-end clamping piece 31 and the flat section of the movable clamping piece 28 is clamped by the clamping unit 8.

The rotation action will be described below.

When the rotation dial 7 is rotated in a state where the needle is clamped or in a state where the needle is not clamped, the rotation power transmission pipe 13, which is an axial member, rotates about the axis thereof as a rotation center. As a result, the rotation power transmission coil 17 that is fixed to the rotation power transmission pipe 13 rotates, and the rotation section base member 25 that is fixed to the rotation power transmission coil 17 also rotates. Because the rotation power transmission pipe 13 rotates correspondingly to the rotation quantity of the rotation dial 7, the rotation quantity corresponding to the rotation quantity of the rotation dial 7 is transmitted to the treatment unit 4. As a result, the distal-end clamping piece 31 and movable clamping piece 28 constituting the clamping unit 8 rotate together in response to the rotation of the rotation section base member 25.

Furthermore because the movable clamping piece 28 and wire retaining and receiving member 26 are fixed and because the protruding sections 29a located in the distal-end clamping piece attachment member 29 are inserted into the notched sections 28a, 28b, the rotation section base member 25 and wire retaining and receiving member 26 rotate cooperatively together.

Furthermore, at this time, because the pulling wire 14 and the wire retaining member 27 fixed to the pulling wire 14 can slide with respect to the wire retaining and receiving member 26, even though the rotation section base member 25 rotates, the pulling wire 14 and the wire retaining member 27 do not rotate together with the rotation section base member 25.

The angle variation action will be described below.

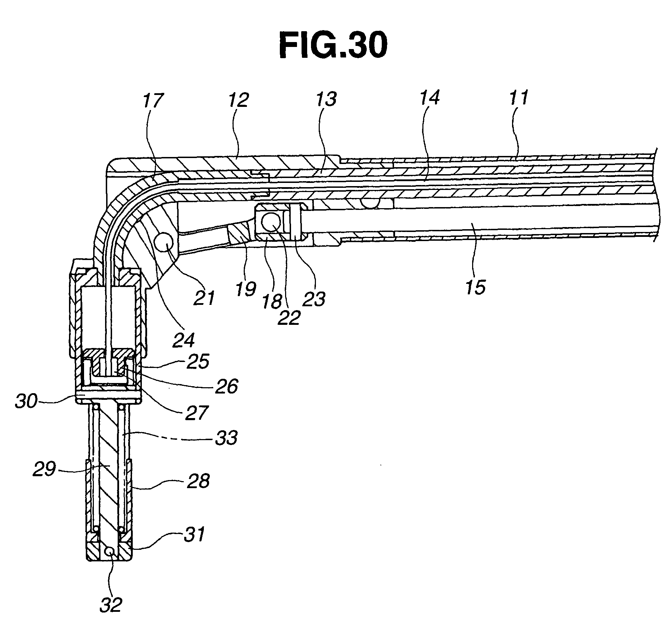

Fig. 30 is a sectional view of the treatment unit 4 where the treatment unit in the needle driver 1 of the first embodiment is curved as shown in Fig. 4. When the curving power transmission rod 15 is pulled toward the operation unit 3 from the state shown in Fig. 15 by the rotation of the angle variation dial 6, the curving section base member 20 is rotated about the pin 24 as a rotation center by the link joint 18 and H-shaped link 19.

When the curving power transmission rod 15 moves back and forth according to the rotation quantity of the angle variation dial 6, the curving amount, that is, the curving angle of the treatment unit 4 changes. As a result, the surgeon sets the treatment unit 4 at the desired angle with respect to the axis of the insertion unit 2 and conducts the treatment corresponding to the state of the procedure as described hereinabove.

The angle between the axis of the treatment unit 4, that is, the axis in the direction perpendicular to the clamping surfaces of the distal-end clamping piece 31 and movable clamping piece 28 of the clamping unit 8 and the axis of the insertion unit 2 can be varied within a range from 0 degree to about 92 degrees, and the angle variation range can be variously changed by adjusting the mutual positions of the components.

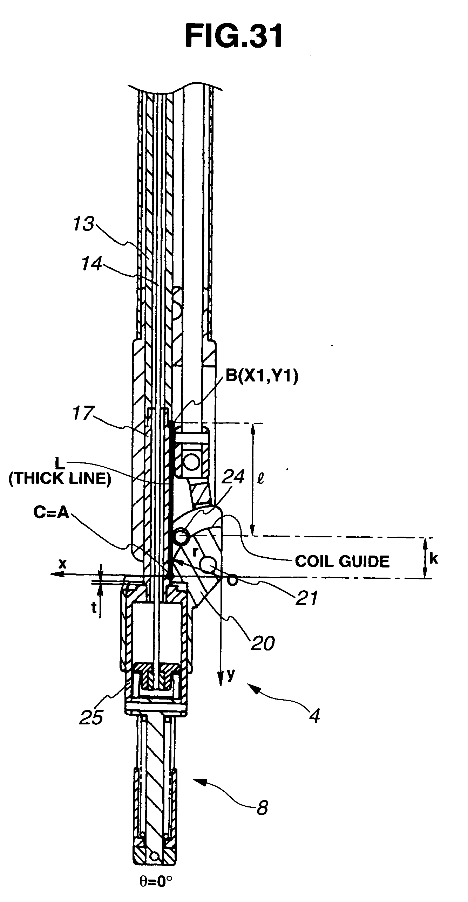

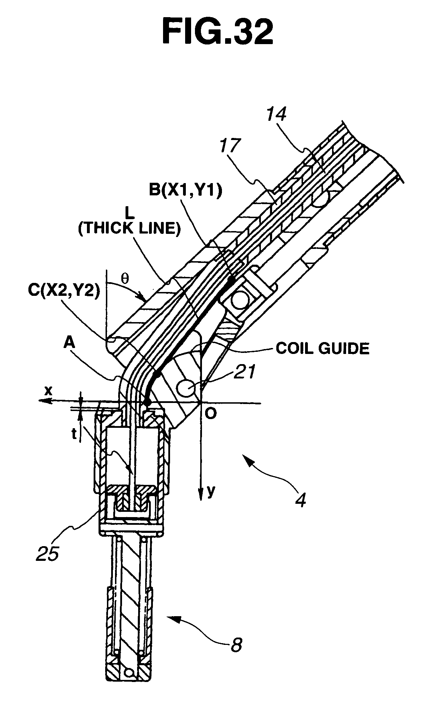

The curve rotary center position of the treatment unit 4 of the needle driver 1 will be described below with reference to Fig. 31 through Fig. 35.

In Fig. 31 through Fig. 33,

1 stands for a distance between the surface where the rotation power transmission coil 17 and the rotation power transmission pipe 13 are joined and the central axis of the pin 24 (curve rotary center) in the lengthwise direction of the insertion unit;

r stands for a radius of the coil guide provided in the curving section base member 20;

k stands for a distance to the rotary center o of the coil guide in the horizontal direction and vertical direction of the pin 24;

the point A (r, 0) is a point where the rotation power transmission coil 17 is in contact with the distance end side of the coil guide provided in the curving section base member 20;

the point B (X1, Y1) is a point on the surface where the rotation power transmission coil 17 and the rotation power transmission pipe 13 are joined, the point being the closest to the curving power transmission rod 15;

the point C (X2, Y2) is a point where the rotation power transmission coil 17 and the coil guide provided at the curving section base member 20 are in contact;

θ is an angle between the insertion unit and distal end section (0 degree to -90 degrees); curving angle;

L stands for a length of the rotation power transmission coil 17 along point A - point C - point B;

t stands for a gap between the curving section base member 20 and rotation section base member 25.

The length L changes as the treatment unit 4 is curved. This change in length is represented by the gap t between the curving section base member 20 and rotation section base member 25. When the change of the gap t is large, the following conditions are encountered.

(1) The needle clamping section at the distal end moves back and forth in the long axis direction of the clamping unit, thereby impeding the operation.
(2) If the length L further increases from the state where the gap t became 0, the rotation section base member 25 is pressed against the curving section base member 20. As a result, the power required for rotation is increased.
(3) If the gap t increases, the clamping power decreases, and if the gap further increases, the clamping section opens.

Because of the above-described conditions (1) to (3), the change of the length L caused by curving has to be greatly reduced.

Accordingly, in the present embodiment, the curve rotary center position of the treatment unit 4 is set in the above-described manner and the change in the length L caused by curving is suppressed.

That is, the length L at a curving angle θ is found by setting r, 1, and k. Then, r, 1, and k are adjusted so that the change of L decreases (coordinate values X1, Y1, X2, Y2 of point B and point C are used in the process of finding L).

Coordinate values of point B

$$X1 = (r-k)\cos\theta + 1\sin\theta + k$$

$$Y1 = (r-k)\sin\theta - 1\cos\theta - k$$

Coordinate values of point C

$$X2 = (r_2-Y1Y2)/X1$$

$$Y2 = [r_2Y1 + \{r_4Y1_2-(X1_2+Y1_2)(r_4-r_2X1_2)\}_{1/2}] / (X1_2+Y1_2)$$

$$(X1 \geq 0)$$

or

$$Y2 = [r_2Y1 - \{r_4Y1_2-(X1_2+Y1_2)(r_4-r_2X1_2)\}_{1/2}] / (X1_2+Y1_2)$$

$$(X1 < 0)$$

Length L on the inner side of the coil

$$L = racos(X2/r) + \{(X1-X2)_2 + (Y1 - Y2)_2\}_{1/2}$$

(actually, it is a function of $\theta$).

The change of L is reduced by adequately setting the rotary center position of the curve accordingly to the above-described formulas.

For example, if

k = $\pi$r/4 (the distance between the radial center O of the coil guide and the curve rotary center is $\sqrt{2}\pi$r/4), then the change of L becomes 0 when $\theta$ is 0° and -90°.

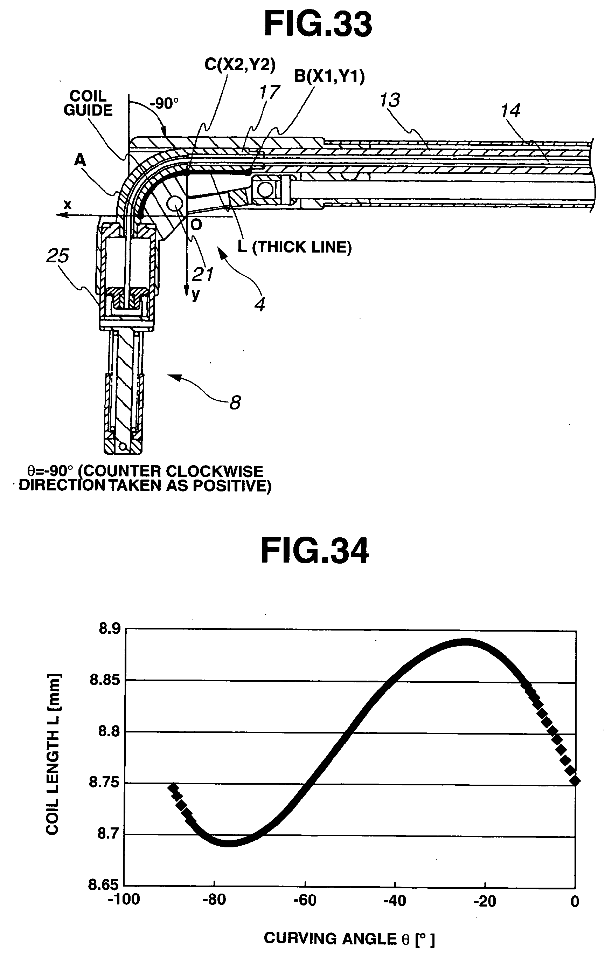

Under conditions of r = 3 [mm] and 1 = 6.4 [mm], if k = 2.3561, then the change of L becomes zero when $\theta$ is 0° and -90°, the change of L becomes as shown in Fig. 34 when $\theta$ changes from 0° to -90°, and the change of L during curving in this range can be confined to about 0.2 [mm].

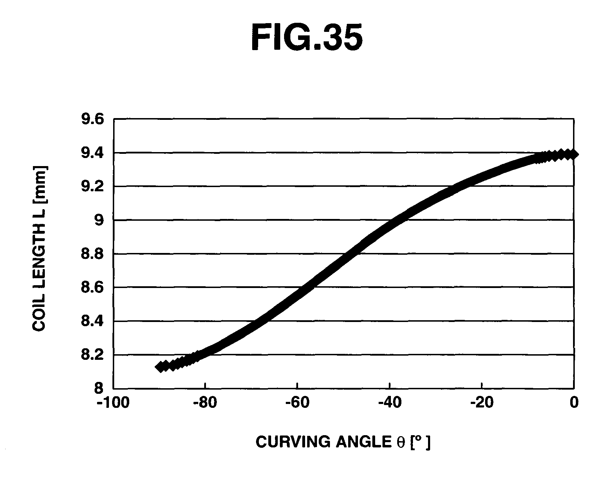

For reference, the change of L in the case where k = 3 is shown in Fig. 35. The change of length L in the curving range from 0° to 90° in this case becomes as large as 1.3 [mm], and the rotary center position is clearly important.

The operation unit in the first embodiment of the invention will be explained below.

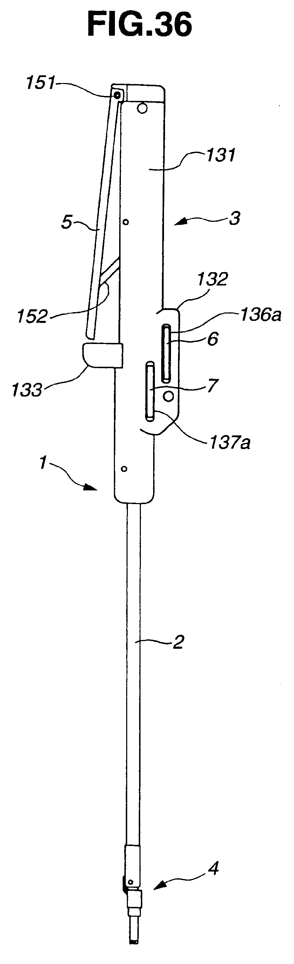

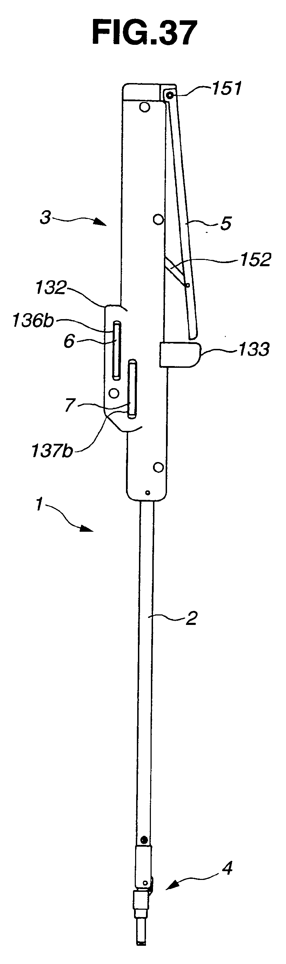

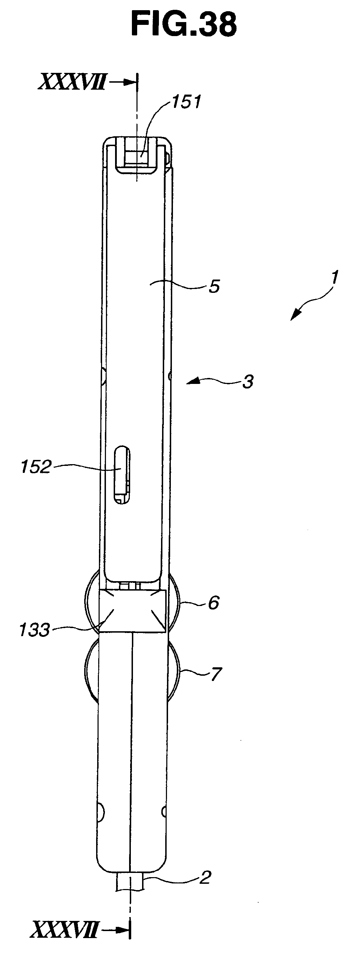

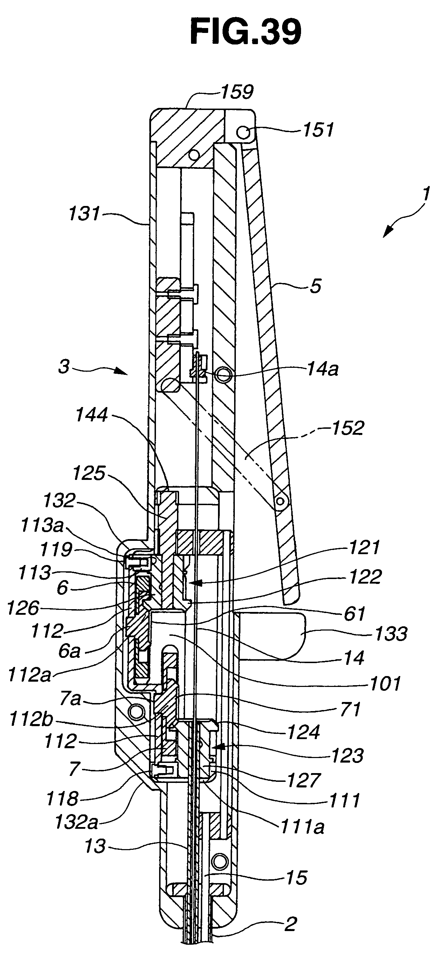

Fig. 36 is a front view of the needle driver of the first embodiment. Fig. 37 is a rear view of the needle driver of the first embodiment. Fig. 38 is a main side view illustrating the operation unit 3 in the needle driver of the first embodiment from one side (left side) thereof. Fig. 39 is a main-part sectional view in which the operation unit 3 is cut along the XXXVII-XXXVII line in Fig. 38; in this view, the operation unit 3 is viewed from the rear surface side.

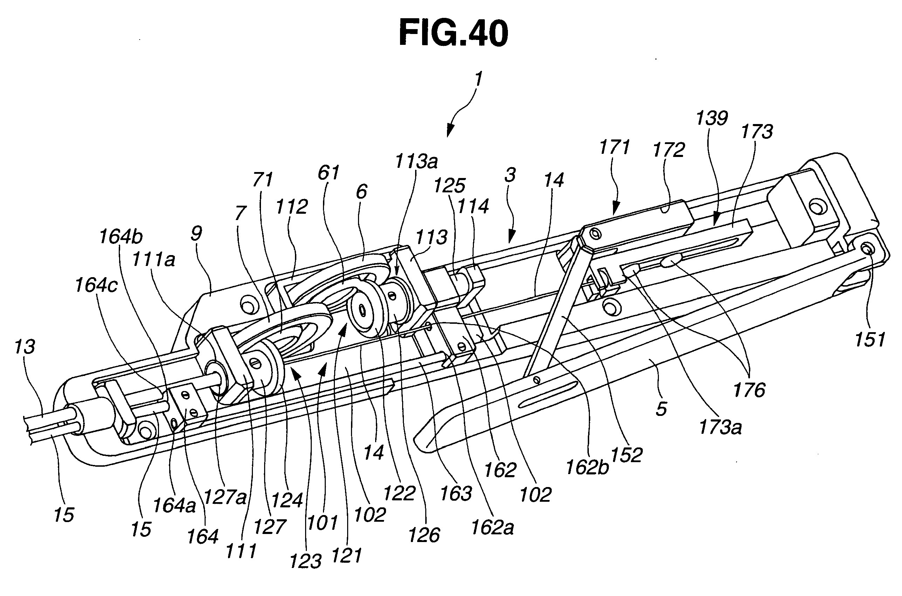

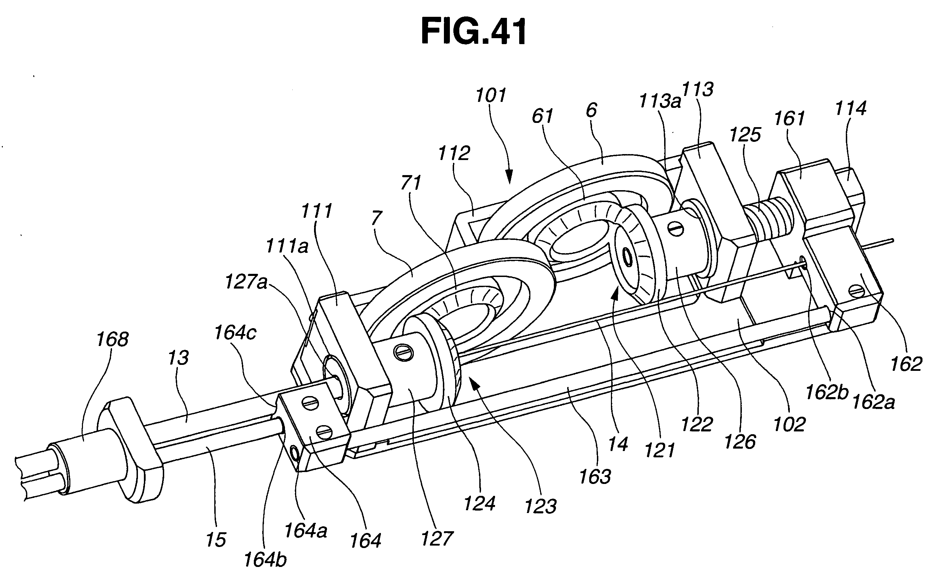

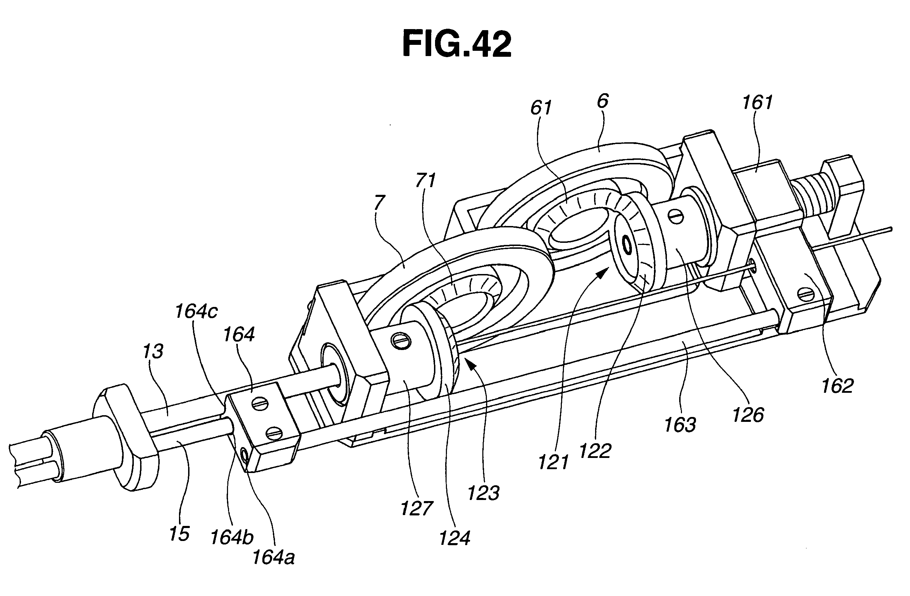

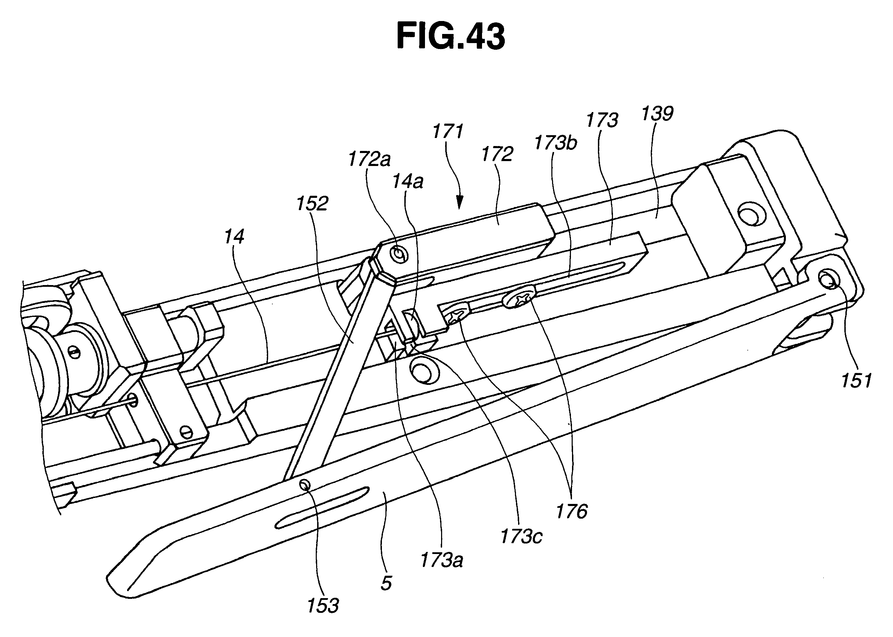

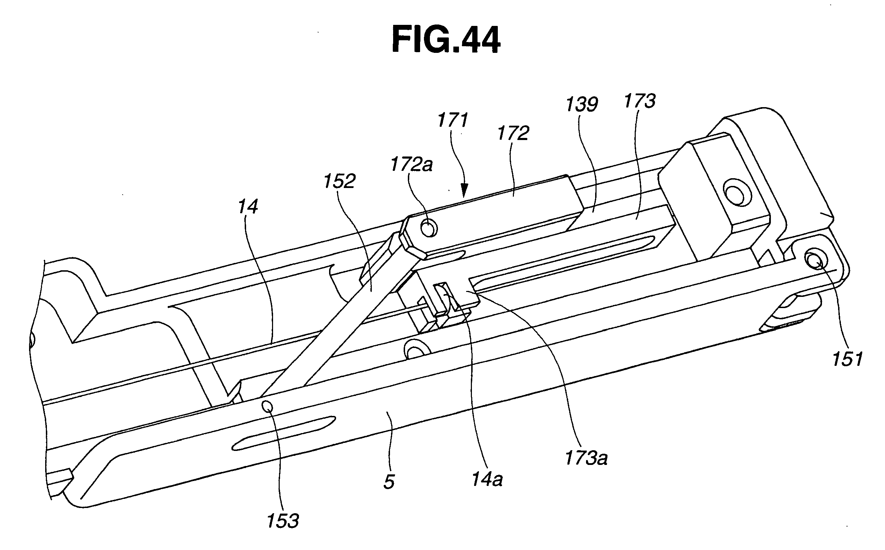

Furthermore, Fig. 40 is a main-part perspective view illustrating the rotation dial 7, angle variation dial 6, and opening and closing lever 5 in the needle driver of the above-described embodiment, wherein the external section of the operation unit 3 is omitted. Fig. 41 is a main-part enlarged perspective view illustrating the rotation dial 7 and angle variation dial 6 in the needle driver of the above-described embodiment, wherein the external section of the operation unit 3 is omitted; this figure shows a state where the treatment unit 4 is curved. Fig. 42 is a main-part enlarged perspective view illustrating the rotation dial 7 and angle variation dial 6 in the needle driver of the above-described embodiment, wherein the external section of the operation unit 3 is omitted; this figure shows the initial state of the treatment unit 4 (no curving). Fig. 43 is a main-part enlarged perspective view illustrating the opening and closing lever 5 in the needle driver of the above-described embodiment, wherein the external section of the operation unit 3 is omitted; this figure shows a state where the treatment unit 4 is closed. Fig. 44 is a main-part enlarged perspective view illustrating the opening and closing lever 5 in the needle driver of the above-described embodiment, wherein the external section of the operation unit 3 is omitted; this figure shows a state where the treatment unit 4 is open.

As described hereinabove, the needle driver as a needle-holding device, which is a surgical instrument of the present embodiment, comprises as the main components the insertion unit 2, operation unit 3, and treatment unit 4 provided at the distal end of the insertion unit 2. The operation unit 3 comprises operation mechanisms for various actions in the treatment unit 4 and is provided integrally at the proximal end side of the insertion unit 2 (see Fig. 36 and Fig. 37).

The operation unit 3 in the needle driver 1 of the present embodiment will be described below.

As shown in Fig. 36 to Fig. 40, the operation unit 3 is provided integrally at the proximal end side of the insertion unit 2 on the same axis as the long axis of the insertion unit 2 and is covered with an external cover member 131 in the form close to that of a rectangular parallelepiped.

At one side of the operation unit 3, the opening and closing lever 5 capable of rotating about the proximal end section of the operation unit 3 is provided so that it can swing toward one side of the operation unit 3.

The opening and closing lever 5 is a lever serving as an opening and closing operation unit for performing the operation of opening and closing the treatment unit 4. This lever is provided so that it can swing about a shaft 151 as a rotation center, this shaft being provided at one side of an end section 159 provided in the proximal end section of the operation unit 3, and the free end section of the opening and closing lever extends from the proximal end side of the operation unit 3 toward the distal end side thereof.

Furthermore, the below-described link 152 is installed between the middle zone of the free end section of the lever and an opening and closing operation mechanism 171 contained in the external cover member 131. An impelling force of the above-described spring 33 is applied in the direction of going away from the external cover member 131 via the link 152.

Furthermore, a convex section 133 for placing the surgeon's finger is provided in a condition of protruding toward one side (to the left, as shown in Fig. 36) in the vicinity of the distal end section of the opening and closing lever 5 in the middle zone in the long axis direction of the external cover member 131 and on the extension line of the free end section of the opening and closing lever 5. The height of the convex section 133 for placing a finger in the protrusion direction thereof is set to an adequate value correspondingly to the swinging range of the free end section of the opening and closing lever 5, that is, correspondingly to the maximum distance position of the distal end section. More specifically, in the present embodiment, the height of the convex section 133 for placing a finger in the protrusion direction thereof is set to a plane substantially identical to that of the maximum distance position of the distal end section of the opening and closing lever 5. Furthermore, in the present embodiment, it is assumed that before and after the surgeon operates the opening and closing lever 5 with the prescribed finger (in the needle driver 1 of the present embodiment, a first finger is assumed) this finger is temporarily placed on the convex section 133 for placing a finger and the operation unit 3 is grasped. When the opening and closing lever 5 is not operated, the needle driver 1 can be grasped with better stability by the first finger placed on the convex section 133 for placing a finger and the middle finger that came into contact with an engagement surface 132a of the below-described convex section 132 and a reliable procedure can be implemented.

Returning to Fig. 36, a convex section 132 having provided therein the below-described dial operation unit 101 is provided in a condition of protruding to the other side (to the right, as shown in Fig. 36) in the middle zone in the long axis direction of the external cover member 131.

On the front surface side (see Fig. 36) of the convex section 132, an open groove 136a for exposing the angle variation dial 6 and an open groove 137a for the rotation dial 7 are provided along the long axis direction of the external cover member 131. Those open groove 136a and open groove 137a are disposed in the rail-like manner along the long axis direction of the external cover member 131, the former being in a position closer to the other side surface (right side surface, as shown in Fig. 36) of the operation unit 3 and the latter being in a position closer to the center. The open groove 136a is disposed on the proximal end side of the operation unit 3 with respect to the open groove 137a.

The convex section 132 is provided in a protruding condition substantially on the side of the rear surface of the convex section 133 for placing a finger located at the outer peripheral surface of the operation unit 3 and serves as a finger placement section having an engagement surface 132a that comes into contact with one engaged finger (in the present embodiment, a middle finger is assumed) of the fingers gripping the operation unit.

The engagement surface 132a is formed as a slanted surface with respect to the operation unit 3 and is formed as a surface most suitable for gripping when the operation unit 3 is gripped by placing a finger operating the opening and closing lever 5 (in the present embodiment, a first finger is assumed) on the convex section 133 for placing a finger and bringing the middle finger into contact with the engagement surface 132a.

On the other hand, an open groove 136b for exposing the angle variation dial 6 and an open groove 137b for the rotation dial 7 are provided in the positions opposite the open groove 136a and 137a, respectively, on the rear surface side (see Fig. 37) of the convex section 132.

The angle variation dial 6 and rotation dial 7 are installed so that part of circumferential surface thereof is exposed from those open grooves 136a, 136b and open grooves 137a, 137b, respectively.

Thus, of the angle variation dial 6 and rotation dial 7 installed so as to be exposed from those open grooves 136a, 136b and open grooves 137a, 137b, respectively, the angle variation dial 6 is disposed in a position on the proximal end side of the operation unit 3 on the outer side of the operation unit 3.

In the present embodiment, the angle variation dial 6 and rotation dial 7 are provided between the convex section 133 for placing a finger and the engagement surface 132a of the convex section 132 within a movement range of the finger operating the two dials 6, 7 (in the present embodiment, an index finger is assumed).

The angle variation dial 6 as an angle variation operation unit for performing the operation of changing the angle of the extension direction of the treatment unit 4, the rotation dial 7 serving as a rotational operation unit for performing the operation of rotating the treatment unit 4, and the peripheral components thereof will be explained below with reference to Fig. 39 to Fig. 42.

Closer to the side of the insertion unit 2 from the middle zone in the long axis direction of the operation unit 3, that is, in the convex section 132, there are provided the rotation dial 7 constituting the rotational operation mechanism of the treatment unit 4 and the angle variation dial 6 constituting the angle variation operation mechanism of the treatment unit 4.

The rotation dial 7 and angle variation dial 6 are together assembled in the dial operation unit 101 and provided in the prescribed positions such that they are free to rotate.

With consideration for operability, in the present embodiment, the outer peripheral surfaces of the angle variation dial 6 and rotation dial 7 are subjected to roulette machining.

In the dial operation unit 101, the rotation dial 7, angle variation dial 6, and bevel gear units 123, 121 that are engaged with the rotation dial 7 and angle variation dial 6, respectively, are assembled in a holding unit comprising a base 102 formed of aluminum or the like, protruding holding-pieces 111, 113, and 114 that are provided perpendicularly protruding on the base 102, and a member 112 linking the protruding holding-pieces 111 and 113.

The base 102 is a hard plate-like member shaped as a substantially rectangular shelf. The protruding holding-piece 111 is provided integrally and vertically with a screw or the like at one end of the base, and the protruding holding-piece 113 is similarly provided integrally and vertically with a screw at the other end of the base. Furthermore, the protruding holding-piece 111 and protruding holding-piece 113 are linked with the link member 112 having formed in the steps corresponding to the installation positions of the angle variation dial 6 and rotation dial 7. The link member 112 is fixedly attached to the protruding holding-pieces 111, 113 with screws 118, 119, respectively.

In the angle variation dial 6 and rotation dial 7, an inner bevel gear 61 and an inner bevel gear 71 are provided coaxially with the angle variation dial 6 and rotation dial 7, respectively, so that the gears rotate integrally with the angle variation dial 6 and rotation dial 7.

A rotation shaft 6a and a rotation shaft 7a that are common rotation shafts for the angle variation dial 6 and rotation dial 7 are formed integrally with the inner bevel gear 61 and inner bevel gear 71, and those rotation shaft 6a and rotation shaft 7a are freely rotatably pivotally supported in a shaft hole 112a and a shaft hole 112b, respectively, those holes being formed in the link member 112.

A bevel gear 122 located in the bevel gear unit 121 is engaged with the inner bevel gear 61, and a bevel gear 124 located in the bevel gear unit 123 is engaged with the inner bevel gear 71.

The bevel gear 122 is integrally and fixedly mounted on the distal end section of a large-diameter rotation shaft 126, and the proximal end section of the rotation shaft 126 is freely rotatably supported by a bearing section 113a formed in the protruding holding-piece 113. On the other hand, the bevel gear 124 is integrally and fixedly mounted on the distal end section of a large-diameter rotation shaft 127, and the proximal end section of the rotation shaft 127 is freely rotatably supported by a bearing section 111a formed in the protruding holding-piece 111.

A power conversion mechanism for transmitting the rotation power of the angle variation dial 6 to the treatment unit 4, and a power conversion mechanism for transmitting the rotation power of the rotation dial 7 to the treatment unit 4 will be described below.

First, the power conversion mechanism relating to the angle variation dial 6 will be described.

In the center of the proximal end surface of the rotation shaft 126 freely rotatably supported by the bearing section 113a in the bevel gear unit 121, a worm screw 125 is integrally provided in a protruding condition coaxially with the rotation shaft 126. The other end of the worm screw 125 is freely rotatably supported by the protruding holding-piece 114 that is provided vertically from the base 102.

A movable member 161 provided so that it can freely move in the long axis direction of the operation unit 3 due to engagement with the worm screw 125 is engaged with the worm screw 125. This movable member 161 is a nut member with a rectangular outer circumferential surface, and the bottom side thereof freely slidably abuts against the base 102. Furthermore, the movable member 161 is engaged by appropriate friction with the worm screw 125 and slides in the long axis direction of the operation unit 3 in an appropriate fashion with respect to the operation unit 3 over the base 102, following the rotation of the worm screw 125. Thus, the worm screw 125 and movable member 161 have a cam - cam follower relationship.

The above-described angle variation dial 6, inner bevel gear 61, and worm screw 125 are configured with the prescribed reduction ratio, and the rotation of the angle variation dial 6 is converted into the movement of the movable member 161 with an appropriate reduction ratio.

Furthermore, a protruding piece 162 is provided in a protruding condition at one side of the movable member 161 and this protruding piece moves in the long axis direction of the operation unit 3 following the movement of the movable member 161. An engagement hole 162a is formed in the distal end section of the protruding piece 162, and one end of a link rod 163 extending toward the insertion unit 2 is joined with and fixedly attached to the engagement hole 162a. Furthermore, a through hole 162b for a pulling wire 14 is drilled on the extension of the rotation shaft of the bevel gear 124 which is the proximal end side of the protruding piece 162. The pulling wire 14 provided in an extending condition from the insertion unit 2 and can freely move inside the through hole 162b, regardless of the position and movement of the protruding piece 162.

On the other hand, the other end of the link rod 163 is joined with and fixedly attached to an engagement hole 164a formed in the link section 164 with the above-described curving power transmission rod 15. The link rod 163 is joined from the proximal end side of the operation unit 3 to one end of the link section 164, whereas at the other end, the curving power transmission rod 15 is joined with and fixedly attached to an engagement hole 164b. As a result, the link section 164 moves in the long axis direction of the operation unit 3 via the protruding piece 162 and link rod 163 in interlocking with the movement of the movable member 161, that is, the curving power transmission rod 15 moves in the long axis direction of the operation unit 3 following the rotation of the angle variation dial 6.

A guide groove 164c that is to be engaged with the above-described rotation power transmission pipe 13 is formed in the link section 164, and the link section 164 moves in the long axis direction of the operation unit 3, while being guided by the rotation power transmission pipe 13.

Fig. 41 illustrates a state where the movable member 161 is positioned at the very end of the proximal side of the operation unit 3; in this state, the treatment unit 4 is curved. Fig. 42 illustrates a state where the movable member 161 is positioned at the very end of the distal side of the operation unit 3; in this state, the treatment unit 4 is not curved.

Here, the action of the treatment unit 4 in response to the operation of the power conversion mechanism relating to the angle variation dial 6 and the operation of the angle variation dial 6 will be described from the standpoint of the surgeon.

When the surgeon rotates the angle variation dial 6, the inner bevel gear 61, bevel gear unit 121, worm screw 125, movable member 161, and protruding piece 162 move according to the rotation quantity of the angle variation dial 6 as described hereinabove and the curving power transmission rod 15 moves (back and forth) in the long axis direction of the operation unit 3. Because of the back and forth movement of the curving power transmission rod 15, the curving quantity of the treatment unit 4, that is, the curving angle, changes (see Fig. 4). Therefore, the surgeon, as described above, can conduct the treatment by setting the treatment unit 4 at the desired angle with respect to the axis of the insertion unit 2 correspondingly to the state of the procedure.

The power conversion mechanism relating to the rotation dial 7 will be described below.

Returning to Fig. 40, as described hereinabove, the bevel gear 124 is engaged with the inner bevel gear 71 that rotates integrally with the rotation dial 7, the bevel gear 124 is integrally and fixedly attached to the distal end section of the large-diameter rotation shaft 127, and the proximal end section of the rotation shaft 127 is freely rotatably supported in the bearing section 111a formed in the protruding holding-piece 111. One end of the rotation power transmission pipe 13 is coaxially and integrally attached to the proximal end surface of the rotation shaft 127, and the rotation power transmission pipe 13 is also integrally rotated following the rotation of the bevel gear 124.

An insertion hole 127a for passing the pulling wire 14 is formed in the center of the rotation shaft 127. The distal end section of the rotation power transmission pipe 13 extending from the insertion unit 2 is inserted into the insertion hole 127a, and the distal end section of the rotation power transmission pipe 13 is fixed inside the insertion hole 127a with a screw threaded in from the outer peripheral surface of the rotation shaft 127. Thus, the pulling wire 14 that extends from the insertion unit 2 inside the rotation power transmission pipe 13 is exposed from the distal end surface of the rotation power transmission pipe 13 and further extends toward the below-described opening and closing operation mechanism 171. The pulling wire 14 provided in a condition of extending from the insertion unit 2 can freely move the rotation power transmission pipe 13, regardless of whether the rotation shaft 127 is present or absent.

Here, the action of the treatment unit 4 caused by the operation of the power conversion mechanism relating to the rotation dial 7 and the operation of the rotation dial 7 will be described below from the standpoint of the surgeon.

If the surgeon rotates the rotation dial 7 in a state where a needle is clamped or in a state where the needle is not clamped in the treatment unit 4, the inner bevel gear 71 and bevel gear 124 move and the rotation power transmission pipe 13 rotates. As a result, the rotation power transmission pipe 17 that is fixed to the rotation power transmission pipe 13 rotates and the rotation section base member 25 that is fixed to the rotation power transmission pipe 17 also rotates. Therefore, the rotation power transmission pipe 13 rotates correspondingly to the rotation quantity of the rotation dial 7, and the rotation power of the rotation dial 7 is transmitted to the treatment unit 4. As a result, the distal-end clamping piece 31 and the movable clamping piece 28 constituting the clamping unit 8 rotate together with the rotation section base member 25.

The opening and closing lever 5 serving as an opening and closing operation unit for performing the operations of opening and closing the treatment unit 4 and the peripheral components thereof will be explained below with reference to Fig. 39 to Fig. 44.

As described above, the opening and closing lever 5 performing the operations of opening and closing the treatment unit 4 is provided so that it can freely swing about the shaft 151 at one side of the operation unit 3. The opening and closing lever 5 is a lever serving as an opening and closing operation unit for performing the operations of opening and closing the treatment unit 4. This lever is provided so that it can freely swing about the shaft 151 as a rotation center, this shaft being provided at one side of the end section 159 where the proximal end section of the operation unit 3 is provided. The free end section of the lever extends from the proximal end section of the operation unit 3 toward the distal end side.

Furthermore, in the middle zone of the free end section, the below-described link 152 is provided between the free end section and the opening and closing operation mechanism 171 contained in the external cover member 131, and an impelling force of the above-described spring 33 is applied in the direction of going away from the external cover member 131 via the link 152.

The opening and closing operation mechanism 171 comprises: an opening and closing operation base member 172 provided so that it can freely swing in the long axis direction of the operation unit 3 inside the proximal end side of the operation unit 3; a pulling wire holding member 173 that serves to hold an engagement piece 14a fixedly attached to the pulling wire 14 in the end section of the pulling wire 14 and moves in response to the movement of the opening and closing operation base member 172; the link 152 provided between the opening and closing operation base member 172 and the middle zone of the opening and closing lever 5; and the like.

The opening and closing operation base member 172 has a shape close to that of a rectangular parallelepiped and is installed such that it can freely swing in a guide groove 139 formed in the long axis direction inside the proximal end side of the operation unit 3. Furthermore, the pulling wire holding member 173 serves to hold engagement piece 14a of the pulling wire 14 of an angular rod shape and has a long hole 173b formed in the long axis direction thereof. The pulling wire holding member 173 is assembled with one side of the opening and closing operation base member 172 with a screw 176 via this long hole 173b. When the pulling wire holding member 173 is assembled with the opening and closing operation base member 172, the adjustment can be conducted so as to obtain a maximum tension of the pulling wire 14. Thus, when the pulling wire holding member 173 is attached, the position of the pulling wire holding member 173 is adjusted so as to absorb the spread in the length of the pulling wire 14 and provide the pulling wire 14 with an appropriate deflection.

A holding section 173a for clamping the engagement piece 14a in the pulling wire 14 is formed at the distal end side of the opening and closing operation base member 172. An opening 173c is formed in one surface of the holding section 173a in the short axis direction of the operation unit 3, and after the engagement piece 14a of the pulling wire 14 has been inserted from this opening 173c, the movement of the engagement piece 14a in the long-axis direction is controlled.

The link 152 is provided so that it can freely move about the shaft 172a provided at the distal end side of the opening and closing operation base member 172 and also about the shaft 153 provided in the middle zone of the free end section of the opening and closing lever 5. As a result, the opening and closing operation base member 172 slides on the guide groove 139 via the link 152 in response to the swinging of the opening and closing lever 5 about the shaft 151 as a rotation center.

On the other hand, the above-described spring 33 applies an impelling force to the pulling wire 14 such that the pulling wire 14 is pulled toward the insertion unit 2. As a result, the opening and closing operation base member 172 is impelled toward the insertion unit 2. Furthermore, the opening and closing lever 5 is impelled by this impelling force via the link 152 in the direction such that the free end section thereof goes away from the external cover member 131.

When the opening and closing lever 5 thus impelled is pressed down against this impelling force, the opening and closing operation base member 172 is displaced toward the proximal end side of the operation unit 3 via the link 152, and a tension force acting toward the proximal end side of the operation unit 3 is applied to the pulling wire 14 via the pulling wire holding member 173, following the displacement of the opening and closing operation base member 172. The pulling wire 14 to which this tension force is applied moves appropriately toward the distal end side after a suitable play.

That is, the opening and closing lever 5 is usually disposed in a location such that the free end section thereof is apart from the external cover member 131 by the impelling force of the spring 33. Furthermore, when the surgeon presses the free end section down toward the operation unit 3 when the device is used, the pulling wire 14 moves toward the proximal end section of the operation unit 3 after a suitable play and the treatment unit 4 opens following this movement of the pulling wire 14.

Fig. 43 shows the opening and closing lever 5 in the usual state thereof; at this time, the treatment unit 4 is closed. Fig. 44 shows the state in which the opening and closing lever 5 is pressed down; at this time, the treatment unit 4 assumes the opened state.

The operation of the treatment unit 4 following the operation of the opening and closing lever 5 will be described below from the standpoint of the surgeon.

When the surgeon presses down, as shown in Fig. 44 (the press-down state shown in Fig. 4), the opening and closing lever 5 that is in the usual state (the initial state shown in Fig. 43), the opening and closing operation base member 172 shifts toward the proximal end side of the operation unit 3 via the link 152, in response to this displacement, the pulling wire 14 is pulled and moved toward the proximal end side of the operation unit 3, and the treatment unit 4 is opened (see Fig. 3). When the surgeon releases the pressing of the opening and closing lever 5 after a suturing needle has been appropriately clamped with the open treatment unit 4, the pulling wire 14 is pulled toward the side of the insertion unit 2 by the impelling force of the spring 33 and the opening and closing lever 5 again swings via the link 152 in the direction of going away from the external cover member 131.

The surgeon can thereafter perform the operation of curving or the operation of rotating of the treatment unit 4 in a desired manner by rotating the angle variation dial 6 or rotation dial 7.

Furthermore, as described above, in the needle driver 1 of the present embodiment, a convex section 133 for placing a finger is formed in the vicinity of the distal end of the opening and closing lever 5, and when the surgeon does not operate the opening and closing lever 5, the finger relating to the opening and closing lever 5 can be brought into contact with the convex section 133 for placing a finger. Bringing the finger into contact with the convex section 133 makes it possible to prevent reliably the opening and closing lever 5 form being inadvertently operated. Furthermore, because the operation unit 3 can be gripped more reliably, the operation of rotating the angle variation dial 6 or rotation dial 7 can be executed with higher stability.

The following effect can be obtained with the needle driver 1 of the present embodiment.

As described above, in the needle driver 1 of the present embodiment, the operation unit 3 can be gripped reliably by the first finger placed on the convex section 133 for placing a finger and the middle finger engaged with the engagement surface 132a of the convex section 132. Therefore, the operation of rotating the angle variation dial 6 or rotation dial 7 can be executed with higher stability.

That is, the surgeon can grip the needle driver 1 almost as a pen and can grip the operation unit 3 reliably and with high stability. Furthermore, the three operations of opening and closing, curving, and rotating in the treatment unit 4 can be conducted skillfully by operating the opening and closing lever 5, angle variation dial 6, and rotation dial 7 provided in the operation unit 3, while maintaining the stable gripping state of the operation unit 3. In other words, the aforementioned three operations of opening and closing, curving, and rotating can be continuously executed with good stability during surgery, without changing the manner of holding or passing the operation unit 3 from one hand to another, and the endoscopic suturing operation can be conducted in a very easy manner and with good reliability.

Furthermore, because the holding power of the suturing needle does not hinder the rotation transmitting power, when the needle is clamped, it can be easily rotated and readily handled.

In the present embodiment, the rotation displacement direction of the angle variation dial 6 and rotation dial 7 is parallel to the long axis direction of the operation unit 3, but this direction is not limiting, and the appropriate direction may be set according to the performance range of the finger used for the operation.

Furthermore, in the present embodiment, the rotation dial 7 and angle variation dial 6 are disposed according to the above-described mutual arrangement, but this arrangement is not limiting, and the rotation dial 7 and angle variation dial 6 may be set in a row, that is, without any offset.

Furthermore, in the present embodiment, the engagement relationship of the inner bevel gear 61 or inner bevel gear 71 with the bevel gear unit 121 or bevel gear unit 123 is set so that the shafts of the gears and units intersected at an angle of about 90 degrees, but such configuration is not limiting and may be changed appropriately according to the disposition of the angle variation dial 6 and rotation dial 7.

The present invention is not limited to the above-described embodiment, and various changes and modifications can be made without departing from the essence of the invention.

In the present embodiment, the rotation displacement direction of the angle variation dial 6 and rotation dial 7 is parallel to the long axis direction of the operation unit 3, but this direction is not limiting, and the appropriate direction may be set according to the performance range of the finger used for the operation.

Furthermore, in the present embodiment, the rotation dial 7 and angle variation dial 6 are disposed according to the above-described mutual arrangement, but this arrangement is not limiting, and the rotation dial 7 and angle variation dial 6 may be set in a row, that is, without any offset.

Furthermore, in the present embodiment, the engagement relationship of the inner bevel gear 61 or inner bevel gear 71 with the bevel gear unit 121 or bevel gear unit 123 is set so that the shafts of the gears and units intersected at an angle of about 90 degrees, but such configuration is not limiting and may be changed appropriately according to the disposition of the angle variation dial 6 and rotation dial 7.

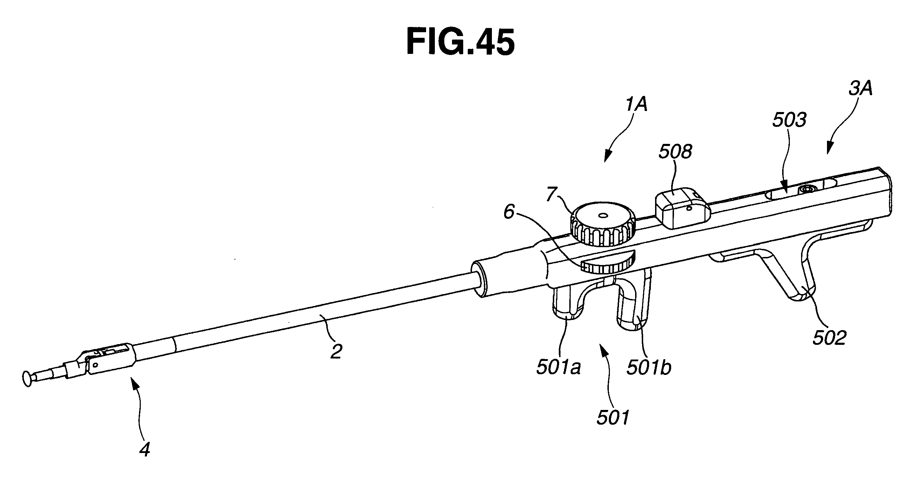

A variation example of the first embodiment will be described below. Fig. 45 is a perspective view illustrating the external appearance of the needle driver 1A of a modification example of the embodiment, as viewed from one side at an angle in a front view.

Specific features of the present modification example are in the operation unit and treatment unit. First, the operation unit will be described. As shown in Fig. 45, the first specific feature of the operation unit is that the operation unit 3A is provided with a finger hook member 501 and a palm placement member 502.

The finger hook member 501 is a member made of plastic and serving for placing a middle finger when the operation unit 3A is gripped. The finger hook member 501 that is fixed, for example, with a screw lock, to the operation unit 3A has two protruding sections 501a, 501b that protrude in the direction perpendicular or substantially perpendicular to the long axis of the operation unit 3A. A middle finger can be placed between the two protruding sections 501a, 501b. The protrusion quantity of the protruding section 501a located on the distal end side of the operation unit 3A from the surface of the operation unit 3A is less than the protrusion quantity of the protruding section 501b located on the proximal end side. The position for placing the middle finger on finger hook member 501 is substantially identical to that of the angle variation dial 6 and rotation dial 7 in the long axis direction of the operation unit 3A, or somewhat shifted toward the proximal end from the angle variation dial 6 and rotation dial 7. In other words, the position for placing the middle finger on finger hook member 501 is provided so as to be substantially identical to respective positions where the angle variation dial 6 and rotation dial 7 are provided or on the proximal end side shifted from those positions.

The palm placement member 502 is provided further on the proximal end side of the operation unit 3A shifted from the finger hook member 501. The palm placement member 502 is a member made of plastic and having a protruding section for placing a root portion between the first finger and index finger when the operation unit 3A is gripped.

Furthermore, the rotation dial 7 is provided so as to be exposed on the surface of the external cover member of the operation unit 3A. This is necessary because of individual difference in the length of fingers (here, the length of the index finger). As a result, the rotation operation of the rotation dial 7 can be easily conducted regardless of the length of the finger.

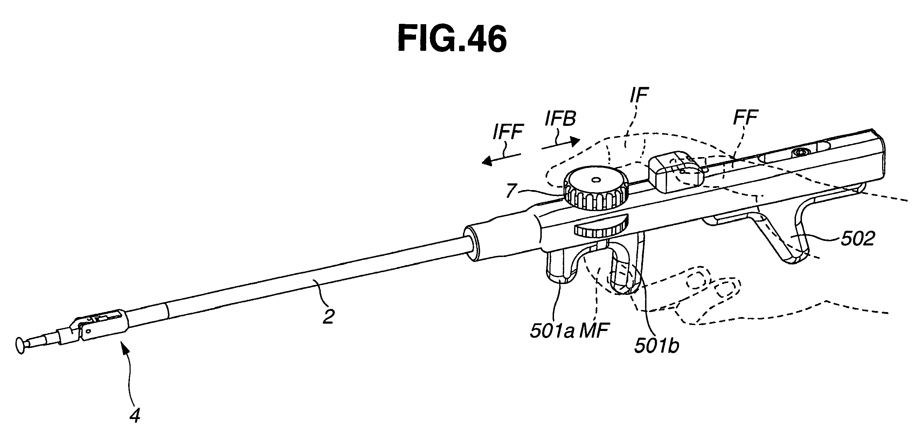

Fig. 46 is an explanatory diagram illustrating the state where the needle driver 1A shown in Fig. 45 relating to the present variation example is gripped. As shown in Fig. 46, when the surgeon brings a root portion between the first finger FF and index finger IF into contact with the palm placement member 502 and places the middle finger MF between the two protruding sections 501a and 501b, the surgeon can firmly grip the needle driver 1A with good stability. As shown in Fig. 46, after the surgeon has gripped the needle driver 1A, the rotation dial 7 and angle variation dial 6 can be operated with the index finger IF and an opening and closing operation button 508 can be operated with the first finger FF.

Furthermore, because the palm placement member 502 protrudes obliquely from the side section of the operation unit 3A slightly toward the proximal end side, the surgeon can grip the needle driver 1A firmly by bringing the palm and the palm placement member 502 into intimate contact during gripping.

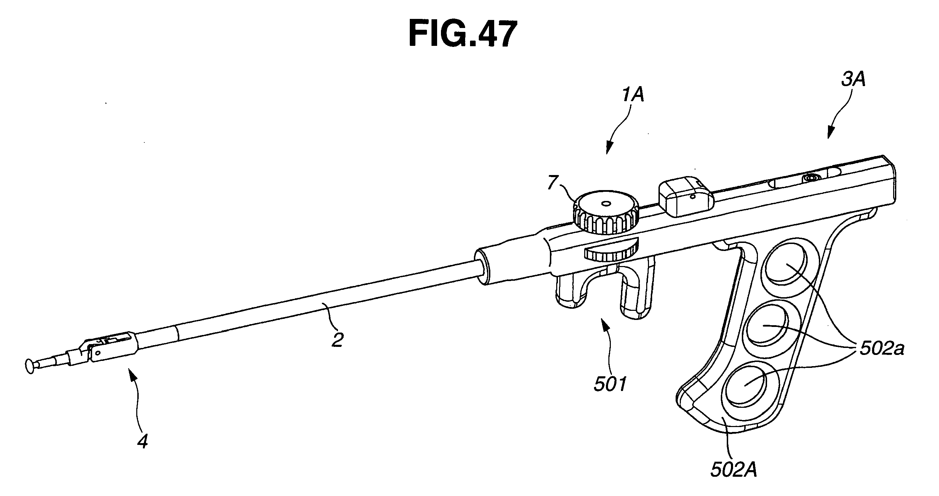

As shown in Fig. 47, in order to further facilitate the gripping, the palm placement member 502A may be made larger than the palm placement member 502 shown in Fig. 45. Fig. 47 is a perspective view illustrating the external appearance of the needle driver 1A of another variation of the present embodiment example, as viewed from one side at an angle in a front view. In particular, to reduce weight, a plurality of holes 502a are formed in the palm placement member 502A which is made of plastic and is larger than the palm placement member 502 shown in Fig. 45. The palm placement member 502A protrudes obliquely from the side section of the operation unit 3A to same extent toward the distal end side, but because the palm placement member 502A and the palm are brought into intimate contact when the needle driver is gripped, the surgeon can grip the needle driver 1A firmly and with good stability.

Furthermore, the palm placement members 502, 502A shown in Fig. 45 and Fig. 46 can be omitted. Even when the palm placement members 502, 502A are absent, the surgeon can grip the needle driver 1A so as to avoid any shaking, that is, with good stability. This is because, even when the palm placement members 502, 502A are absent, when the operation of rotating the rotation dial 7 is carried out with the index finger IF, the two protruding sections 501a, 501b function, with regard to both rotation directions of the rotation dial 7 which are designated with the index finger IF, as support sections for supporting the finger hook member 501 in the direction opposite, which is designated with the middle finger MF, to the rotation direction of the rotation dial 7. For example, in the case where the palm placement member 502 shown in Fig. 46 is absent, when the index finger IF is moved in the IFF direction on the distal end side of the needle driver 1A and the rotation dial 7 is rotated, the middle finger MF comes into contact with the protruding section 501b and the protruding section 501b functions as a support section. Similarly, when the index finger IF is moved in the IFB direction on the proximal end side of the needle driver 1 and the rotation dial 7 is rotated, the middle finger MF comes into contact with the protruding section 501a and the protruding section 501a functions as a support section.

Furthermore, the position of the palm placement member 502 in the long axis direction of the operation unit 3A can be adjusted. That is, the operation unit 3A has a spacing adjustment mechanism for adjusting the space between the finger hook member 501 and palm placement member 502. This is done to adapt the needle driver 1A to the difference in the palm size and finger length among the surgeons.

Furthermore, as described above, in the present variation example, because the rotation dial 7 is provided on the outside of the operation unit 3A, it is adapted to the difference in the palm size and finger length among the surgeons. Therefore, the spacing adjustment mechanism may be omitted. However, when the spacing adjustment mechanism is present, even if only part of the rotation dial 7 protrudes from the surface of the operation unit 3, as shown in Fig. 1, the position of the palm placement member 502 can be adjusted, so as to facilitate the operation of the rotation dial 7, according to the length of the surgeon's fingers.

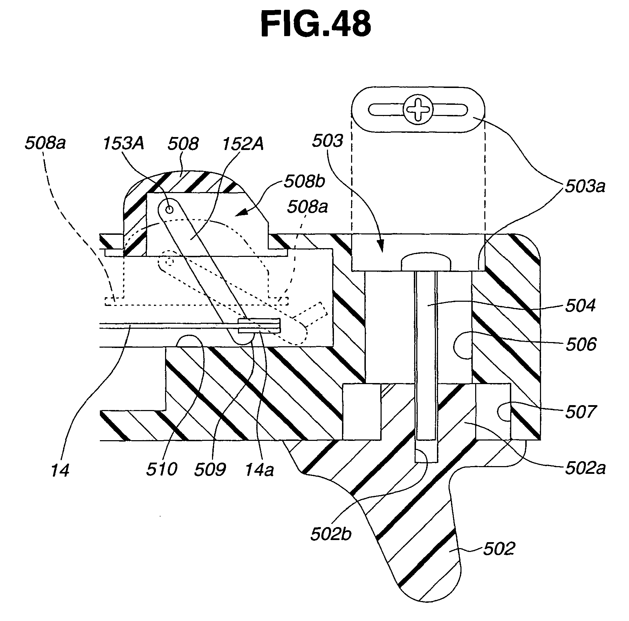

An elongated reel hole 503 is formed in the proximal end side of the operation unit 3A. The elongated reel hole 503 has a seat section 503a that abuts against the screw head of an adjustment screw 504, as shown in Fig. 48. Fig. 48 is a partial sectional view of the operation unit 3A of this modification example. A long hole 506, which is smaller than the inner diameter of the elongated reel hole 503, is formed in the seat section 503a. On the surface of the operation unit 3A on the opposite side from the elongated reel hole 503, a long hole 507 is formed for accepting the protruding section 502a of the palm placement member 502. The adjustment screw 504 is inserted from the elongated reel hole 503, the threaded section of the adjustment screw 504 is screwed into an orifice 502b of the protruding section 502a where an internal thread is formed, and the palm placement member 502 is fixed to the operation unit 3A by tightening the screw. During such fixing by adjusting the position of the adjustment screw 504 inside the long hole 506 in the long axis direction of the operation unit 3A, the position of the palm placement member 502 in the long axis direction of the operation unit 3A can be adjusted according to the size of the surgeon's hand.

The second specific feature of the operation unit is in that the opening and closing operation of the treatment unit 4 is carried out with a press-button as shown in Fig. 45.

An opening and closing press-button 508 for carrying out the operation of opening and closing the treatment unit 4 is provided in the operation unit 3A. The opening and closing press-button 508 is made of plastic or aluminum. The opening and closing press-button 508 is provided between the finger hook member 501 and palm placement member 502 in the long axis direction of the operation unit 3A on the side opposite that of the finger hook member 501 and palm placement member 502 (on the same side with the rotation dial 7). Furthermore, as shown in Fig. 46, the opening and closing press-button 508 is provided in a position such that the surgeon can operate the opening and closing press-button 508 by pressing it down with the first finger FF, while gripping the needle driver 1A.

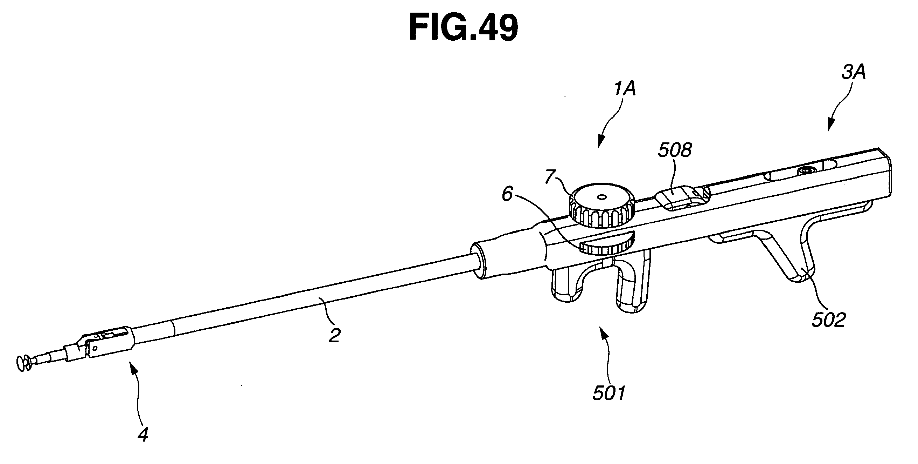

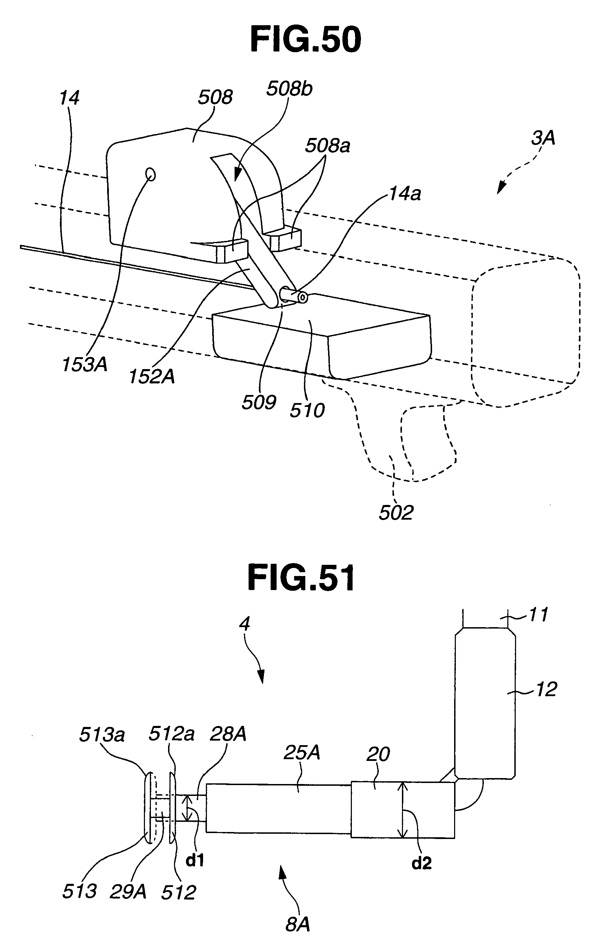

Fig. 49 is an external view illustrating the state where the opening and closing press-button 508 is pressed down. Fig. 50 is an explanatory diagram illustrating the opening and closing action mechanism of the treatment unit 4 in response to the opening and closing press-button 508. When the opening and closing press-button 508 is pressed down, it is pressed into the operation unit 3A. That is, the opening and closing press-button 508 is pressed down in the direction perpendicular to the opening of the hole provided in the external cover member of the operation unit 3A. Furthermore, because the opening and closing press-button 508 has a flange section 508a that abuts against the inner side of the external cover member 131, the opening and closing press-button is not separated from the external cover member even when the button is not pressed.

Furthermore, as shown in Fig. 48 and Fig. 50, a groove section 508b is formed along the long axis direction of the operation unit 3A in the opening and closing press-button 508. Inside the groove section 508, one end of a link 152A is provided so that it can freely rotate about the axis of a shaft 153A. The engagement piece 14a fixedly attached to the pulling wire 14 in the end section of the pulling wire is fixedly attached to the other end of the link 152A. A curved surface section 509 is formed in the distal end section on the other end of the link 152A having the engagement piece 14a fixedly attached thereto.

One end of the link 152A located inside the groove section 508 is positioned closer to the distal end side in the long axis direction of the operation unit 3A, that is, closer to the treatment unit 4, than the other end having the engagement piece 14a fixedly attached thereto. In other words, the link 152A is provided such that one end of the link 152A located inside the groove section 508 is positioned obliquely along the long axis of the operation unit 3A from the proximal end side toward the distal end side as against the other end having the engagement piece 14a fixedly attached thereto. In such state, the curved surface section 509 of the link 152A abuts against a sliding section 510 formed inside the external cover member of the operation unit 3A or positioned close thereto. When the opening and closing press-button 508 is pressed down, the other end of the link 152A having the engagement piece 14a fixedly attached thereto moves further toward the proximal end side in the long axis direction of the operation unit 3A.

More specifically, when the opening and closing press-button 508 is pressed down, the link 152A moves toward the inner side of the operation unit 3A, and the other end of the link 152A slides while coming into contact with the sliding section 510 located inside the operation unit 3A. Thus, when the opening and closing press-button 508 is pressed down, as shown in Fig. 48, the curved surface section 509 of the link 152A moves toward the proximal end side or the surface of the sliding section 510 that has a surface substantially parallel to the long axis direction of the operation unit 3. As a result, the pulling wire 14 is pulled toward the proximal end side of the operation unit 3A and the clamping unit 8 is, therefore, opened.

Therefore, with the above-described configuration of the operation unit 3A, the surgeon can grip firmly the operation unit 3A and can open the clamping unit 8 by pressing down the opening and closing press-button 508.

The treatment unit of the present variation example will the described below. As shown in Fig. 51, a specific feature of this treatment unit is that two clamping members have respective disk-shaped sections.

Fig. 51 is a side surface view of the treatment unit 4 of the present variation example.

As shown in Fig. 51, a clamping member 29A is made of metal and has a disk-shaped flange section 513 at the distal end side thereof. The disk-shaped flange section 513 is formed integrally with the clamping member 29A. The clamping member 29A is obtained by integrating the distal-end clamping piece 31 and the distal-end clamping piece attachment member 29 of the above-described embodiment and making a portion of the distal-end clamping piece 31 in the form of a thin disk. Furthermore, a curved surface section 513a is formed on the periphery of the distal end side of the disk-shaped flange section 513. The periphery of the distal end side of the disk-shaped flange section 513 may be a slanted section rather than curved surface section.

Another clamping member 28A is also made of metal and has a disk-shaped flange section 512 at the distal end side thereof. The disk-shaped flange section 512 is formed integrally with the clamping member 28A. The clamping member 28A is obtained by somewhat reducing the size of the shaft portion of the movable clamping piece 28 of the above-described embodiment and making a portion of the distal end side in the form of a thin disk. Furthermore, a curved surface section 512a is formed on the periphery of the proximal end side of the disk-shaped flange section 512.

Thus, the clamping member 29A and clamping member 28A are configured to comprise respective substantially disk-shaped portions constituting thin flat sections.

The thickness of the flange sections 512 and 513 in the long axis direction of the operation unit 3A is 0.5 mm or less. The flat sections of the clamping member 29A, which is a distal-end clamping piece, and the clamping member 28A, which is a movable clamping piece, have a surface shape subjected to slipping preventing processing such as electric discharge processing, so that the clamped needle can be tightly clamped thereby.

Furthermore, the rotation section base member 25A having part of the distal end side of the clamping piece 28A inserted thereinto is formed to be longer than the rotation section base member 25 of the above-described first embodiment. The clamping member 28A is pressed at the distal end side against the rotation section base member 25A, as shown by a broken line in Fig. 51. By pressing down the opening and closing press-button 508, the pulling wire 41 is pulled toward the operation unit 3 and, as shown in Fig. 51, the movable clamping piece 28A moves toward the proximal end side. As a result, the clamping unit 8 is opened.

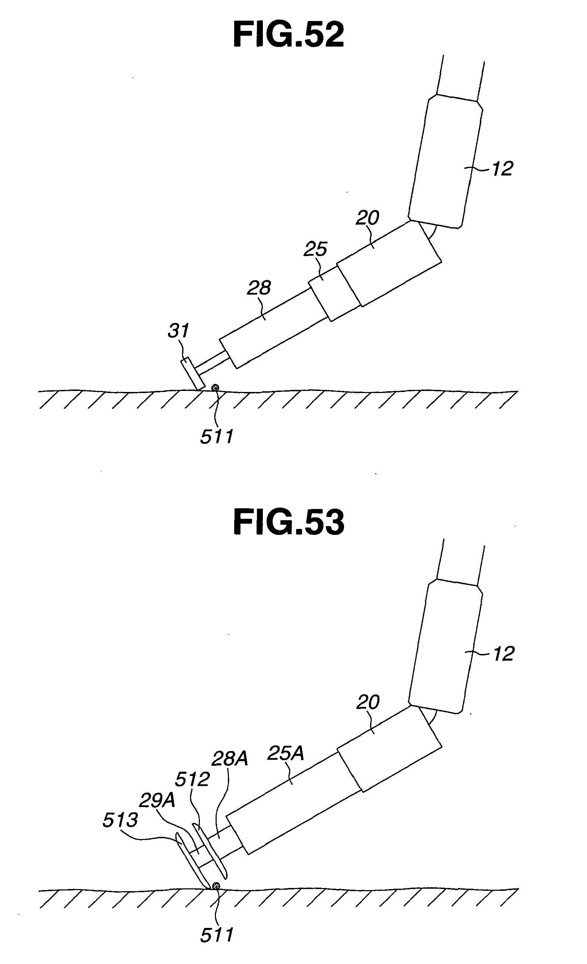

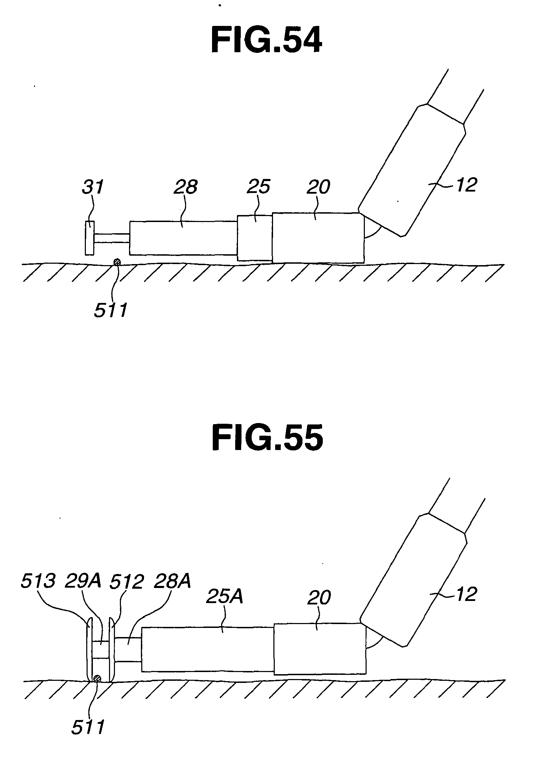

The diameters of the flange sections 512, 513 are equal to each other. The diameter d1 of the flange sections 512, 513 is larger than the diameter d2 of the cylindrical section 20a of the curving section base member 20. That is, the diameter d1 of the flange sections 512, 513 is larger than the diameter of the members other than flange sections 512, 513 in the treatment unit 4. Fig. 52 to Fig. 55 illustrate the action of clamping a needle by using the needle driver 1 of the embodiment and the needle driver 1A of the variation example thereof. Fig. 52 to Fig. 55 illustrate the clamping action, for example, in the case where the needle is placed on the tissue of a living body and the manner of holding the needle is changed. Fig. 52 and Fig. 54 are the explanatory drawings illustrating the action of clamping a needle by using the needle driver 1 of the embodiment. Fig. 53 and Fig. 55 are the explanatory diagrams illustrating the action of clamping a needle by using the needle driver 1A of the variation example of the embodiment.

As shown in Fig. 52, in the case of the needle driver 1, when a needle 511 is clamped in a state where the clamping unit 8 is bent with respect to the insertion unit 2, because of the thickness of the distal-end clamping section 31, the needle 511 is difficult to bring into contact with the flat section of the distal-end clamping piece 31 at certain bending angles of the clamping unit 8 and with certain states of the body cavity wall. As a result, the needle 511 placed on the body cavity wall is difficult to clamp. By contrast, as shown in Fig. 53, in the case of the needle driver 1A, when the needle 511 is clamped in a state where the clamping unit 8 is bent with respect to the insertion unit 2, because the thickness of the flange section 513 of the clamping member 29A is small, the needle 511 is easy to bring into contact with the flat section of the flange section 513. Furthermore, because a curved section 513a is formed in the periphery portion of the distal end side of the flange section 513, the needle 511 is easy to bring into contact with the flat section of the flange section 513. Furthermore, in the case shown in Fig. 53, at least only the flange section 513 at the distal end side may be in the form of a thin disk.

As shown in Fig. 54 and Fig. 55, the axial direction of the clamping unit 8 can be substantially parallel to the surface of the body cavity wall at certain bending angles of the clamping units 8 of the needle drivers 1, 1A. As shown in Fig. 54, in the case of the needle driver 1, the diameter of the curved section base member 20 of the clamping unit is larger than that of the distal-end clamping section 31. As a result, the needle 511 is sometimes difficult to bring into contact with the flat section of the distal-end clamping piece 31 and the needle 511 is difficult to clamp. By contrast, as shown in Fig. 55, in the case of the needle driver 1A, the diameter of the flange section 513 of the clamping member 29A and the flange section 512 of the clamping member 28A is larger than the diameter of the member with the largest diameter at the time the clamping unit 8 is bent, here, the diameter of the curved section base member 20. Therefore, the needle 511 is easy to bring into contact with the respective flat sections of the flange sections 513 and 512.

Therefore, with the above-described configuration of the clamping unit 8A, the surgeon can easily clamp the needle at any bending angle of the clamping unit 8A and in any state of the body cavity wall.

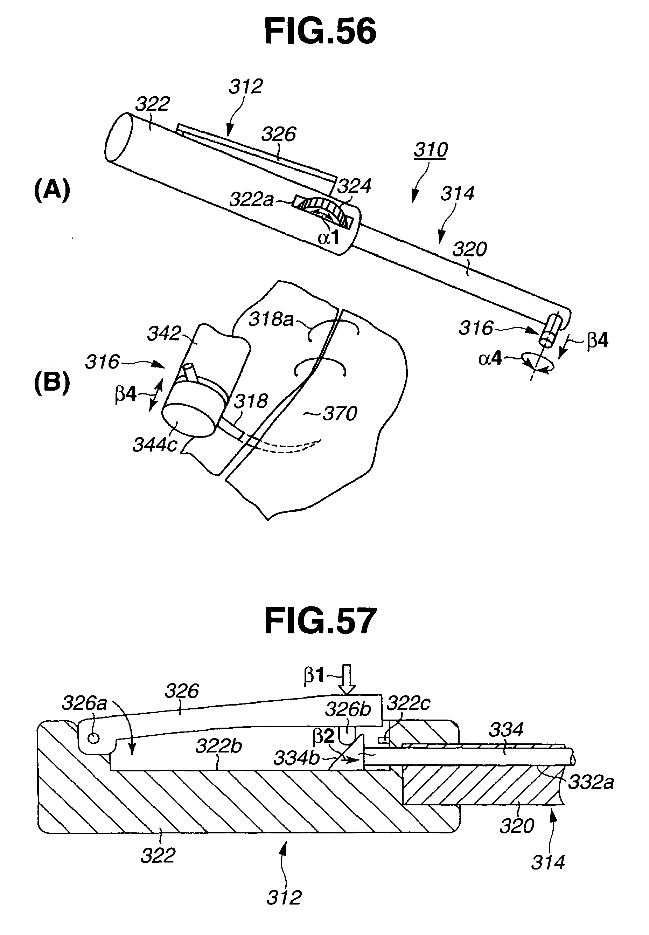

A second embodiment of the present invention will be described below with reference to Fig. 56 to Fig. 58.

As shown in Fig. 56(A), a needle-holding device 310, which is the surgical instrument of the present embodiment, comprises an operation unit (main unit) 312 operated by the surgeon, an insertion unit 314 for insertion into the body cavity of a patient, the insertion unit being connected to the operation unit 312, and a needle-holding unit (clamping section) 316 which is provided at the distal end of the insertion unit 314 for clamping an anastomosis needle (clamping object) 318 (see Fig. 56(B)).

The operation unit 312 comprises a substantially cylindrical grip 322 held by the surgeon and a needle-holding unit rotary dial (disk body) 324 and an opening and closing lever 326 disposed at the grip 322. The needle-holding unit rotary dial 324 and opening and closing lever 326 are disposed in positions such that they can be operated in a state where the grip 322 is held by the surgeon. The needle-holding unit rotary dial 324 partially protrudes to the outside from a notch 322a formed in the side surface of the grip 322. The needle-holding unit rotary dial 324 comprises a shaft (not shown in the figure) in the direction perpendicular to the lengthwise axis (central axis) of the grip 322. As a result, the needle-holding unit rotary dial 324 can rotate in the direction of arrow α1 shown in Fig. 56(A).

As shown in Fig. 57, a concave section 322b is formed in the side portion of the grip 322. One end of the opening and closing lever 326 is pivotally supported via a rotary shaft 326a in the proximal end section of the concave section 322b of the grip 322. The rotary shaft 326a is disposed in the direction perpendicular to the lengthwise direction of the grip 322. As a result, the opening and closing lever 326 can rotate in the direction of arrow β1 shown in Fig. 57.

The rotation distal end section, which is the other end section of the opening and closing lever 326, can rotate with respect to the grip 322 about the rotary shaft 326a as a center. A push pin 326b is integrally attached to the rotation distal end section (other end section) of the opening and closing lever 326 in the direction (side of the concave section 322b) perpendicular to the lengthwise direction of the grip 322. The distal end section of the push pin 326b, which protrudes from the opening and closing lever 326, is formed to have a substantially spherical shape and comes into contact with a slant surface 334b of the below-described slant block 334a.

A stopper 322c for restricting the motion range of the opening and closing lever 326 is attached to the grip 322. As a result, when the opening and closing lever 326 is operated in the direction of arrow β1 shown in Fig. 57, the rotation distal end section of the opening and closing lever 326 abuts against the stopper 322c, thereby controlling the rotation of the opening and closing lever 326.

The distal end section of the grip 322 and the proximal end section of the insertion unit 314 are connected integrally together. A first guide section (first channel) 332a for guiding the below-described slide rod 334 along the lengthwise axis of the insertion unit 314 is formed in the insertion unit 314. The proximal end section of the first guide section 332a reaches the distal end section of the grip 332 and is linked to a space formed by the concave section 322b of the grip 322.

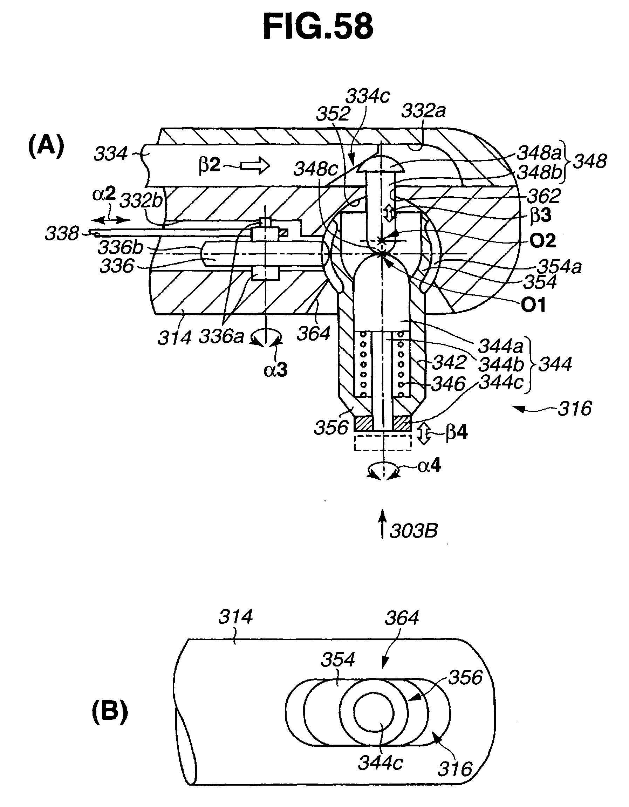

The first guide section 332a is provided with a slide rod (slider) 334 such that the rod can freely slide along the lengthwise axis of the insertion unit 314. One end (end on the side of the operation unit 312) of the slide rod 334 reaches the concave section 322b of the grip 322. A slant block 334a having a slant surface 334b is integrally attached to one end of the slide rod 334. As shown in Fig. 58(A), a taper surface 334c abutted against a round head section 348a of the below-described push rod 348 is formed at the other end of the slide rod 334.

A second guide section (second channel) 332b where the below-described belt 338 is provided is formed side by side with the above-described first guide section 332a in the insertion unit 314. The proximal end section of the second guide section 332b reaches the aforementioned rotary dial 324 of the operation unit 312.

A gear (rotary body) 336 having a shaft 336a parallel to the shaft (not shown in the figure) of the rotary dial 324 and perpendicular to the lengthwise axis of the insertion unit 314 is pivotally supported at the distal end of the second guide section 332b. A belt (operation power transmission member) 338 is provided in the lengthwise direction of the insertion unit 314 in the second guide section 332b. This belt 338 is stretched over the rotary dial 324 and gear 336 (see Fig. 58) with an appropriate tension. As a result, when the surgeon rotates the rotary dial 324, the belt 338 rotates and causes the rotation of the gear 336.

As shown in Fig. 58(A), a socket section 352 which is linked to the second guide section 332b in the distal end of the second guide section 332b and has a spherical inner peripheral surface is formed in the distal end section of the insertion unit 314. As shown in Fig. 58(A) and Fig. 58(B), this socket section 352 is linked to the outside of the insertion unit 314 by a long orifice 364 formed in the side of the distal end section of the insertion unit 314. The long orifice 364 has a width slightly larger than the diameter of the below-described needle-holding unit body 342 of the needle-holding unit 316 and a length such that the needle-holding unit body 342 can be inclined about the center of the below-described spherical body 354 as a point of support within the prescribed range. The edge of the long orifice 364 on the side of the distal end section of the insertion section 314 and the edge thereof on the side of the proximal end section of the insertion unit 314 are inclined at the same angle as the inclination angle of the needle-holding unit body 342. That is, the long orifice is expanded from the inside to the outside of the insertion unit 314. With such a long orifice 364, the needle-holding unit 316 inclines with respect to the insertion section 314 by moving only along the lengthwise direction of the long orifice 364.

As shown in Fig. 58(A), the needle-holding unit 316 comprises a needle-holding unit body (needle-clamping section body) 342, a needle-holding rod (needle-clamping rod) 344, a coil spring (elastic member) 346, and a push rod 348. For the needle-holding unit body 342, needle-holding rod 344, coil spring 346, and push rod 348, the side of the needle-holding unit body 342 that is close to the below-described rotation center O1, that is, the inner side of the insertion unit 314, as one end section, and the side at a distance from the rotation center O1, that is, the side going away from the inside of the insertion unit 314, as the other end section.

A hollow spherical body (holding section spherical body) 354, which is partially spherical, is formed in one end section (upper end section, as shown in Fig. 58(A)) of the needle-holding unit body 342. This spherical body 354 is fit into the aforementioned socket section 352 of the insertion unit 314. As a result, a ball joint is formed by the socket section 352 of the insertion unit 314 and the spherical body 354 of the needle-holding unit body 342. The center of the socket section 352 coincides with the rotation center of the needle-holding unit body 342. Furthermore, a friction force between the socket section 352 and spherical body 354 is set such that the inclination angle of the needle-holding unit body 342 does not change, that is, the orientation of the above-described anastomosis needle 318 does not change during the anastomosis.

A tooth section 354a engaged with a tooth section 336b of the gear 336 is formed at the outer circumferential surface of the spherical body 354. As a result, when the gear 336 rotates, the spherical body 354 also rotates with respect to the socket section 352. Thus, when the gear 336 rotates, the needle-holding unit body 342 rotates about the central axis thereof.

A flange section 356 protruding radially and inwardly of the needle-holding unit body 342 is formed at the outer end section (lower end in Fig. 58(A)) of the needle-holding unit body 342. The needle-holding rod 344 is slidably provided in the needle-holding unit body 342. The needle-holding rod 344 comprises a head section (rod head section for gripping) 344a, rod section 344b, and holding section (clamping section) 344c.

The head section 344a can slide along the inner circumferential surface of the needle-holding unit body 342, and part thereof is provided inside the spherical body 354. In the rod section 344b, one end section (the upper end section, as shown in Fig. 58(A)) is connected to the other end section (lower end section, as shown in Fig. 58(A)) of the head section 344a and extends toward the lower end section of the needle-holding unit body 342 on the central axis of the needle-holding unit body 342. The holding section 344c is disposed in one end section (lower end section, as shown in Fig. 58(A)) of the rod section 344b and can be brought into contact with and going away from the other end surface (lower end surface as shown in Fig. 58(A)) of the flange section 356 at the lower end section of the needle-holding unit body 342.

A coil spring 346 is provided between the other end surface (lower end in Fig. 58(A)) of the head section 344a of the needle-holding rod 344 and one end surface (upper end in Fig. 58(A)) of the flange section 356 of the needle-holding unit body 342. The head section 344a is impelled upward of the needle-holding unit body 342 by the elastic force of the coil spring 346. As a result, the above-described anastomosis needle 318 (see Fig. 56(B)) is clamped between the lower end surface of the flange section 356 of the needle-holding unit body 342 and one end surface (upper end in Fig. 58(A)) of the holding section 344c of the needle-holding rod 344. Furthermore, one end section (upper end in Fig. 58(A)) of the head section 344a of the needle-holding rod 344 is formed to have a spherical shape. The upper end position of the head section 344a substantially coincides with the rotary center O1 of the needle-holding unit body 342 in a state where the anastomosis needle 318 is not clamped between the holding section 344c and needle-holding unit body 342.

A cylindrical opening 362 having a central axis in the direction (up-down direction in Fig. 58(A)) perpendicular to the lengthwise direction of the insertion unit 314 is formed in the socket section 352 of the insertion unit 314. A push rod 348 is provided in the opening 362. The push rod 348 comprises a round head section (push rod head section) 348a and a shaft section 348b. The round head section is provided inside a space for sliding in the distal end section of the first guide section 332a and comes into contact with the taper surface 334c of the above-described slider rod 334.

Here, the operation of pressing in the opening and closing lever 326 of the operation unit 312 (the operation in the direction of arrow β 1 in Fig. 57) moves the slide rod 334 toward the distal end side (direction of arrow β 2) of the insertion unit 314. As a result, the round head section 348a is pushed by the taper surface 334c of the slide rod 334. That is, the movement of the slide rod 334 in the direction of arrow β 2 is converted into the movement of the shaft section 348b of the push rod 348 in the direction of arrow β 3. Thus, the slide rod 334 and push rod 348 constitute operation direction conversion means.

As a result, the operation of pressing in the opening and closing lever 326 causes the slide rod 334 to slide in the direction of arrow β 2. Therefore, the push rod 348 can freely slide in the direction (direction of arrow β 3) perpendicular to the axial direction of the insertion unit 314 when the slide rod 334 slides.

A spherical surface section (circular-arc section in the form of a circular arc) 348c in the form of a spherical surface is formed in one end section (lower end in Fig. 58(A)) of the shaft section 348b of the push rod 348. The lower end section of the shaft section 348b comes into contact with the upper end section of the head section 344a of the needle-holding rod 344 in the above-described rotary center O1. The length (radius) between the center O2 of the spherical surface section 348c of the lower end of the shaft section 348b and the lower end surface (center O1) is formed such as to be substantially equal to the thickness of the proximal end section of the above-described anastomosis needle 318. As a result, in a state where the anastomosis needle 318 is grasped, clamped between the holding section 344c and needle-holding unit body 342, the center 02 of the spherical surface section 348c of the lower end of the shaft section 348b of the push rod 348 coincides with the rotation center of the needle-holding unit body 342.

The operation of the needle-holding device 310 of the above-described configuration will be described below.

When the opening and closing lever 326 is operated to the very limit of the operation range in the direction of arrow β 1 in Fig. 57, the distal end section of the push pin 326b comes into contact with the slant surface 334b of the slant block 334a, and the slant block 334a moves toward the distal end section along the lengthwise direction of the insertion unit 314. As a result, the slide rod 334 slides toward the distal end section (direction of arrow β 2) along the first guide section 332a.

When the slide rod 334 moves in the direction of arrow β2, the round head section 348a of the push rod 348 is pressed by the taper surface 334c of the slide rod 334 in the direction of arrow β 3 (downward) in Fig. 58(A). At this time, the center O2 of the spherical section 348c of the lower end of the shaft section 348b of the push rod 348 moves till it passes over the center O1 of the socket section 352. As a result, the upper end section of the head section 344a of the needle-holding rod 344 is pressed to the lower end of the shaft section 348b of the push rod 348. The head section 344a moves along the inside of the needle-holding unit body 342 and causes the holding section 344c of the needle-holding rod 344 to move downward in the direction of arrow β 4 till it reaches the position of the broken line in Fig. 58 (A) against the impelling force of the coil spring 346.

In a state where the holding section 344c is disposed in the position of a broken line, there is an opening quantity sufficient to clamp the anastomosis needle 318 having a thread 318a provided therein between the holding section and the lower end surface of the needle-holding unit body 342, that is, an opening quantity larger than the thickness of the anastomosis needle 318, so that the needle can be used for anastomosis or suturing. In this state, the anastomosis needle 318 is disposed between the holding section 344c and the lower end section of the needle-holding unit body 342 and the hand is removed from the opening and closing lever 326. At the same time as the needle-holding rod 344 moves under the effect of the elastic force of the coil spring 346 and the anastomosis needle 318 is sandwiched between the holding section 344c and the lower end surface of the needle-holding unit body 342, the push rod 348 and slide rod 334 are pushed back by the elastic force of the coil spring 346. As a result, the anastomosis needle 318 is clamped (mounted) between the holding section 344c and the lower end surface of the needle-holding unit body 342.

Then, the insertion unit 314 of the needle-holding device 310 in which the anastomosis needle 318 is clamped between the holding section 344c and the lower end surface of the needle-holding unit body 342 is inserted into the body cavity from one of a plurality of ports (not shown in the figure) opened in the body of the patient. The needle-holding section 316 and anastomosis needle 318 are brought close to an anastomosis position 370 (see Fig. 56(B)), and the inclination angle of the needle-holding section 316 is changed by using a grasping forceps (not shown in the figure) inserted from another port usually used in endoscopic surgery so that the inclination of the needle-holding section 316 is optimum for anastomosis. At this time, because a state is assumed in which the distal end center O2 of the spherical section 348c of the push rod 348 substantially coincides with the center O1 of the socket section 352, the needle-holding rod 344 does not move in the direction of arrow β 4. As a result, the anastomosis needle 318 held (clamped) between the holding section 344c and the needle-holding unit body 342 does not fall down.

When the needle-holding section rotary dial 324 is rotated in the direction of arrow α 1 in Fig. 56(A) in this state, the belt 338 moves in the direction of arrow α 2 and the gear 336 is rotated in the direction of arrow α 3. Because of this rotation of the gear 336, the needle-holding unit body 342 rotates in the direction of arrow α 4. Thus, the needle-holding section 316 is rotated in the direction of arrow α 4, the anastomosis needle 318 is operated, and the anastomosis is performed. Here, because the friction force between the spherical body 354 of the needle-holding unit body 342 and the socket section 352 of the insertion unit 314 is set to an optimum value, the inclination angle of the needle-holding unit body 342 is prevented from changing during the anastomosis. Thus, the orientation of the anastomosis needle 318 is prevented from changing during the anastomosis.

As described hereinabove, with the second embodiment mode, the following effect can be obtained.

The needle-holding section 316 can be inclined (rotated) with respect to the distal end section of the insertion unit 314, and when it is desired to be held in an inclined state, it is not necessary to operate other components and the inclination angle can be changed to the desired angle only by applying an external force to the needle-holding unit 316 by using other forceps or the like. The anastomosis needle 318 can be operated and the anastomosis operation can be performed by an operation of rotating the needle-holding unit 316 or an operation of moving the needle-holding device 310 itself in a state where the attained desired angle is maintained. Therefore, the treatment procedure can be conducted in an easy manner. Furthermore, the needle-holding device 310 has a simple structure and can be manufactured at a low cost. Moreover, the weight thereof can be reduced so that a large load is not placed on the surgeon.

In the present embodiment, it is preferred that the tooth sections 354a of the usually employed involute shape be formed on the outer circumference of the spherical body 354 that is engaged with the tooth sections 336b of the gear 336, but the same effect can be obtained with any shape that engages with the tooth sections 336b of the gear 336, for example, such as roulette of the same pitch.

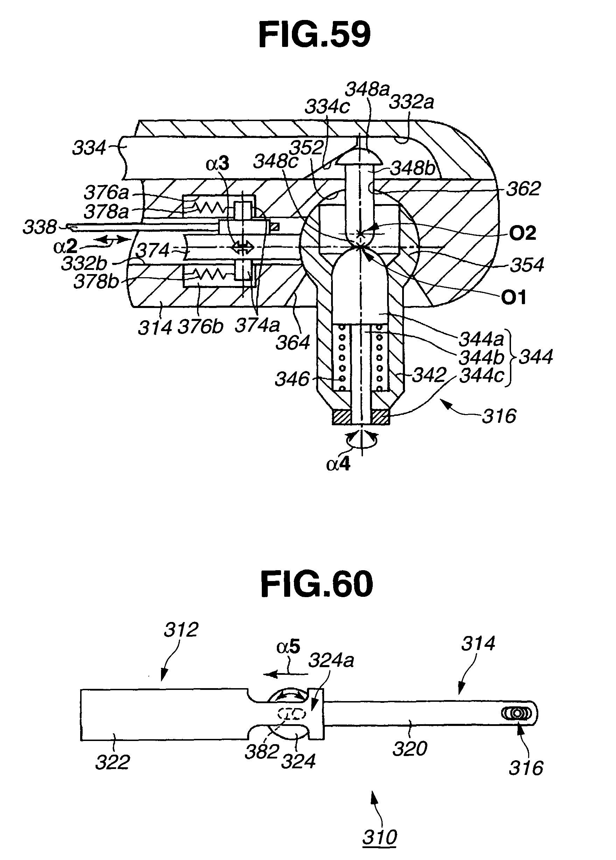

A third embodiment will be described below with reference to Fig. 59 and Fig. 60. The third embodiment is a variation example of the second embodiment, and the components identical to those explained in the second embodiment will be denoted with the same symbols and the detailed explanation thereof will be omitted.

As shown in Fig. 59, a friction wheel (rotary body) 374 is pivotally supported instead of the gear 336 shown in Fig. 58(A) at the distal end of the second guide section 332b of the insertion unit 314. The tooth sections 354a are removed from the outer circumferential surface of the spherical body 354 of the needle-holding unit body 342, and the outer peripheral surface of the spherical body 354 is pressed against the outer peripheral surface of the friction wheel 374. That is, the friction wheel 374 has a brake action upon the spherical body 354. Here, the friction force of the mutually contacting surfaces of the spherical body 354 of the needle-holding unit body 342 and the socket section 352 of the insertion unit 314 is less than that explained in the second embodiment and is set to the friction force that is optimum for adjusting the inclination of the needle-holding section 316.

A pair of grooves 373a, 376b are formed in the side section at the distal end of the second guide section 332b. The respective compression springs 378a, 378b are provided between the proximal end sections of those grooves 376a, 376b and a rotary shaft 374a of the friction wheel 374. As a result, the rotary shaft 374a of the friction wheel 374 can be rotated by the grooves 376a, 376b and impelled toward the distal end section of the insertion unit 314 so that it can move in the direction of arrow $\alpha$ 2. The impelling forces of the compression springs 378a, 378b are set to less than the tension of the belt 338. As a result, the compression springs 378a, 378b can be caused to contract. The friction force between the outer circumferential surface of the spherical body 354 and friction wheel 374 is set to a value such that the needle-holding section 316 can be rotated by an adequate operation power when the anastomosis needle 318 is handled during the rotation of the friction wheel 374.

As shown by a broken line in Fig. 60, a pair of grooves 382 provided so that the rotary shaft 324a of the needle-holding unit rotary dial 324 can slide in the direction of arrow $\alpha$ 5 are formed in the grip 322.

A belt 338 shown in Fig. 59 links the needle-holding unit rotary dial 324 and the friction wheel 374 together so that the rotation of the needle-holding unit rotary dial 324 shown in Fig. 60 can be transmitted to the friction wheel 374. Here, the belt 338 can be extended and contracted to a certain degree, and the friction wheel 374 is pressed by the compression springs 378a, 378b against the outer circumference of the spherical body 354. In this state, the tension enabling the transmission of the rotary power is constantly applied to the belt 338.

The operation of the needle-holding device 310 of the above-described configuration will be described below.

When the surgeon wishes to incline the needle-holding section 316 with respect to the insertion unit 314, he/she rotates the needle-holding unit rotary dial 324 shown in Fig. 60 in the direction of arrow $\alpha$ 5. Under the tension of the belt 338 that is wound about the rotary dial 324, the rotary shaft 374a of the friction wheel 374 moves toward the proximal end section (direction of arrow $\alpha$ 2) of the insertion unit 314 along the grooves 376a, 376b against the impelling force of the compression springs 378a, 378b. As a result, the friction wheel 374 goes away from the outer circumferential surface of the spherical body 354 of the needle-holding unit body 342. In this state, when an external force is applied to the needle-holding unit 316 to cause the inclination along the long orifice 364a of the desired quantity about the center of the spherical body 354 as a center and the desired quantity of inclination is attained, the hand is removed from the needle-holding unit rotary dial 324. As a result, the friction wheel 374 is moved toward the distal end section of the insertion unit 314 along the grooves 376a, 376b and pressed against the outer circumference of the spherical body 354 by the elastic forces of the compressive springs 378a, 378b. As a result, the described inclined state of the needle-holding section 316 is maintained by the friction (brake action) between the friction wheel 374 and spherical body 354. In this state, as is explained in the second embodiment, the anastomosis needle 318 is held and the anastomosis operation is performed.

As described hereinabove, with the third embodiment, the following effect is obtained in addition to the effect explained in the second embodiment.

When the inclination angle of the needle-holding section 316 is adjusted to the adequate angle, the adjustment can be carried out with an optimum power. Furthermore, during the anastomosis operation, the inclination angle can be strictly maintained.

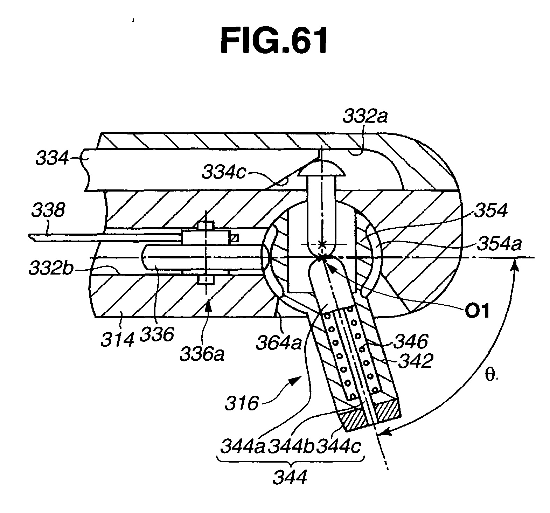

A fourth embodiment will be described below with reference to Fig. 61. The fourth embodiment is a variation example of the second embodiment, and the components identical to those explained in the second embodiment will be denoted with the same symbols and the detailed explanation thereof will be omitted.

As shown in Fig. 61, in a state where the tooth sections 336b of the gear 336 are engaged in the vicinity of the center in the width direction of the tooth sections 354a of the spherical body 354, the needle-holding unit body 342 is tilted from the direction perpendicular to the lengthwise direction of the insertion unit 314. In the case shown in Fig. 61, the needle-holding unit body 342 is tilted toward the distal end of the insertion unit 314. The long orifice 364a formed in the side of the distal end section of the insertion unit 314 is formed so that the needle-holding unit body 342 is disposed in the center in the state where the tooth sections 336b of the gear 336 are engaged in the vicinity of the center in the width direction of the tooth sections 354a of the spherical body 354. For this purpose, the inclination angle $\theta$ of the needle-holding unit body 342 with respect to the lengthwise axis of the insertion unit 314 extending further toward the distal end side than the distal end section of the insertion unit 314 is set to less than 90°. The angle at this time can be set appropriately to the inclination angle suitable for the anastomosis. Other aspects of the structure are identical to those of the structure explained in the second embodiment.

In a fourth embodiment, the configuration is such that the inclination angle $\theta$ with respect to the lengthwise direction of the insertion section 314 is set to less than 90°, but the same action can be attained with the configuration in which the inclination angle $\theta$ is set larger than 90°.

The operation of the needle-holding device 310 of the above-described configuration will be described below.

Similarly to the action explained in the second embodiment, the needle-holding unit body 342 is inclined along the long orifice 364a. The needle-holding unit body 342 is further inclined so that the angle $\theta$ decreases as shown in Fig. 61 (the central axis of the needle-holding unit body 342 approaches the lengthwise axis of the insertion unit 314) from the state where the needle-holding unit body is disposed in the center of the long orifice 364a of the insertion unit 314. In a state where the needle-holding unit body 342 is disposed in the center of the long orifice 364a, because the gear 336 is formed so as to be positioned in the center, in the tooth width direction, of the tooth sections 354a formed on the outer circumference of the spherical body 354, a sufficient movement range can be obtained by using as a reference the inclination angle suitable for anastomosis.

As described hereinabove, with the fourth embodiment, the following effect is obtained in addition to the effect explained in the second embodiment.

Even though the inclined movement range of the needle-holding unit body 342 is set to be identical to the range explained in the second embodiment, a small (large) inclination angle θ of the needle-holding unit body 342 with respect to the insertion unit 314 can be set.

Furthermore, the operation of causing the inclination by directly applying an external force to the needle-holding unit body 342 is employed as the input means for causing the inclination of the needle-holding unit body 342, but the needle-holding unit 316 can be also inclined by providing a configuration in which a wire or rod (not shown in the figures) is passed from the operation unit 312 inside the insertion unit 314 and the wire or rod can be pressed and pulled in the lengthwise direction of the long orifice 364a and operating the wire or rod with the operation unit 312.

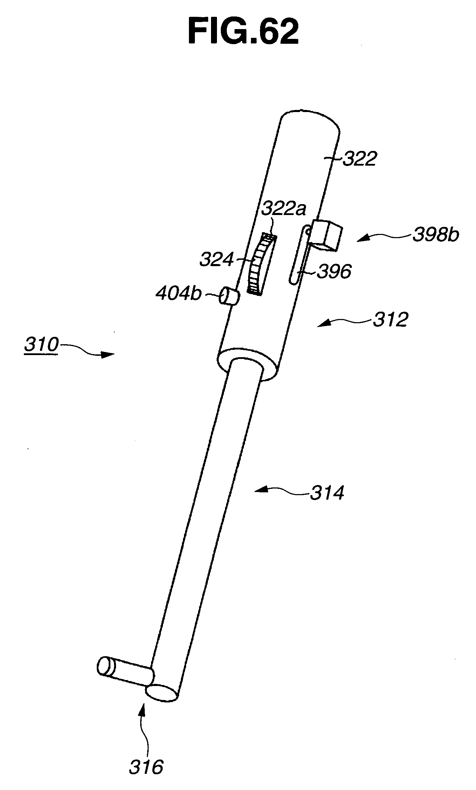

A fifth embodiment will be explained below with reference to Fig. 62 to Fig. 67.

As shown in Fig. 62, the needle-holding device (surgical instrument) 310 of the fifth embodiment comprises an operation unit 312, an insertion unit 314, and a needle-holding unit (treatment unit) 316. The needle-holding device 310 comprises a rotary mechanism for rotating the needle-holding unit 316, an angle variation switching mechanism for switching between a state where the angle of the needle-holding unit 316 is allowed to be changed and a state where this change is prohibited, and a holding section opening and closing mechanism for opening and closing the holding section 344c with respect to the needle-holding section body 342 so as to enable the clamping of the needle in the needle-holding unit 316.

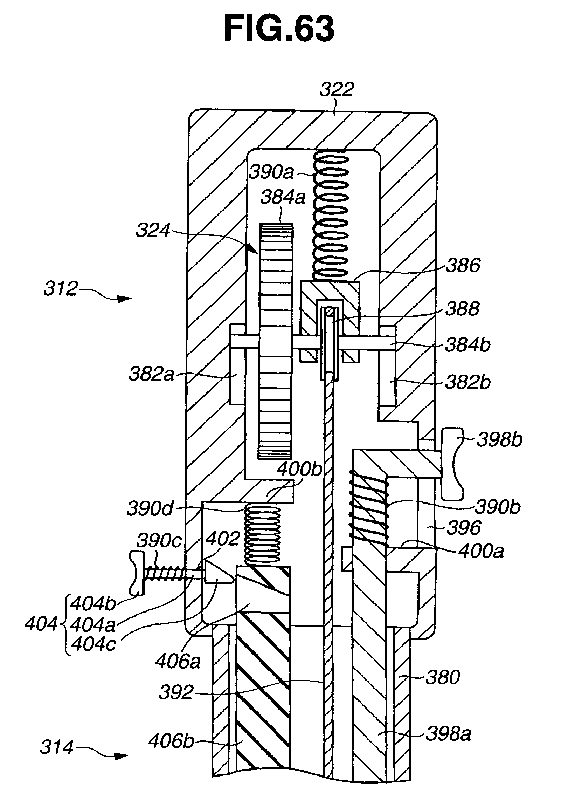

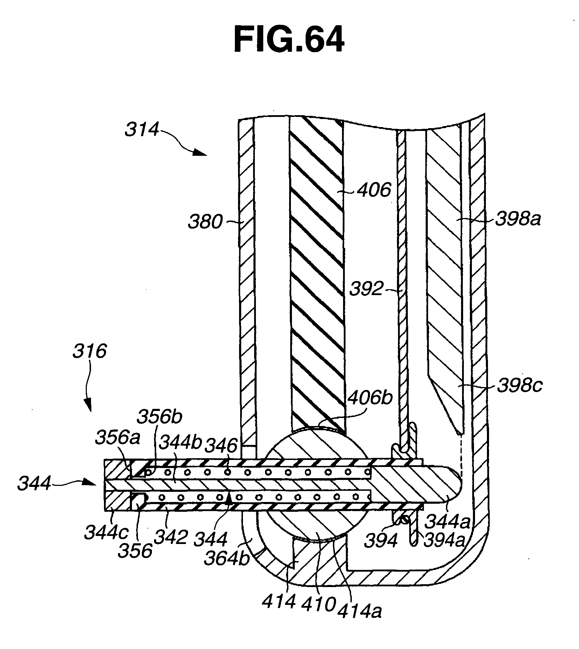

As shown in Fig. 63 and Fig. 64, the above-described rotary mechanism, angle variation switching mechanism, and holding section opening and closing mechanism are inserted into the grip 322 of the operation unit 312 and the insertion unit 314 comprising a sheath 380. As shown in Fig. 63, the proximal end section of the sheath 380 is integrally linked to the distal end section of the grip 322.

First, the rotary mechanism will be described. As shown in Fig. 63, a pair of bearing grooves 382a, 382b for receiving the below-described rotary shaft 384b of the rotary dial 324 are formed in the grip 322. Those bearing grooves 382a, 382b are formed to have the prescribed length along the lengthwise direction of the grip 322 on the inner side of the grip 322.

The rotary dial 324 comprises a disk body 384a and a rotary shaft 384b passing through the center of the disk body 384a and fixedly attached thereto. An operation unit pulley pulling member 386 with a substantially U-shaped cross section and an operation unit pulley 388 disposed on the inner side of the operation unit pulley pulling member 386 are disposed at the side of the disk body 384a. The rotary shaft 384b passes through the disk body 384a, operation unit pulley pulling member 386, and operation unit pulley 388. The operation unit pulley 388 is fixedly attached to the rotary shaft 384b. The operation unit pulley pulling member 386 freely rotates with respect to the rotary shaft 384b. Both ends of the rotary shaft 384b are supported by respective bearing grooves 382a, 382b. Part of the disk body 384a of the rotary dial 324 protrudes from the grip 322 through notches 322a (see Fig. 62) in both sides provided in the grip 322 so that the surgeon can perform the rotational operation. Therefore, when the disk body 384a is rotated, the operation unit pulley 388 rotates together with the rotary shaft 384b. The operation unit pulley pulling member 386 retains the position thereof even when the rotary shaft 384b rotates.

The operation unit pulley pulling member 386 is connected with a coil spring 390a to the inner surface (upper end in Fig. 63) of the proximal end section of the grip 322 (end section on the remote side from the needle-holding unit (clamping unit) 316). This coil spring 390a impels the operation unit pulley pulling member 386 toward the proximal end side of the grip 322 (upper side in Fig. 63). As a result, the disk body 384a, rotary shaft 384b, operation unit pulley pulling member 386, and operation unit pulley 388 are impelled toward the proximal end side of the grip 322 (upper side in Fig. 63).

A wire (operation power transmission member) 392 is wound about the operation unit pulley 388. This wire 392 extends to the distal end side of the insertion unit 314. As a result, when the disk body 384a is rotated, the wire moves back and forth along the lengthwise direction of the insertion unit 314.

As shown in Fig. 64, the needle-holding unit 361 comprises a cylindrical needle-holding unit body 342, a needle-holding rod 344, and a coil spring 346. One end section (right end in Fig. 64) and the other end section (left end section, as shown in Fig. 64) of the cylindrical needle-holding unit body 342 are open, and a flange section 356 protruding radially and inwardly is formed in the other end section.

A needle-holding rod 344 is inserted inside the needle-holding unit body 342. The needle-holding rod 344 comprises a head section 344a, a rod section (slide shaft) 344b, and a holding section (clamping section) 344c.

The head section 344a can slide along the inner circumferential surface of the needle-holding unit body 342 in one end section (right end in Fig. 64) of the needle-holding unit body 342.

The rod section 344b is connected by one end section thereof (right end in Fig. 64) to the other end section (left end in Fig. 64) of the head section 344a and extends toward the other end section (holding section 344c) of the needle-holding unit body 342 on the central axis of the needle-holding unit body 342. The holding section 344c is provided in the other end section (left end in Fig. 64) of the rod section 344b and can be brought into contact with and going away from a left end surface (referred to as "the end surface on the side of the holding unit") 356a, as shown in Fig. 64, of the flange section 356 at the other end of the needle-holding unit body 342.

The holding section 344c is formed to have a ring-like shape and joined, for example, by laser welding to the other end section (left end in Fig. 64) of the rod section 344b. The holding section 344c is provided on the outside of the other end section of the needle-holding unit body 342 and can be brought into contact and going away from the end surface 356a on the side of the holding unit of the flange section 356.

A coil spring 346 is provided between the other end of the head section 344a of the needle-holding rod 344 and the right end surface (referred to hereinbelow as "end surface on the side of the elastic member") 356b, as shown in Fig. 64, of the flange section 356 of the needle-holding unit body 342. The coil spring 346 is placed, while being compressed, between the end surface 356b on the side of the elastic member of the flange section 356b and one end of the head section 344a of the needle-holding rod 344. As a result, the head section 344a is impelled toward one end side of the needle-holding unit body 342 by the compression force of the coil spring 346. Therefore, the holding unit 344c is constantly impelled by the prescribed force toward the end surface 356a on the side of the holding unit of the flange section 356. As a consequence, for example, when the anastomosis needle 318 (see Fig. 56(B)) is not clamped between the end surface 356a on the side of the holding unit of the flange section 356 and the holding section 344c, the holding section 344c is impelled, in the abutting state thereof, by the prescribed force to the end surface 356a on the side of the holding unit of the flange section 356. On the other hand, the anastomosis needle 318 (see Fig. 56(B)) is clamped between the end surface 356a on the side of the holding unit of the flange section 356 of the needle-holding unit body 342 and the holding section 344c of the needle-holding rod 344.

A pulley (rotary body) 394 having a central axis on the same axis as the central axis of the needle-holding unit body 342 is mounted (fixedly attached) on the outer peripheral surface of the other end section of the cylindrical needle-holding unit body 342. The wire 392 is wound about the pulley 394. As a result, the pulley 394 and operation unit pulley 388 (see Fig. 63) are linked together by the wire 392. At this time, the wire 392 is dragged toward the proximal end side (upper side in Fig. 63 and Fig. 64) of the grip via the operation unit pulley pulling member 386, rotary shaft 384b, and operation unit pulley 388 by the coil spring 390a provided between the operation unit pulley pulling member 386 and the proximal end section of the grip 322 shown in Fig. 63.

The holding section opening and closing mechanism will be described below. As shown in Fig. 63, a long orifice 396 is formed along the lengthwise direction of the grip 322 in the side section of the grip 322 at the side shifted to the distal end from the bearing grooves 382a, 382b of the grip 322. The long orifice 396 links the inside of the grip 322 to the outside. A L-shaped member 398a curved to have an L-like shape is provided in the long orifice 396, the proximal end section of this member protrudes to the outside of the grip 322, and the distal end section thereof extends toward the distal end side of the insertion unit 314. An opening and closing knob 398b is installed at the proximal end section of the L-shaped member 398a. A first protruding section 400a that protrudes inward of the grip 322 is formed in the distal end section of the long orifice 396 of the grip 322. A coil spring 390b is provided on the outer periphery of the L-shaped member 398a between the curved section of the L-shaped member 398a and the first protruding section 400a. A through hole through which the L-shaped member 398a is passed is formed along the lengthwise direction of the grip 322 in the first protruding section 400a. Because of this through hole, the L-shaped member 398a is slidably held on a straight line along the lengthwise direction of the insertion unit 314.

As shown in Fig. 64, a wedge section 398c having a taper surface is formed in the distal end section of the L-shaped member 398a. The wedge section 398c is disposed, from the side of the operation unit 312, facing the apex of the spherical part of the head section 344a of the needle-holding rod 344. The distal end of the taper surface provided in the wedge section 398c and the apex of the spherical part of the head section 344a of the needle-holding rod 344 are disposed so as to be aligned on a substantially straight line. As a result, when the surgeon moves the opening and closing knob 398b shown in Fig. 63 toward the distal end side of the grip 322 along the long orifice 396 against the impelling force of the coil spring 390b and moves the L-shaped member 398a toward the distal end side (downward in Fig. 64) of the insertion unit 314, the wedge section 398c can come into contact with the head section 344a of the needle-holding rod 344.

The angle variation switching mechanism will be described below. As shown in Fig. 63, a through hole 402 is formed in the direction perpendicular to the lengthwise direction of the grip 322 in the position substantially facing the aforementioned long orifice 396 in a side section at the distal end of the grip 322. An angle variation switching button (brake operation unit) 404 is provided in the through hole 402. The angle variation switching button 404 comprises a shaft section 404a that can slide along the through hole 402, a button section 404b that is mounted on one end of the shaft section 409a and provided outside the grip 322, and a wedge member 404c linked to the outer end of the shaft section 404a and provided inside the grip 322. A coil spring 390c is provided on the outer circumferential surface of the shaft section 404a between the button section 404b and the outer circumferential surface of the grip 322. As a result, the button section 404b is impelled so that the wedge member 404c is always going away from an orifice 406a having the inclined surface of the below-described brake member 406.

A second protruding section 400b that protrudes inward inside the grip 322 is formed on the proximal end side (upper side in Fig. 63) of the grip 322 from the above-described through hole 402. One end section (upper end in Fig. 63) of a coil spring 390d is supported on the surface of the second protruding section 400b on the distal end side of the insertion unit 314. A proximal end section of the brake member 406 extending toward the distal end side along the lengthwise direction of the insertion unit 314 is mounted on the other end section (lower end in Fig. 63) of the coil spring 390d. The coil spring 390d impels the brake member 406 toward the distal end side (lower side in Fig. 63) of the insertion unit 314. The orifice 406a into which the above-described wedge member 404c is removably inserted is formed in the proximal end section of the brake member 406. This orifice 406a comprises a slanted surface section abutting against the slanted surface section of the wedge member 404c.

As shown in Fig. 64, a cylindrical member (cylindrical rod-shaped member) 410 is provided on the outer circumferential surface of the needle-holding unit body 342. A through hole having provided therein with the needle-holding unit body 342 is formed in this cylindrical member 410 in the direction perpendicular to the central axis thereof. The needle-holding unit body 342 can rotate with respect to the through hole of the cylindrical member 410.

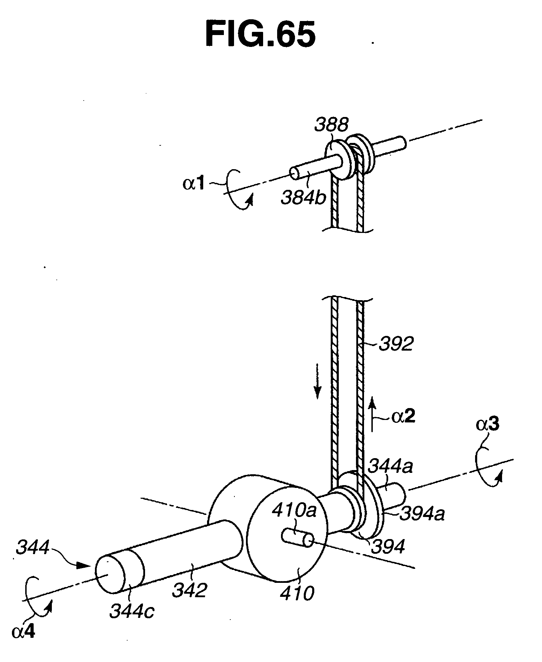

As shown in Fig. 65, a pair of pivot shafts 410a are provided on the central axis of the cylindrical member 410. Those pivot shafts 410a are inserted into orifices (not shown in the figures) provided in the sheath 380 of the insertion unit 314. Those pivot shafts 410a are provided in the direction perpendicular to the axial direction of the lengthwise direction of the insertion unit 314 and also in the direction perpendicular to the axial direction of the needle-holding unit body 342.

As shown in Fig. 64, a protruding section 414 extending from the distal end to the proximal end side of the insertion unit 314 is formed in the distal end section of the sheath 380 of the insertion unit 314. A cylindrical contact surface 414a having the shape identical to that of the outer circumferential surface of the cylindrical member 410 is formed, so as to support part of the outer circumferential surface of the cylindrical member 410, in the surface (upper end in Fig. 64) of the protruding section 414 on the proximal end side of the insertion unit 314.

In the above-described brake member 406, a brake surface (abutment surface) 406b is formed so as to face the cylindrical contact surface 414a. This brake surface 406b is preferably roughened by cutting or discharge processing. Furthermore, it may be also roughened by causing particles of an ultrahard material such as diamond and sapphire to adhere thereto by metal plating. The central axis of the brake member 406 is perpendicular to the central axis (pivot shaft 410a) of the cylindrical member 410 and positioned on the central axis of the protruding section 414 supporting the cylindrical contact surface 414a.

The operation of the needle-holding device 310 of the above-described configuration will be described below.

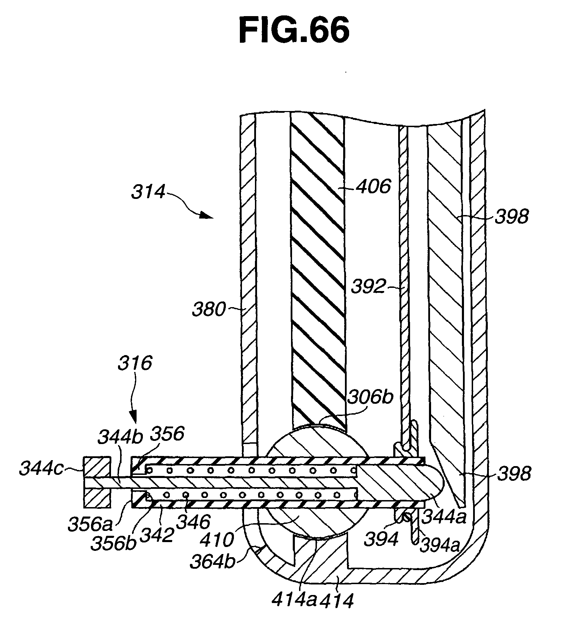

The surgeon causes the opening and closing knob 398b shown in Fig. 63 to slide to the distal end section of the insertion unit 314 along the long orifice 396 of the grip 322. The wedge section 398c of the distal end section of the L-shaped member 398a moves toward the distal end section of the insertion unit 314. As shown in Fig. 66, the slanted surface section of the wedge section 398c abuts against the spherical section of the head section 344a of the needle-holding rod 344 and causes the head section 344a to move toward the distal end side (downward in Fig. 66) of the insertion unit 314, so as to cause the head section to slide toward the other end side (leftward in Fig. 66) of the needle-holding rod 344. That is, the shaft section (slide shaft) 344a of the needle-holding rod 344 is caused to slide along the axial direction of the needle-holding unit body 342 of the insertion unit 314. At this time, the coil spring 346 is compressed between the head section 344a of the needle-holding rod 344 and the end surface 356b on the side of the elastic member of the flange section 356 of the needle-holding unit body 342. As a result, the holding section 344c goes away the end surface 56a on the side of the holding unit of the flange section 356 of the needle-holding unit body 342 against the impelling force of the coil spring 346, and a gap is produced for holding the anastomosis needle 318 between the holding section 344c and the flange section 356 of the needle-holding unit body 342. At this time, the anastomosis needle 318 is disposed between the holding section 344c and the flange section 356 of the needle-holding unit body 342.

When the surgeon removes the finger from the opening and closing knob 398, the L-shaped member 398a is moved toward the proximal end side of the insertion unit 314 by the impelling force of a coil spring 390b located inside the grip 322. Thus, the wedge section 398c is caused to move to the original position and goes away from the head section 344a of the needle-holding rod 344. As a result, the head section 344a and shaft section (slide shaft) 344b tend to be automatically returned to the original positions by the impelling force (spring force) of the coil spring 346, and the holding section (ring member) 344c moves in the direction of abutting against the end surface 356a on the side of the holding unit of the flange section 356 of the needle-holding unit body 342.

Because the coil spring 346 is impelled toward one end side (right side in Fig. 64) in the lengthwise direction of the needle-holding rod 344 between the flange section 356 of the needle-holding unit body 342 and the head section 344a of the needle-holding rod 344, a compressive force is constantly generated between the flange section 356 of the needle-holding unit body 342 and the holding section 344c. As a result, the anastomosis needle 318 (refer to Fig. 56(B)) can be held by a sufficient holding force between the holding section 344c and the flange section 356.

When the surgeon rotates the rotary dial 324 in the direction of arrow α1, as shown in Fig. 65, the rotary shaft 384b rotates via the disk body 384a. As a result, the operation unit pulley 388 rotates and the wire 392 moves in the direction of arrow α2, following the rotation of the operation unit pulley 388. Because of this movement of the wire 392, the pulley 394 rotates in the direction of arrow α3 and the needle-holding unit body 342 rotates in the direction of arrow α4. That is, due to the rotation of the rotary dial 324, a rotary power is transmitted to the pulley 394 via the disk body 384a, rotary shaft 384b, operation unit pulley 388, and wire 392, and the needle-holding unit 316 rotates. At this time, the needle-holding unit body 342 rotates about the central axis of the needle-holding unit body 342 with respect to the cylindrical member 410. As a result, in the state where the anastomosis needle 318 is held, the anastomosis needle 318 can be freely rotated about the axis of the needle-holding unit body 342.

In the brake member 406, the brake surface 406b is impelled and brought into contact against the outer circumferential surface of the cylindrical member 410 by the coil spring 390d located at the proximal end of the brake member 406. That is, the brake surface 406b of the brake member 406 is pressed against the outer circumferential surface of the cylindrical member 410. As a result, the cylindrical member 410 is held, so that it does not move around the pivot shaft 410a, between the brake surface 406b and the cylindrical contact surface 414a.

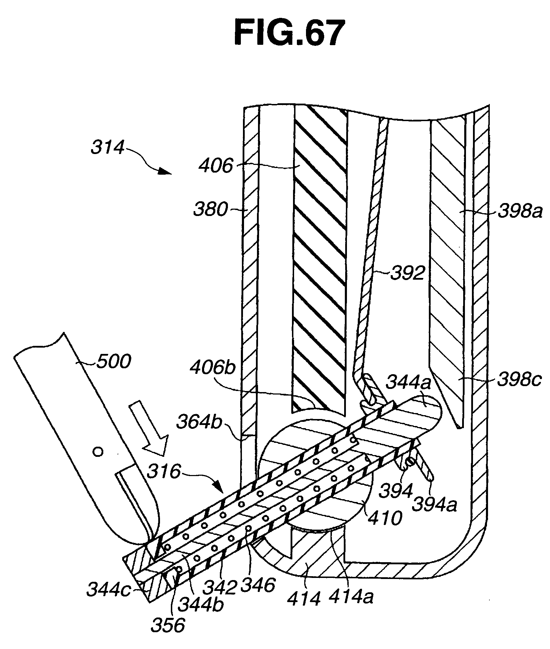

When the surgeon presses the angle variation switching button section 404b into the grip 322, the inclined surface of the wedge member 404c comes into contact with the inclined surface of the orifice section 406a of the brake member 406, slides over the inclined surface and causes the orifice section 406a to move toward the proximal end side of the grip 322. When the orifice section 406a of the brake member 406 moves toward the proximal end side of the grip 322, as shown in Fig. 67, the brake surface 406b of the brake member 406 goes away from the cylindrical member 410 and the brake is released. As a result, the cylindrical member 410 assumes a state where it can freely rotate about the axis of the pivot shaft 410a.

In this state, if the surgeon presses the needle-holding unit body 342, e.g., by the grasping forceps 500 gripped by another hand of the surgeon inside the body cavity, as shown in Fig. 67, then the angle of protrusion of the needle-holding unit body 342 with respect to the lengthwise direction of the insertion unit 314 is changed within the range of the long orifice 364b formed in the side section of the distal end section of the insertion unit 314. If the pressing of the angle variation switching button section 404b is released when the needle-holding unit body 342 is at the desired angle with respect to the lengthwise direction of the insertion unit 314, then the wedge member 404c is pulled out from the orifice section 406a of the brake member 406 by the impelling force of the coil spring 390d and the brake member 406 moves toward the distal end section of the insertion unit 314 by the impelling force of the coil spring 390. As a result, the brake surface 406b of the brake member 406 is again abutted against the cylindrical member 410 and the angle of the needle-holding unit body 342 is maintained.

As shown in Fig. 64, the coil spring 390a constantly impels the operation unit pulley 388 and rotary shaft 384b in the direction of pulling toward the proximal end side of the grip 322 via the operation unit pulley pulling member 386. As a result, a tension is always applied to the wire 392.

As shown in Fig. 67, if the angle of protrusion of the needle-holding unit body 342 with respect to the distal end section of the insertion unit 314 is changed, the wire 392 is inclined with respect to the lengthwise axis of the sheath 380. As a result, if the position of the rotary dial 324 shown in Fig. 63 is assumed to be same, the length of the wire 392 between the pulley 394 and operation unit pulley 388 will become different from the one before the wire 392 is inclined. The coil spring 390a fixedly attached between the operation unit pulley pulling member 386 and the abutment surface of the proximal end section of the grip 322 extends and the entire rotary dial 324, that is, the rotary shaft 384b of the disk body 384a moves along the bearing grooves 382a, 382b, and the operation unit pulley 388 and operation unit pulley pulling member 386 move toward the distal ends side. As a result, the length of the wire 392 is made consistent and the state in which the prescribed tension is constantly applied to the wire 392 can be maintained.

When the angle variation button section 404b is pressed and the brake surface 406b is released after the angle has been changed, because the tension is constantly applied to the wire 392, the wire 392 generates a force trying to return the needle-holding unit body 342 to the original position via the pulley 394. The needle-holding unit body 342 is automatically returned to the original position.

As described above, the following effect can be obtained with the fifth embodiment.

Because the angle of the needle-holding unit body 342 can be changed according to the procedure state, it is not necessary to shift to another surgical tool and usability can be increased. Moreover, the surgery duration can be shortened and the degree of intrusion with respect to the patient can be reduced.

The needle-holding unit 316 maintains the state of holding the anastomosis needle 318 independently, that is, without the application of external force, and the holding power transmission unit provides absolutely no effect on the load of the rotational operation, as in the conventional devices. Therefore, the rotation is smoothly performed even in a state where the holding object is held by the holding unit, and usability can be improved. In addition, because the surgeon is not required to apply constantly a power to the needle-holding device 310 in order to hold the holding object, the operability is very good. The surgeon can concentrate his attention on the rotation of the holding object (held object) and other operations.

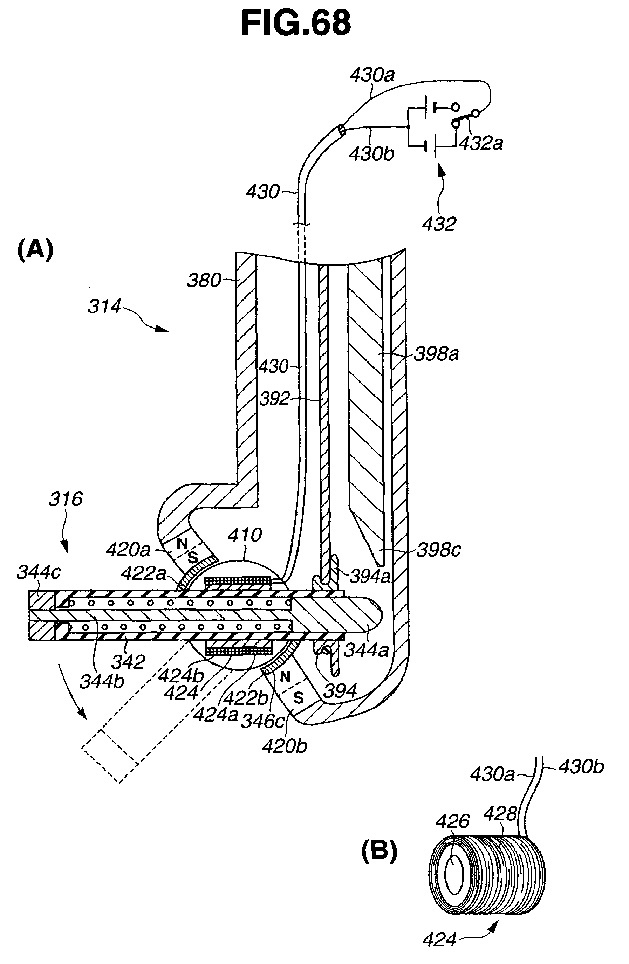

A sixth embodiment will be described below with reference to Fig. 68. The sixth embodiment is a variation example of the fifth embodiment, the components explained in the fifth embodiment are assigned with the same reference symbols and the detailed explanation thereof will be omitted. Only the components different from those of the fifth embodiment will be explained.

As shown in Fig. 68(A), the protrusion section 414 and brake member 406 shown in Fig. 64 are removed from the inside of the sheath 380 of the insertion unit 314.

A long orifice 364c is formed in the distal end section of the sheath 380 of the insertion unit 314. First and second permanent magnets 420a, 420b are provided in respective edge sections of the long orifice 364c. In the first permanent magnet 420a, the S pole is disposed close to the second permanent magnet 420b. In the second permanent magnet 420b, the N pole is disposed close to the first permanent magnet 420a. A first sliding surface member 422a is provided at the S pole of the first permanent magnet 420a. A second sliding surface member 422b is provided at the N pole of the second permanent magnet 420b. The cylindrical member 410 slidably and rotatably abuts against and is supported by the mutually opposite surfaces of the first and second sliding surface members 422a, 422b. Because those first and second sliding surface members 422a, 422b and the outer circumferential surface of the cylindrical member 410 are brought into contact with each other, the rotation of the cylindrical member 410 is impeded. The cylindrical member 410 is held on the sheath 380 of the insertion unit 314 by the pivot shaft 410a (see Fig. 65).

An electromagnet (solenoid) 424 is mounted on the outer circumferential surface of the needle-holding unit body (rotation member) 342 inside the cylindrical member 410. As shown in Fig. 68(B), the electromagnet 424 is formed by multi-turn winding of a conductive wire 428 on a core 426. The core 426 is formed of cylindrical material with a high magnetic permeability, for example, iron.

As shown in Fig. 68(A), both ends of the wire material of the conductive wire 428 of the electromagnet 424 are electrically connected to respective electric wires 430a, 430b. Those electric wires 430a, 430b are disposed in the operation unit 312 via the sheath 380 of the insertion unit 314. A power-source switch unit 432 is provided in the operation unit 312. A switch 432a of the power-source switch unit 432 is operated by a press-button switch (not shown in the figures) provided in the operation unit 312. In the state of the switch 432a shown in Fig. 68(A), the position of the electromagnet 424 shown by the reference symbol 424a (right side in Fig. 68(A)) is the S pole, and the position shown by the reference symbol 424b (left side in Fig. 68(A)) is the N pole.

The operation of the needle-holding device 310 of the above-described configuration will be described below.

The respective attraction forces act between the N pole of the electromagnet 424 that is shown by the reference symbol 424b and the S pole of the first permanent magnet 420a and between the S pole of the electromagnet 424 that is shown by the reference numeral 424a and the N pole of the second permanent magnet 420b. Therefore, in the state where the position of the electromagnet 424 that is shown by the reference symbol 424a in Fig. 68(A) is magnetized into the S pole, the position of the needle-holding unit body 342 is maintained in the state shown in Fig. 68(A).

When the switch 432a of the power-source switch unit 432 is switched, the polarity of the electromagnet 424 is reversed. The position of the electromagnet 424 that is shown by the reference numeral 424a in Fig. 68(A) (right side on the paper sheet) is switched to the N pole, and the position shown by the reference symbol 424b (left side on the paper sheet) is switched to the S pole. As a result, the respective repulsion forces act between the S pole of the electromagnet 424 shown by the reference numeral 424b and the S pole of the first permanent magnet 420a and between the N pole of the electromagnet 424 shown by the reference symbol 424a and the N pole of the second permanent magnet 420b. Because the first and second permanent magnets 420a, 420b do not move, the cylindrical member 410 rotates about the central axis (pivot shaft 410a) as a support point, while sliding over the sliding surface members 422a, 422b. As a result, the needle-holding unit body 342 rotates. At this time, the rotation continues till the N pole of the electromagnet 424 shown by the reference symbol 424a comes close to the S pole of the first permanent magnet 420a and the S pole of the electromagnet 424 shown by the reference numeral 424b comes close to the N pole of the second permanent magnet 420b. Furthermore, the needle-holding unit body 342 rotates till the needle-holding unit body 342 abuts against the first sliding surface member 422a. That is, the edge sections of the first and second sliding surface members 422a, 422b are established as the edge sections of the long orifice 364c.

As described hereinabove, with the sixth embodiment, the following effects can be obtained.

In the above-described fifth embodiment, an external force, for example from other forceps 500 (see Fig. 67) is necessary to change the angle of the needle-holding unit 316, but in the sixth embodiment, this external force is not necessary and the angle of the needle-holding unit body 342 can be changed merely by operating the switch 432a. Therefore, the usability of the needle-holding device 310 can be improved. At this time, the needle-holding device 316 can be shifted between the position shown by a solid line in Fig. 68(A) and the position shown by a broken line by switching the switch 432a of the power-source switch unit 432.

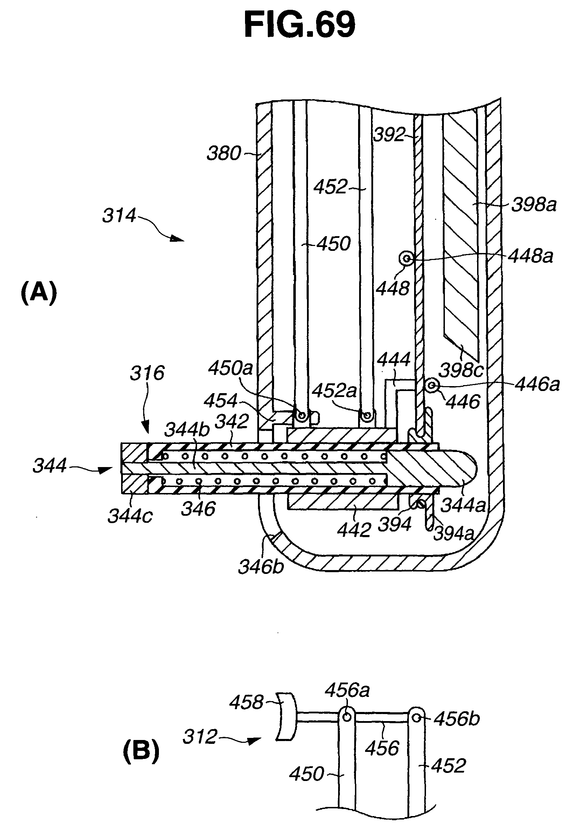

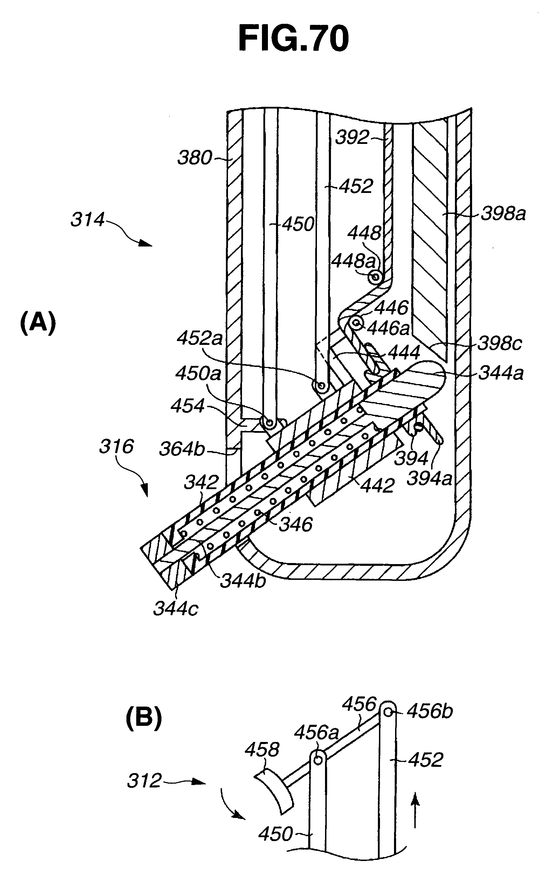

A seventh embodiment will be described below with reference to Figs. 69 and 70. The seventh embodiment is a variation example of the fifth embodiment, the components explained in the fifth embodiment are assigned with the same reference symbols and the detailed explanation thereof will be omitted.

A sleeve 442 is provided on the outer circumferential surface of the needle-holding unit body 342. That is, the needle-holding unit body 342 is inserted into the sleeve 442. One end of an L-shaped wire pulling member 444 is fixedly attached to one end section (right end in Fig. 69(A)) of the sleeve 442. A first wire receiving pulley (wire receiving member) 446 is freely rotatably provided via a core (pivot shaft) 446a on the other end of the wire pulling member 444. The first wire receiving pulley 446 is disposed so as to be in contact in a state where the wire 392 is pulled. A second wire receiving pulley 448 is freely rotatavely provided at the sheath 380 via pivot shaft 448a, in a position shifted toward the side of the operation unit 312 from the first wire receiving pulley 446, with the position and the first wire receiving pulley 446 sandwiching the wire 392. The first and second wire receiving pulleys 446, 448 are mutually arranged so as to sandwich the wire 392 and maintain the linear state of the wire 392.

First and second link rods 450, 452 are connected to the sleeve 442 via the pivot shafts 450a, 452a, respectively. Those link rods 450, 452 can freely rotate parallel to each other about the respective pivot shafts 450a, 452a as centers.

A fixing stand 454 protruding inward in the sheath 380 is provided at the distal end section of the sheath 380. The pivot shaft 450a is inserted into the fixing stand 454 and fixed thereto in order to prevent the pivot shaft 450a of the first link rod 450 from moving from its position.

The other end sections of the first and second link rods 450, 452 are linked to a link movement rod 456 via pivot shafts 456a, 456b in the grip (operation cover) 322 (here, not shown in the figure) of the operation unit 312. The first and second link rods 450, 452 can freely rotate with respect to the link movement rod 456 about the pivot shafts 450a, 452a as centers. As shown in Fig. 69(B), the aforementioned pivot shaft 456b is provided at one end of the link movement rod 456.

On the other hand, this pivot shaft 456a is inserted into an orifice (not shown in the figures) provided in the grip 322 and fixed thereto in order to prevent the pivot shaft 456a located on the side of the operation unit 312 from moving from its position.

As shown in Fig. 69(B), an angle variation knob (inclination operation section) 458 is fixedly attached to the other end section of the link movement rod 456. The angle variation knob 458 protrudes outward from the grip 322 so that it can be operated by the surgeon.

The operation of the needle-holding device 310 of the above-described configuration will be described below.

As shown in Fig. 70(B), when the surgeon moves the angle variation knob 458 toward the distal end side of the insertion unit 314, the link movement rod 456 rotates about the pivot shaft 456a as a center. Due to a parallel link effect, the sleeve 442 is rotated parallel to the link movement rod 456 via the pivot shaft 450a. As a result, as shown in Fig. 70(A), the angle of the needle-holding section body 342 can vary within the prescribed range, e.g., the range of the long orifice 364b.

When the angle of the needle-holding unit body 342 is changed, the wire 392 is pulled by the first and second wire receiving pulleys 446, 448. The mutual positional relation of the first wire receiving pulley 446 and pulley 394 is maintained such that the wire 392 is parallel to the flange 394a of the pulley 394.

The wire 392 that is stretched from the second wire receiving pulley 448 toward the operation unit 312 can be maintained parallel to the axial direction of the sheath 380 by the second wire receiving pulley 448. As a result, the pulley 394 and wire 392 are pulled with better stability when the angle of the needle-holding unit 316 is changed compared with the fourth and sixth embodiments.

Because a tension maintaining mechanism (first and second wire receiving pulleys 446, 448) of the wire 392 located between the operation unit pulley 388 and pulley 394 acts at this time, even if the consistency of the length in the axial direction of the wire 392 is lost, as shown in Fig. 65 of the fifth embodiment, this is absorbed by the coil spring 390a.

As described hereinabove, with the seventh embodiment, the following effects can be obtained.

By contrast with the fifth embodiment, the angle of the needle-holding unit 316 can be changed only by operating the angle variation knob 458 in the operation unit. Therefore, usability can be improved.

By contrast with the sixth embodiment, the angle of the needle-holding unit 316 can be changed continuously. Therefore, usability can be improved.

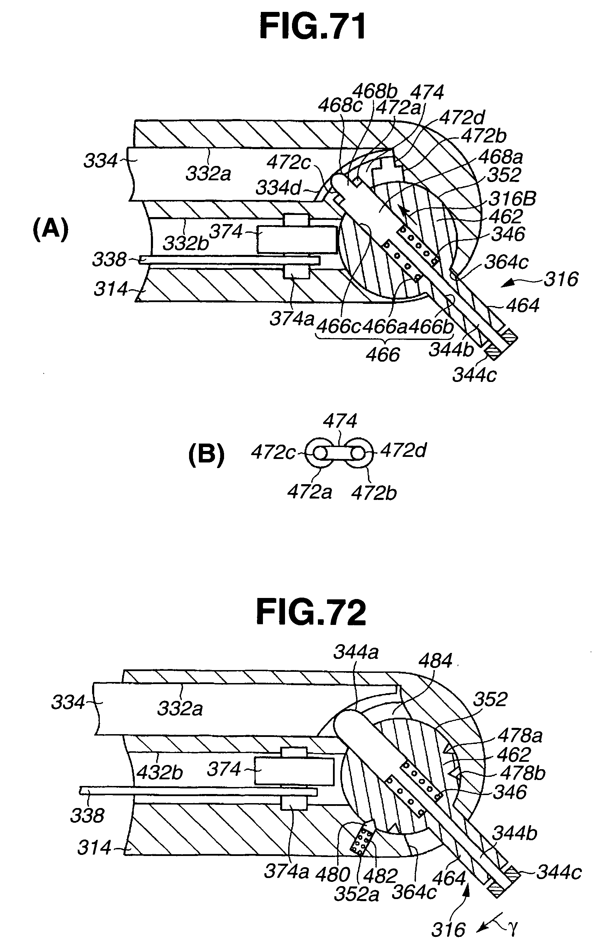

An eighth embodiment will be described below with reference to Fig. 71. The eighth embodiment is a variation example of the first embodiment, the components explained in the second embodiment are assigned with the same reference symbols and the detailed explanation thereof will be omitted.

As shown in Fig. 71(A), the needle-holding unit body 342a comprises a spherical body (holding section spherical body) 462 and a tubular section 464 extending from the spherical body 462, the two components being integrated with each other. The tubular section 464 extends radially and outwardly from the center of the spherical body 462. A through hole 466 passing through the central axis of the tubular section 464 and the center of the spherical body 462 is formed in the spherical body 462 and tubular body 464. The through hole 466 comprises a step section 466a inside the spherical body 462 and a small-diameter section is formed in the tubular section 464 and the spherical body 462 on the side of the tubular section 464 and a large-diameter section 466c with a diameter larger than that of the small-diameter section 466b is formed in the spherical body 462.

A needle-holding rod 344 and coil spring 346 are provided in the through hole 466. The needle-holding rod 344 comprises a head section 344a, a rod section 344b, and a holding section 344c. The head section 344a comprises a large-diameter section 468a connected to the rod section 344b and a small-diameter section 468b formed at the large-diameter section 468a. A spherical body section 468c is formed at the distal end (upper end in Fig. 71(A)) of the small-diameter section 468b.

The coil spring 346 is provided between the large-diameter section 468a of the head section 344a of the needle-holding rod 344 and the step section 466a of the large-diameter section 466c of the through hole 466. As a result, the needle-holding rod 344 is impelled in the direction such that the holding section 344c comes into contact with the distal end of the tubular section 464.

A plurality, for example two, of orifice sections (concave sections) 472a, 472b are formed in the socket section 352 of the distal end section of the insertion unit 314. The diameter of those orifice sections 472a, 472b is slightly larger than the outer diameter of the large-diameter section 468a of the head section 344a of the needle-holding rod 344. Small orifice sections 472c, 472d that are slightly larger than the outer diameter of the small-diameter section 468b of the head section 344a are formed in the centers of those orifice sections 472a, 472b. The small orifice sections 472c, 472d of the two orifice sections 472a, 472b are linked by the grooves 474.

A positioning mechanism for positioning the spherical body 462 with respect to the socket section 352 is thus formed.

A slide rod 334 that can slide in the axial direction is provided in the first guide section 332a of the insertion unit 314. A circular arc surface 334d that is in contact with the small-diameter section 468b of the head section 344a of the needle-holding rod 344 is formed in the distal end section of the slide rod 334.

A friction wheel 374 is provided at the distal end section of the second guide section 332b. The friction wheel 374 is constantly pressed against the spherical body 462 of the needle-holding unit body 342a. The friction wheel 374 comprises a rotary shaft 374a in the direction perpendicular to the axial direction of the insertion unit 314 at the distal end section of the second guide section 332b. A belt 338 is stretched over the rotary shaft 374a. The belt 338 is stretched over the rotary dial 324 (Fig. 56(A)) in the operation unit 312.

The operation of the needle-holding device 310 of the above-described configuration will be described below.

The surgeon causes the slide rod 334 to move toward the distal end side of the insertion unit 314 from the operation unit 312. The small-diameter section 468b of the head section 344a of the needle-holding rod 344 is pressed by the circular arc surface 334d of the slide rod 334 and the coil spring 346 located inside the spherical body 462 is contracted. The space between the distal end surface of the tubular section 464 and the holding section 344c is opened and the anastomosis needle 318 (see Fig. 56(B)) is positioned between the distal end surface of the tubular section 464 and the holding section 344c. In this state, the slide rod 334 is moved toward the proximal end side of the insertion unit 314. The anastomosis needle 318 is held (clamped) between the distal end surface of the tubular section 464 and holding section 344c by the elastic force of the coil spring 346.

The operation of rotating the rotary dial 324 (see Fig. 56(A)) of the operation unit 312 causes the movement of the belt 338 and the rotation of the friction wheel 374. Under the effect of friction between the friction wheel 374 and spherical body 462, the spherical body 462 and tubular section 464, and the needle-holding rod 344 rotate together with the anastomosis needle 318 about the axis of the through hole 466.

When the surgeon wishes to change the direction of the tubular section 464 or holding section 344c with respect to the insertion unit 314, he/she pulls the holding section 344c in the direction of going away from the distal end surface of the tubular section 464 and compresses the coil spring 346. The large-diameter section 468a of the head section 344a of the needle-holding rod 344 is pulled out from the orifice section 472a, the small-diameter section 468b is pulled out from the small orifice section 472c, the small-diameter section 468b is moved along the groove section 474, and large-diameter section 468a is inserted into the other orifice section 472b, and the small-diameter section 468b is inserted into the small orifice section 472d.

Thus, even when the direction of the needle-holding unit 316 is changed, the holding and rotation of the anastomosis needle 318 can be performed by similar operations.

As described above, with the present embodiment, the following effect can be obtained.

The direction of the needle-holding unit 316 can be changed in an easy manner and the unit can be held in a state with a stable direction.

A ninth embodiment will be described below with reference to Fig. 72. The ninth embodiment is a variation example of the eighth embodiment, the components explained in the eighth embodiment are assigned with the same reference symbols and the detailed explanation thereof will be omitted.

The needle-holding device 310 of the ninth embodiment comprises a positioning mechanism for positioning the spherical body 462 with respect to the socket section 352.

A plurality, for example two, of V-shaped grooves (concave sections) 478a, 478b are formed in the outer circumferential surface of the spherical body 462. Those V-shaped grooves 478a, 478b are formed in the outer circumferential surface of the spherical body 462 about the axis of the through hole 466. A concave section 352a is formed in the inner circumferential surface of the socket section 352 of the insertion unit 314. A compression spring 482 is provided in the bottom section of the concave section 352a. A pin 480 that can be engaged with the above-described V-shaped grooves 478a, 478b is provided above the compression spring 482 inside the concave section 352a.

A groove section 484 of the shape enabling the rotation and movement of the needle-holding rod 344 is formed in the distal end section of the insertion unit 314. Other aspects of configuration are identical to those of the eighth embodiment.

The operation of the needle-holding device 310 of the above-described configuration is described below.

The belt 338 is moved and the friction wheel 374 is rotated by rotating the rotary dial 324 (see Fig. 56(A)) of the operation unit 312. The spherical body 462, tubular section 464, and needle-holding rod 344 rotate together with the anastomosis needle 318 about the axis of the needle-holding rod 344 due to the friction between the friction wheel 374 and spherical body 462. At this time, the spherical body 462 rotates about the pin 480 along the V-shaped groove 478a, while the V-shaped groove 478a and pin 480 maintain the engaged state.

When the direction of the needle-holding unit 316 is changed with respect to the insertion section 314, the tubular section 464 is pressed by the finger in the direction of arrow γ with respect to the insertion unit 314. As a result, the pin 480 is pressed in the V-shaped groove 478a, the compression spring 482 is compressed and the pin 480 is pulled into the concave section 352a. In this state, the needle-holding rod 344 moves along the groove section 484, and the pin 480 is engaged with the other V-shaped groove 478b.

Even when the direction of the needle-holding unit 316 is thus changed, the action of holding the needle 318 and rotating the tubular section 464 can be performed by the operations similar to the above-described operations.

As described hereinabove, the effect obtained with the ninth embodiment is identical to that obtained in the eighth embodiments.

Several embodiments were explained hereinabove with reference to the appended drawings, but the present invention is not limited to the above-described embodiments and includes all the possible implementation modes that can be realized without departing from the scope of the invention.

Industrial Applicability

with the present invention, endoscopic tissue suturing can be conducted easily and reliably. As a result, surgical operations conducted in the hospital are facilitated, the surgery time is shortened and surgery quality is improved. Therefore, the load on the patient is reduced, the stay period of the patient in the hospital is shortened, the patient can be rapidly returned to society, and the effective hospital management can be realized.

**Claims**

1. A surgical instrument comprising:

   an insertion unit;
   a treatment unit provided at one end of the insertion unit and capable of performing a plurality of actions;
   an operation unit provided at the other end of the insertion unit;
   a first power transmission mechanism, provided between the operation unit and the treatment unit along the insertion unit, for transmitting a first power, from the operation unit to the treatment unit, for causing the treatment unit to perform a prescribed first action;
   a second power transmission mechanism, provided between the operation unit and the treatment unit along the insertion unit, for transmitting a second power, from the operation unit to the treatment unit, for causing the treatment unit to perform a prescribed second action that differs from the first action;
   a third power transmission mechanism, provided between the operation unit and the treatment unit along the insertion unit, for transmitting a third power, from the operation unit to the treatment unit, for causing the treatment unit to perform a third action that differs from the first and second actions;
   a first operation mechanism provided in the operation unit for applying the first power to the first power transmission mechanism;
   a second operation mechanism provided in the operation unit for applying the second power to the second power transmission mechanism; and
   a third operation mechanism provided in the operation unit for applying the third power to the third power transmission mechanism.

2. The surgical instrument according to claim 1, wherein
   the treatment unit comprises a clamping unit which is freely rotatable about one axis as a center and can perform an opening and closing action and a bending action.

**3.** The surgical instrument according to claim 1 or 2, wherein
the first action is an action of rotating the treatment unit about one axis as a center.

**4.** The surgical instrument according to any one of claims 1 to 3, wherein
the second action is an action of opening and closing the treatment unit.

**5.** The surgical instrument according to any one of claims 1 to 4, wherein
the third action is an action of bending the treatment unit.

**6.** A surgical instrument comprising:

an insertion unit;
a treatment unit provided at one end of the insertion unit and comprising a clamping unit which is freely rotatable about one axis as a center and can perform an opening and closing action and a bending action;
an operation unit provided at the other end of the insertion unit;
a rotation power transmission mechanism, provided between the operation unit and the treatment unit along the insertion unit, for transmitting a rotation power, from the operation unit to the treatment unit, for rotating the treatment unit;
an opening and closing power transmission mechanism, provided between the operation unit and the treatment unit along the insertion unit, for transmitting an opening and closing power, from the operation unit to the treatment unit, for an action of opening and closing the clamping unit;
a bending power transmission mechanism, provided between the operation unit and the treatment unit along the insertion unit, for transmitting a bending power, from the operation unit to the treatment unit, for an action of bending the clamping unit;
an opening and closing operation mechanism, provided in the operation unit, for applying the opening and closing power;
a rotational operation mechanism, provided in the operation unit, for applying the rotation power; and
a bending operation mechanism, provided in the operation unit, for applying the bending power, wherein
the rotational operation mechanism and the bending operation mechanism are provided within the operation range of the same operation fingers among the fingers gripping the operation unit during all the prescribed operations.

**7.** The surgical instrument according to claim 6, further comprising a control holding unit provided in a condition of protruding sidewise from one side surface of the operation unit facing the position where the rotational operation mechanism and the bending operation mechanism are disposed.

**8.** The surgical instrument according to claim 6, wherein the opening and closing operation mechanism has an operation lever having a free end section inclined at a prescribed angle.

**9.** The surgical instrument according to any one of claims 6 to 8, wherein the rotational operation mechanism has a rotatable first operation dial having a disk-like shape.

**10.** The surgical instrument according to claim 9, wherein the bending operation mechanism has a rotatable second operation dial which has a disk-like shape and which is provided in the operation range of a dial operation finger that operates the operation dial among the fingers operating the operation unit with respect to the first operation dial.

**11.** The surgical instrument according to claim 10, wherein at least one operation dial from among the first operation dial and the second operation dial is provided to be able to rotate in the direction corresponding to the bending and stretching action of the operation fingers performing the operation of rotating the operation dial.

**12.** The surgical instrument according to claim 11, wherein the first operation dial is disposed in a position where the rotational operation is performed in a state where the operation fingers are stretched with respect to the second operation dial.

**13.** The surgical instrument according to claim 11, wherein the first operation dial is disposed in a position where the rotational operation is performed in a state where the operation fingers are bent with respect to the second operation dial.

14. The surgical instrument according to any one of claims 10 to 13, wherein the bending operation mechanism has a displacement direction conversion mechanism for converting the displacement due to the rotation of the second operation dial into the displacement in the long axis direction of the operation unit.

15. The surgical instrument according to any one of claims 10 to 14, wherein the rotational operation mechanism has a rotary direction conversion mechanism for converting the rotation of the first operation dial in the prescribed direction into the rotation about the axis as a center in the long axis direction of the operation unit.

16. The surgical instrument according to any one of claims 6 to 15, wherein the same operation finger from among the fingers gripping the operation unit is an index finger.

17. The surgical instrument according to any one of claims 8 to 16, further comprising a finger placement section, which is the outer circumferential surface of the operation unit, is provided on the extension line of the free end section of the operation unit, and serves for placing the operation finger for the operation lever.

18. The surgical instrument according to claim 17, wherein the placement surface for the operation finger for the operation lever in the finger placement section is a surface substantially identical to that of the distal end section of the operation lever in the position of a maximum height of the free end section of the operation lever.

19. The surgical instrument according to claim 17 or 18, wherein the operation finger for the operation lever is the thumb.

20. The surgical instrument according to claim 17, further comprising a finger hook section having an engagement surface to be brought into contact with one engagement finger from among the fingers gripping the operation unit, the section substantially protruding toward the rear surface side of the finger placement section in the outer circumferential surface of the operation unit.

21. The surgical instrument according to claim 20, wherein the engagement finger to be brought into contact with the finger placement section and gripping the operation unit is a middle finger.

22. The surgical instrument according to claims 10 to 21,
wherein the first operation dial and the second operation dial are provided between the finger placement section and finger hook section.

23. The surgical instrument according to claim 22, wherein the first operation dial and the second operation dial are provided between the finger placement section and finger hook section, and in a state where the operation finger for the operation lever is placed on the finger placement section and the engagement finger came into contact with the finger hook section, the first operation dial and second operation dial are provided in the range where the dial operation finger can move for operation.

24. A surgical instrument comprising:

an insertion unit;
an operation unit provided at one end of the insertion unit; and
a treatment unit provided so as to extend from the other end of the insertion unit and having a clamping unit, wherein
the operation unit comprises a rotational operation section for performing the operation of rotating the treatment unit, an opening and closing operation section for performing the operation of opening and closing the treatment unit, and an angle change operation section for performing the operation of changing the angle in the extension direction of the treatment unit;
the treatment unit can rotate about the axis of the treatment unit in response to the rotational operation in the rotational operation section and can execute the action of opening and closing the clamping unit in response to the opening and closing operation in the opening and closing operation section, and has angle change means for changing the angle of the treatment unit with respect to the axis of the insertion unit in response to the changing operation to the angle change operation unit.

25. The surgical instrument according to claim 24, wherein the clamping unit has two clamping members having respective flat sections, and the action of opening and closing the clamping unit can be executed by moving at least one of the two clamping members in the direction substantially perpendicular to the plane of the flat section in response to the opening and closing operation in the opening and closing operation section.

26. The surgical instrument according to claim 25, wherein
the treatment unit comprises impelling means for constantly impelling at least one of the two clamping members in the direction of coming into intimate contact with the other, and
the one clamping member is moved by the opening operation of the opening and closing operations, in the direction of going away from the other clamping member against the impelling force in the direction of coming into intimate contact.

27. The surgical instrument according to claim 26, wherein
the other clamping member is positioned in the distal end section of the treatment unit, while the one clamping member is constantly impelled to come into intimate contact with the other clamping member by the impelling means.

28. The surgical instrument according to any one of claims 24 to 27, wherein
the rotational operation section transmits a quantity of rotation to the treatment unit by the rotational action of a shaft member about the axis as a rotation center; and
the treatment unit rotates the treatment unit in response to the rotational action of the shaft member.

29. The surgical instrument according to claim 28, wherein
the shaft member is a cylindrical member;
the opening and closing operation section transmits an opening and closing quantity to the treatment unit by the back and forth movement of a wire member inserted through the cylindrical member; and
the treatment unit is opened and closed in response to the back and forth movement action of the wire member.

30. The surgical instrument according to any one of claims 24 to 29, wherein
the angle change operation section transmits the quantity of angle change to the angle change means by the back and forth movement of a rod member; and
the angle change means changes the angle of the extension direction of the treatment unit in response to the back and forth movement action of the rod member.

31. The surgical instrument according to claim 30, wherein the angle change means has coil length variation suppression means that decreases the variation of length of a coil member connected to the rod member.

32. The surgical instrument according to claim 31, wherein the coil length variation suppression means has a curving center of the coil in a position at a distance of about ($\sqrt{2}\pi r/4$) from the radial center of the coil member, where $\pi$ stands for a ratio of the circumference of a circle to its diameter and r stands for a radius of the coil member.

33. A surgical instrument comprising:

an insertion unit;
an operation unit provided at one end of the insertion unit; and
a treatment unit provided so as to extend from the other end of the insertion unit and having a clamping unit, wherein
the operation unit comprises a rotational operation section for performing the operation of rotating the treatment unit, and an opening and closing operation section for performing the operation of opening and closing the treatment unit;
the treatment unit can rotate about the axis of the treatment unit in response to the rotational operation in the rotational operation section and can execute the action of opening and closing the clamping unit in response to the opening and closing operation in the opening and closing operation section;
the rotational operation section transmits a quantity of rotation to the treatment unit by the rotational action of a shaft member about the axis as a rotation center; and
the treatment unit rotates in response to the rotational action of the shaft member.

34. The surgical instrument according to claim 33, wherein the clamping unit has two clamping members having flat sections respectively, and the action of opening and closing the clamping unit can be executed by moving at least one of the two clamping members in the direction substantially perpendicular to the plane of the flat section in response to the opening and closing operation in the opening and closing operation section.

**35.** The surgical instrument according to claim 34, wherein
the shaft member is a cylindrical member;
the opening and closing operation section transmits an opening and closing quantity to the treatment unit by the back and forth movement of a wire member or a rod member inserted through the cylindrical member; and
the treatment unit is opened and closed in response to the back and forth movement action of the wire member.

**36.** The surgical instrument according to claim 34 or 35, wherein the treatment unit comprises impelling means for constantly impelling at least one of the two clamping members in the direction of coming into intimate contact with the other, and
the one clamping member is moved in the direction of going away from the other clamping member against the impelling force in the direction of coming into intimate contact by the opening operation of the opening and closing operations.

**37.** The surgical instrument according to claim 36, wherein
the other clamping member is positioned in the distal end section of the treatment unit, and the one clamping member is constantly impelled to come into intimate contact with the other clamping member by the impelling means.

**38.** The surgical instrument according to claim 34, wherein
the two clamping members have disk-like portions having the flat sections respectively.

**39.** The surgical instrument according to claim 34, wherein
the thickness of the respective disk-like portions of the two clamping members is small.

**40.** The surgical instrument according to claim 34, wherein
a peripheral section on the distal end side of the disk-like portion of the clamping member on the distal end side, of the two clamping members, is a curved section or slanted section.

**41.** The surgical instrument according to claim 34, wherein
the diameter of disk-like portions of the two clamping members is larger than the diameter of the portions of the treatment unit other than the disk-like portions.

**42.** A surgical instrument comprising:

an insertion unit;
a treatment unit provided at one end of the insertion unit and comprising a clamping unit which is freely rotatable about one axis as a center and can perform an opening and closing action and a bending action;
an operation unit provided at the other end of the insertion unit;
a rotation power transmission mechanism, provided between the operation unit and the treatment unit along the insertion unit, for transmitting a rotation power for rotating the treatment unit from the operation unit to the treatment unit;
an opening and closing power transmission mechanism, provided between the operation unit and the treatment unit along the insertion unit for transmitting an opening and closing power for an action of opening and closing the clamping unit from the operation unit to the treatment unit;
a bending power transmission mechanism, provided between the operation unit and the treatment unit along the insertion unit, for transmitting a bending power for an action of bending the clamping unit from the operation unit to the treatment unit;
an opening and closing operation mechanism provided in the operation unit for applying the opening and closing power;
a rotational operation mechanism provided in the operation unit for applying the rotation power;
a bending operation mechanism provided in the operation unit for applying the bending power; and
a finger hook section provided in the operation unit and provided in a position substantially identical to the position where the rotational operation mechanism and the bending operation mechanism are provided or on the proximal end side from this position.

**43.** The surgical instrument according to claim 42, wherein
the finger hook section has two protruding sections and a finger is hooked between the two protruding sections.

**44.** The surgical instrument according to claim 42 or 43, further comprising a palm placement section provided in the operation unit and provided on the proximal end side from the position where the finger hook section is provided.

45. The surgical instrument according to claim 44, wherein
the palm placement section is provided with at least one hole.

46. The surgical instrument according to claim 44 or 45, further comprising a position adjusting mechanism for adjusting the position of the palm placement in the long axis direction of the operation unit.

47. The surgical instrument according to any one of claims 42 to 46, wherein the opening and closing operation mechanism comprises a press-button.

48. The surgical instrument according to any one of claims 42 to 47, wherein the rotational operation mechanism comprises a dial provided on the external cover surface of the operation unit.

49. A hollow viscera anastomosis method for anastomosing a graft hollow viscera to a recipient hollow viscera, comprising:

a first anastomosis opening formation step of forming a first anastomosis opening in the graft hollow viscera;
a second anastomosis opening formation step of forming a second anastomosis opening to be anastomosed to the first anastomosis opening in the recipient hollow viscera;
a hollow viscera suturing step of rotating a bent suturing needle that has a suturing thread connected to the distal end thereof by a surgical instrument having a clamping unit that releasably clamps the suturing needle and can rotate the suturing needle in the clamping surface, thereby suturing the first anastomosis opening and the second anastomosis opening with the suturing thread; and
an extension direction angle adjustment step of adjusting an angle of an extension direction of the clamping unit, in which the angle in the extension direction in a plane including the insertion axis of the surgical instrument can be adjustable.

50. A surgical instrument comprising:

an insertion unit;
a gripping unit extending out from the distal end section of the insertion unit; and
an operation unit provided at the proximal end of the insertion unit and serving to be gripped by a surgeon, wherein the gripping unit comprises a rotation mechanism for rotating the gripping unit about an axis substantially identical to the extension direction of the gripping unit; an angle variation mechanism that can change the extension angle of the gripping unit within the prescribed range with respect to the insertion unit; and a gripping mechanism that can be opened and closed for holding a gripping object with a prescribed power.

51. The surgical instrument according to claim 50, wherein
the rotation mechanism and the angle variation mechanism comprise, in the gripping unit, a cylindrical gripping unit body that regulates the axial direction of the gripping unit and extends out from the insertion unit, and a gripping unit rotation body that is provided at the proximal end of the gripping unit, causes axial rotation of the gripping unit body, and causes the rotation of the gripping unit body with respect to the insertion unit;
the insertion unit comprises, in the distal end section thereof, a socket into which the gripping unit rotation body is fitted rotatably about the axis of the gripping unit body and rotatably about the gripping unit rotation body as a support point with respect to the axial direction of the insertion unit, and a long orifice through which the gripping unit body is passed and which regulates the rotation range of the gripping unit body, and
the gripping mechanism comprises
a holding section for gripping that can move along the axial direction of the gripping unit body and can be brought into contact with or distanced from the end section on the side opposite to the side where the gripping unit rotation body of the gripping unit body is provided;
a gripping rod that has a shaft section for gripping that can move along the axial direction of the gripping unit body and has one end section provided in the holding section for gripping, and a rod head section for gripping that can slide with respect to the inner circumferential surface of the gripping unit body and is provided in the other end section of the shaft section for gripping;
an elastic member for impelling in the direction of closing the space between the gripping rod and the holding section for gripping with respect to the gripping unit body; and
a power direction conversion mechanism which comprises a circular arc section such that the zone thereof to be brought into contact with the rod head section for gripping is formed as the circumference of a circle, in which the center of the circular arc of the circular arc section and the center of the gripping unit rotation body coincide when the gripping object is gripped between the gripping unit body and the holding body for gripping, and which converts a power along the axial direction of the insertion unit into a power along the axial direction of the shaft section for the gripping unit of the gripping rod in response to the operation of the operation unit.

52. The surgical instrument according to claim 50, wherein
the rotation mechanism comprises a cylindrical gripping unit body that regulates the axial direction of the gripping unit and extends out from the insertion unit, and a rotation member that is provided on the outer periphery of the gripping unit body and causes the gripping unit body to rotate about the axis of the gripping unit body in response to the operation of the operation unit;
the angle variation mechanism comprises a pivot shaft provided on the outer periphery of the gripping unit body between the rotation member and a distal end section of the gripping unit body with respect to the insertion unit; and
the insertion unit comprises
a bearing for receiving the pivot shaft;
a brake mechanism that can switch between a suppression state, in which the action of the pivot shaft on the bearing is prohibited in response to the operation of the operation unit after pivot shaft has been received by the bearing, and a permission state in which the action is allowed; and
a tension maintenance mechanism that maintains the tension of a linking member, which links the rotation member and the operation unit.

# FIG.1

# FIG.2

# FIG.3

SUTURING
THREAD

SUTURING
NEEDLE

# FIG.4

# FIG.5

```
            ┌──────────────────────┐
            │     SEQUENCE OF       │
            │   THE ANASTOMOSIS     │
            └──────────────────────┘
                       │
            ┌──────────────────────────────────────┐   S51
            │ PORT HOLES ADDED AND TROCARS CHANGED  │
            └──────────────────────────────────────┘
                       │
            ┌──────────────────────────────────────┐   S52
            │    INSTRUMENT INSERTED INTO TROCARS    │
            └──────────────────────────────────────┘
                       │
            ┌──────────────────────────────────────┐   S53
            │            CUT PERICARDIAL SAC         │
            └──────────────────────────────────────┘
                       │
            ┌──────────────────────────────────────┐   S54
            │   SUPPRESS EFFECT OF HEART PULSATIONS  │
            │            WITH STABILIZER             │
            └──────────────────────────────────────┘
```

**ANASTOMOSIS PROCEDURE PREPARATION STEP** — S51, S52, S53, S54

```
            ┌──────────────────────────────────────┐   S55
            │ SEAL INTERNAL THORACIC ARTERY SIDE WITH│
            │            HEMASTOSIS CLIP             │
            └──────────────────────────────────────┘
                       │
            ┌──────────────────────────────────────┐   S56
            │      STOP BLEEDING BY TOURNIQUET       │
            └──────────────────────────────────────┘
                       │
            ┌──────────────────────────────────────┐   S57
            │   CUT INTERNAL THORACIC ARTERY SIDE    │
            │            HEMASTOSIS CLIP             │
            └──────────────────────────────────────┘
                       │
            ┌──────────────────────────────────────┐   S58
            │ TRIM CUT SURFACE OF INTERNAL THORACIC ARTERY │
            └──────────────────────────────────────┘
```

**INTERNAL THORACIC ARTERY PREPARATION STEP** — S55, S56, S57, S58

```
            ┌──────────────────────────────────────┐   S59
            │   CORONARY ARTERY ON PIVOT SIDE IS SEALED │
            └──────────────────────────────────────┘
                       │
            ┌──────────────────────────────────────┐   S60
            │ CUT PERICARDIAL SAC, AND EXPOSE CORONARY │
            │                ARTERY                  │
            └──────────────────────────────────────┘
                       │
            ┌──────────────────────────────────────┐   S61
            │     CUT SIDE WALL OF CORONARY ARTERY   │
            └──────────────────────────────────────┘
                       │
            ┌──────────────────────────────────────┐   S62
            │     INSERT SHUNT INTO CORONARY ARTERY  │
            └──────────────────────────────────────┘
                       │
            ┌──────────────────────────────────────┐   S63
            │   RELEASE SEALING OF CORONARY ARTERY ON│
            │                PIVOT SIDE              │
            └──────────────────────────────────────┘
```

**CORONARY ARTERY PREPARATION STEP** — S59, S60, S61, S62, S63

```
            ┌──────────────────────────────────────┐   S64
            │ INSERT NEEDLE DRIVER AND ADJUST ANGLE OF│
            │ EXTENSION DIRECTION OF TREATMENT UNIT  │
            └──────────────────────────────────────┘
                       │
            ┌──────────────────────────────────────┐   S65
            │   SUTURE INTERNAL THORACIC ARTERY AND  │
            │ CORONARY ARTERY AND ADJUST ANGLE OF    │
            │ EXTENSION DIRECTION OF TREATMENT UNIT  │
            └──────────────────────────────────────┘
                       │
            ┌──────────────────────────────────────┐   S66
            │  PULL OUT SHUNT FROM CORONARY ARTERY   │
            └──────────────────────────────────────┘
                       │
            ┌──────────────────────────────────────┐   S67
            │     LIGATE THREAD FOR ANASTOMOSIS      │
            └──────────────────────────────────────┘
                       │
            ┌──────────────────────┐
            │       RETURN          │
            └──────────────────────┘
```

**INTERNAL THORACIC ARTERY AND CORONARY ARTERY SUTURING STEP** — S64, S65, S66, S67

# FIG.6

# FIG.7

# FIG.8

# FIG.9

# FIG.10

# FIG.11

# FIG.12

FIG.13

IX

IX

2

4

FIG.14

EP 1 723 913 A1

FIG.15

# FIG.16

# FIG.17

# FIG.18

# FIG.19

# FIG.20

# FIG.21

# FIG.22

# FIG.23

# FIG.24

# FIG.25

EP 1 723 913 A1

# FIG.26

**FIG.27**

# FIG.28

# FIG.29

FIG.30

# FIG.31

θ=0°

# FIG.32

# FIG.33

COIL
GUIDE

C(X2,Y2)   B(X1,Y1)

-90°   17   13   14

A

x

O

25

21
y

L (THICK LINE)

4

8

θ=-90° (COUNTER CLOCKWISE
DIRECTION TAKEN AS POSITIVE)

# FIG.34

CURVING ANGLE θ [°]

# FIG.35

# FIG.36

# FIG.37

# FIG.38

# FIG.39

FIG.40

# FIG.41

EP 1 723 913 A1

# FIG.42

EP 1 723 913 A1

# FIG.43

# FIG.44

FIG.45

# FIG.46

EP 1 723 913 A1

FIG.47

# FIG.48

508a  153A  508  152A  503

508b
508a

503a

504

506

507

502a

14  510  509  14a

502b

502

FIG.49

# FIG.50

# FIG.51

# FIG.52

# FIG.53

# FIG.54

# FIG.55

# FIG.56

**(A)**

**(B)**

# FIG.57

# FIG.58

**(A)**

**(B)**

# FIG.59

# FIG.60

# FIG.61

# FIG.62

# FIG.63

# FIG.64

# FIG.65

# FIG.66

# FIG.67

# FIG.68

**(A)**

**(B)**

# FIG.69

**(A)**

**(B)**

# FIG.70

**(A)**

**(B)**

# FIG.71

**(A)**

**(B)**

# FIG.72

## INTERNATIONAL SEARCH REPORT

| | International application No. |
|---|---|
| | PCT/JP2005/004236 |

| A. CLASSIFICATION OF SUBJECT MATTER |
|---|
| Int.Cl⁷ A61B17/06, 17/28 |

According to International Patent Classification (IPC) or to both national classification and IPC

| B. FIELDS SEARCHED |
|---|
| Minimum documentation searched (classification system followed by classification symbols) |
| Int.Cl⁷ A61B17/04-17/062, 17/28 |

Documentation searched other than minimum documentation to the extent that such documents are included in the fields searched

| Jitsuyo Shinan Koho | 1922-1996 | Jitsuyo Shinan Toroku Koho | 1996-2005 |
|---|---|---|---|
| Kokai Jitsuyo Shinan Koho | 1971-2005 | Toroku Jitsuyo Shinan Koho | 1994-2005 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X<br><br>Y | JP 2003-520623 A (LA PRECISION),<br>08 July, 2003 (08.07.03),<br>Par. Nos. [0030] to [0043]; Figs. 1 to 3<br>& US 6666854 B1<br>column 4, line 58 to column 6, line 32;<br>Figs. 1 to 3<br>& WO 2001/000095 A1 & EP 1189539 A1<br>& FR 2795301 A1 | 1-6,9-16,24,<br>28,30,50<br>7,8,17-23,<br>25-27,29,<br>36-48 |
| Y | US 5951575 A (Heartport, Inc.),<br>14 September, 1999 (14.09.99),<br>Column 6, line 55 to column 7, line 10;<br>Figs. 1, 2<br>(Family: none) | 7,8,17-23 |

| ☒ | Further documents are listed in the continuation of Box C. | ☐ | See patent family annex. |
|---|---|---|---|

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 03 June, 2005 (03.06.05) | 21 June, 2005 (21.06.05) |

| Name and mailing address of the ISA/<br>    Japanese Patent Office | Authorized officer |
|---|---|
| Facsimile No. | Telephone No. |

Form PCT/ISA/210 (second sheet) (January 2004)

INTERNATIONAL SEARCH REPORT

International application No.

PCT/JP2005/004236

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 2004-16662 A  (Olympus Corp.),<br>22 January, 2004 (22.01.04),<br>Par. No. [0040]; Fig. 1<br>(Family: none) | 7,17-23,<br>42-48 |
| Y | JP 2000-189425 A  (Ethicon Endo-Surgery, Inc.),<br>11 July, 2000 (11.07.00),<br>Par. No. [0016]; Figs. 1, 3<br>& EP 0931510 A1<br>Par. No. [0016]; Figs. 1, 3<br>& US 6206894 B1 | 25-27,36-41,<br>47 |
| X<br>Y | JP 7-275253 A  (Terumo Corp.),<br>24 October, 1995 (24.10.95),<br>Par. Nos. [0062] to [0065]; Figs. 1 to 5<br>(Family: none) | 33-35<br>29,36-41 |
| Y | JP 2003-310624 A  (Yuichiro NISHIJIMA),<br>05 November, 2003 (05.11.03),<br>Par. No. [0027]; Fig. 1<br>(Family: none) | 45 |
| A | JP 2002-501413 A  (Yoon InBae),<br>15 January, 2002 (15.01.02),<br>Full text; all drawings<br>& US 6004332 A        & WO 1998/048705 A1<br>& EP 0987987 A1 | 1-48,50-52 |
| A | JP 7-163574 A  (Ethicon, Inc.),<br>27 June, 1995 (27.06.95),<br>Full text; all drawings<br>& US 5405344 A | 1-48,50-52 |
| A | US 5300082 A  (Sharpe Endosurgical Corp.),<br>05 April, 1994 (05.04.94),<br>Full text; all drawings<br>(Family: none) | 1-48,50-52 |

Form PCT/ISA/210 (continuation of second sheet) (January 2004)

## EP 1 723 913 A1

<div align="center">

### INTERNATIONAL SEARCH REPORT

</div>

| International application No. |
|---|
| PCT/JP2005/004236 |

**Box No. II    Observations where certain claims were found unsearchable (Continuation of item 2 of first sheet)**

This international search report has not been established in respect of certain claims under Article 17(2)(a) for the following reasons:

1. ☒ Claims Nos.: 4 9
   because they relate to subject matter not required to be searched by this Authority, namely:
   Claim 49 pertains to methods for treatment of the human body by therapy and thus relates to a subject matter which this International Searching Authority is not required, under the provisions of the PCT Rule 39.1(iv), to search.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**      ☐ The additional search fees were accompanied by the applicant's protest.

☐ No protest accompanied the payment of additional search fees.

Form PCT/ISA/210 (continuation of first sheet (2)) (January 2004)

<div align="center">

**104**

</div>

**REFERENCES CITED IN THE DESCRIPTION**

**Patent documents cited in the description**

- US 5951575 A
- JP 2002306495 A
- US 5807243 A